# FASCICULE DE BREVET EUROPEEN

(11) **EP 1 844 159 B1**
(45) Date de publication et mention de la délivrance du brevet: **17.09.2014**
(21) Numéro de dépôt: 06709454.0
(22) Date de dépôt: 30.01.2006
(51) Int. Cl.: C12Q 1/68

(54) **PROCEDE POUR LE PRONOSTIC D'UN SYNDROME SEPTIQUE**
VERFAHREN ZUR PROGNOSE EINES SEPTISCHEN SYNDROMS
METHOD FOR PROGNOSIS OF A SEPTIC SYNDROME

(30) Priorité: 31.01.2005 FR 0550267
(43) Date de publication de la demande: 17.10.2007
(60) Demande divisionnaire: 10180574.5
(73) Titulaire: BIOMERIEUX, 69280 Marcy-L'Etoile (FR)
(72) Inventeur: PACHOT, Alexandre, F-01400 Sulignat (FR); MONNERET, Guillaume, F-69008 Lyon (FR); LEPAPE, Alain, F-69230 Saint-Genis-Laval (FR)
(74) Mandataire: Bitaud, Valérie Marie-Odile
(86) Numéro de dépôt international: PCT/FR2006/050070
(87) Numéro de publication internationale: WO 2006/079760

(56) Documents cités:
- EP-A- 1 310 567
- WO-A-2004/043236
- WO-A-2004/087949
- WO-A-2004/108957
- "Affimetrix GeneChip Human Genome U133 Array Set HG-U133A" GEO, 11 avril 2002 (2002-04-11), XP002254749
- PRUCHA M ET AL: "EXPRESSION PROFILING: TOWARD AN APPLICATION IN SEPSIS DIAGNOSTICS" SHOCK, vol. 22, no. 1, juillet 2004 (2004-07), pages 29-33, XP008036997
- COBB J P ET AL: "SEPSIS GENE EXPRESSION PROFILING: MURINE SPLENIC COMPARED WITH HEPATIC RESPONSES DETERMINED BY USING COMPLEMENTARY DNA MICROARRAYS" CRITICAL CARE MEDICINE, WILLIAMS AND WILKINGS COMPANY, BALTIMORE, MA, US, vol. 30, no. 12, décembre 2002 (2002-12), pages 2711-2721, XP008037048 ISSN: 0090-3493

### SEQUENCE LISTING

<110> bioMerieux SA
<120> Procede pour le diagnostic/pronostic d'un syndrome septique
<130> Unknown
<160> 34
<170> PatentIn version 3.3
<210> 1
   <211> 3100
   <212> DNA
   <213> Homo sapiens
<400> 1
   actcgtctct ggtaaagtct gagcaggaca gggtggctga ctggcagatc cagaggttcc      60
   cttggcagtc cacgccaggc cttcaccatg gatcagttcc ctgaatcagt gacagaaaac     120
   tttgagtacg atgatttggc tgaggcctgt tatattgggg acatcgtggt ctttgggact     180
   gtgttcctgt ccatattcta ctccgtcatc tttgccattg gcctggtggg aaatttgttg     240
   gtagtgtttg ccctcaccaa cagcaagaag cccaagagtg tcaccgacat ttacctcctg     300
   aacctggcct tgtctgatct gctgtttgta gccactttgc ccttctggac tcactatttg     360
   ataaatgaaa agggcctcca caatgccatg tgcaaattca ctaccgcctt cttcttcatc     420
   ggcttttttg gaagcatatt cttcatcacc gtcatcagca ttgataggta cctggccatc     480
   gtcctggccg ccaactccat gaacaaccgg accgtgcagc atggcgtcac catcagccta     540
   ggcgtctggg cagcagccat tttggtggca gcaccccagt tcatgttcac aaagcagaaa     600
   gaaaatgaat gccttggtga ctaccccgag gtcctccagg aaatctggcc cgtgctccgc     660
   aatgtggaaa caaattttct tggcttccta ctccccctgc tcattatgag ttattgctac     720
   ttcagaatca tccagacgct gttttcctgc aagaaccaca agaaagccaa agccattaaa     780
   ctgatccttc tggtggtcat cgtgtttttc ctcttctgga caccctacaa cgttatgatt     840
   ttcctggaga cgcttaagct ctatgacttc tttcccagtt gtgacatgag gaaggatctg     900
   aggctggccc tcagtgtgac tgagacggtt gcatttagcc attgttgcct gaatcctctc     960
   atctatgcat ttgctgggga gaagttcaga agataccttt accacctgta tgggaaatgc    1020
   ctggctgtcc tgtgtgggcg ctcagtccac gttgatttct cctcatctga atcacaaagg    1080
   agcaggcatg gaagtgttct gagcagcaat tttacttacc acacgagtga tggagatgca    1140
   ttgctccttc tctgaaggga atcccaaagc cttgtgtcta cagagaacct ggagttcctg    1200
   aacctgatgc tgactagtga ggaaagattt ttgttgttat ttcttacagg cacaaaatga    1260
   tggacccaat gcacacaaaa caaccctaga gtgttgttga gaattgtgct caaaatttga    1320
   agaatgaaca aattgaactc tttgaatgac aaagagtaga catttctctt actgcaaatg    1380
   tcatcagaac tttttggttt gcagatgaca aaaattcaac tcagactagt ttagttaaat    1440
   gagggtggtg aatattgttc atattgtggc acaagcaaaa gggtgtctga gccctcaaag    1500
   tgaggggaaa ccagggcctg agccaagcta gaattccctc tctctgactc tcaaatcttt    1560
   tagtcattat agatccccca gactttacat gacacagctt tatcaccaga gagggactga 1620
   cacccatgtt tctctggccc caagggaaaa ttcccaggga agtgctctga taggccaagt 1680
   ttgtatcagg tgcccatccc tggaaggtgc tgttatccat ggggaaggga tatataagat 1740
   ggaagcttcc agtccaatct catggagaag cagaaataca tatttccaag aagttggatg 1800
   ggtgggtact attctgatta cacaaaacaa atgccacaca tcacccttac catgtgcctg 1860
   atccagcctc tcccctgatt acaccagcct cgtcttcatt aagccctctt ccatcatgtc 1920
   cccaaacctg caagggctcc ccactgccta ctgcatcgag tcaaaactca aatgcttggc 1980
   ttctcatacg tccaccatgg ggtcctacca atagattccc cattgcctcc tccttcccaa 2040
   aggactccac ccatcctatc agcctgtctc ttccatatga cctcatgcat ctccacctgc 2100
   tcccaggcca gtaagggaaa tagaaaaacc ctgcccccaa ataagaaggg atggattcca 2160
   accccaactc cagtagcttg ggacaaatca agcttcagtt tcctggtctg tagaagaggg 2220
   ataaggtacc tttcacatag agatcatcct ttccagcatg aggaactagc caccaactct 2280
   tgcaggtctc aacccttttg tctgcctctt agacttctgc tttccacacc tgcactgctg 2340
   tgctgtgccc aagttgtggt gctgacaaag cttggaagag cctgcaggtg ccttggccgc 2400
   gtgcatagcc cagacacaga agaggctggt tcttacgatg gcacccagtg agcactccca 2460
   agtctacaga gtgatagcct tccgtaaccc aactctcctg gactgccttg aatatcccct 2520
   cccagtcacc ttgtgcaagc ccctgcccat ctgggaaaat accccatcat tcatgctact 2580
   gccaacctgg ggagccaggg ctatgggagc agcttttttt tcccccctag aaacgtttgg 2640
   aacaatgtaa aactttaaag ctcgaaaaca attgtaataa tgctaaagaa aaagtcatcc 2700
   aatctaacca catcaatatt gtcattcctg tattcacccg tccagacctt gttcacactc 2760
   tcacatgttt agagttgcaa tcgtaatgta cagatggttt tataatctga tttgttttcc 2820
   tcttaacgtt agaccacaaa tagtgctcgc tttctatgta gtttggtaat tatcatttta 2880
   gaagactcta ccagactgtg tattcattga agtcagatgt ggtaactgtt aaattgctgt 2940
   gtatctgata gctctttggc agtctatatg tttgtataat gaatgagaga ataagtcatg 3000
   ttccttcaag atcatgtacc ccaatttact tgccattact caattgataa acatttaact 3060
   tgtttccaat gtttagcaaa tacatatttt atagaacttc 3100
<210> 2
   <211> 783
   <212> DNA
   <213> Homo sapiens
<400> 2
   atgcagagga tctcctccct catccatctc tctctcttct gggcaggagt catgtcagcc      60
   attgagttgg tgcctgaaca ccaaacagtg cctgtgtcaa taggggtccc tgccaccctc     120
   aggtgctcca tgaaaggaga agcgatcggt aactactata tcaactggta caggaagacc     180
   caaggtaaca caatgacttt catataccga gaaaaggaca tctatggccc tggtttcaaa     240
   gacaatttcc aaggtgacat tgatattgca aagaacctgg ctgtacttaa gatacttgca     300
   ccatcagaga gagatgaagg gtcttactac tgtgcctgtg acaccttggg gatggggggg     360
   gaatacaccg ataaactcat ctttggaaaa ggaacccgtg tgactgtgga accaagaagt     420
   cagcctcata ccaaaccatc cgtttttgtc atgaaaaatg gaacaaatgt cgcttgtctg     480
   gtgaaggaat tctaccccaa ggatataaga ataaatctcg tgtcatccaa gaagataaca     540
   gagtttgatc ctgctattgt catctctccc agtgggaagt acaatgctgt caagcttggt     600
   aaatatgaag attcaaattc agtgacatgt tcagttcaac acgacaataa aactgtgcac     660
   tccactgact ttgaagtgaa gacagattct acagatcacg taaaaccaaa ggaaactgaa     720
   aacacaaagc aaccttcaaa gagctgccat aaacccaaag ccatagttca taccgagaag     780
   taa                                                                   783
<210> 3
   <211> 5191
   <212> DNA
   <213> Homo sapiens
<400> 3
   gcggccgcgg gctctcgcgg ggcggcgacg ccgcggggag gatgctgctt gccgcgcccg     60
   cgtcctcacc gtcctcccgg gccgcctgct ggggctttgt tgtggcccgg acgccgcggg    120
   ccaccccctg aagtcgcctg ccgccgccgc cgccgcacct agcggacggg cgggcgggcg    180
   cgcgtgtgcc caggagtgcg cgcctgtcgc ggtggtgggt gcaggactgg acccacgggc    240
   ccattgtgcg cccgcccgcg gcagccagga ccatgtgggt gaacccggag gaggtgttgc    300
   tggccaacgc gctgtggatc accgagaggg ccaacccata cttcatcctg cagcggagga    360
   agggccacgc cggcgatgga ggcggcggcg gcggactggc gggcctgctg gtgggtaccc    420
   ttgatgttgt gttggactcc agcgcccggg tcgctcctta ccgaatcttg taccagactc    480
   cagactccct ggtctactgg accatcgcct gtggtggttc caggaaagaa atcactgaac    540
   actgggaatg gcttgagcaa aatctcttgc agacactctc catctttgaa aatgagaatg    600
   atatcaccac atttgtgaga ggaaaaatac agggcatcat tgcagaatac aacaaaatca    660
   atgatgtaaa ggaagatgat gacacggaga agtttaaaga agccattgtg aaatttcata    720
   ggctgtttgg gatgccagag gaagagaaac tcgtcaacta ttactcttgc agctattgga    780
   aggggaaggt cccccgtcag ggttggatgt acctcagcat taaccacctt tgcttttatt    840
   cttttcttat gggaagggaa gcgaaactgg tcatccggtg ggtagacatc actcagctcg    900
   agaagaatgc caccctgctt ctgcctgatg tgatcaaagt gagcacacgg tccagtgagc    960
   atttcttctc tgtattcctc aacatcaacg agaccttcaa gttaatggag cagcttgcca   1020
   acatagccat gaggcaactc ttagacaatg agggatttga acaagatcga tccctgccca   1080
   aactcaaaag gaaatctcct aaaaaagtgt ctgctctaaa acgtgatctt gatgccaggg   1140
   caaagagtga gagataccgt gcacttttcc ggctgcccaa agatgaaaaa ttagatggcc   1200
   acacagactg cactctctgg actccattta acaaaatgca cattttgggg cagatgtttg   1260
   tgtccacaaa ttacatctgt tttaccagca aggaggagaa cttatgtagc ctcattatcc   1320
   cgctccgtga ggtgacaatt gtggaaaagg cagacagctc cagtgtgctc cccagtccct   1380
   tatccatcag cacccgaaac aggatgacct tcctatttgc caacttgaaa gatagagact 1440
   ttctagtgca gaggatctca gatttcctgc aacagactac ttccaaaata tattctgaca 1500
   aggagtttgc aggaagttac aacagttcag atgatgaggt gtactctcga cccagcagcc 1560
   tcgtctcctc cagcccccag agaagcacga gctctgatgc tgatggagag cgccagttta 1620
   acctaaatgg caacagcgtc cccacagcca cacagaccct gatgaccatg tatcggcggc 1680
   ggtctcccga ggagttcaac ccgaaattgg ccaaagagtt tctgaaagag caagcctgga 1740
   agattcactt tgctgagtat gggcaaggga tctgcatgta ccgcacagag aaaacgcggg 1800
   agctggtgtt gaagggcatc ccggagagca tgcgtgggga gctctggctg ctgctgtcag 1860
   gtgccatcaa tgagaaggcc acacatcctg ggtactatga agacctagtg gagaagtcca 1920
   tggggaagta taatctcgcc acggaggaga ttgagaggga tttacaccgc tcccttccag 1980
   aacacccagc ttttcagaat gaaatgggca ttgctgcact aaggagagtc ttaacagctt 2040
   atgcttttcg aaatcccaac atagggtatt gccaggccat gaatattgtc acttcagtgc 2100
   tgctgcttta tgccaaagag gaggaagctt tctggctgct tgtggctttg tgtgagcgca 2160
   tgctcccaga ttactacaac accagagttg tgggtgcact ggtggaccaa ggtgtctttg 2220
   aggagctagc acgagactac gtcccacagc tgtacgactg catgcaagac ctgggcgtga 2280
   tttccaccat ctccctgtct tggttcctca cactatttct cagtgtgatg ccttttgaga 2340
   gtgcagttgt ggttgttgac tgtttcttct atgaaggaat taaagtgata ttccagttgg 2400
   ccctagctgt gctggatgca aatgtggaca aactgttgaa ctgcaaggat gatggggagg 2460
   ccatgaccgt tttgggaagg tatttagaca gtgtgaccaa taaagacagc acactgcctc 2520
   ccattcctca cctccactcc ttgctcagcg atgatgtgga accttaccct gaggtagaca 2580
   tctttagact catcagaact tcctacgaga aattcggaac tatccgggca gatttgattg 2640
   aacagatgag attcaaacag agactgaaag tgatccagac gctggaggat actacgaaac 2700
   gcaacgtggt acgaaccatt gtgacagaaa cttcctttac cattgatgag ctggaagaac 2760
   tttatgctct tttcaaggca gaacatctca ccagctgcta ctggggcggg agcagcaacg 2820
   cgctggaccg gcatgacccc agcctgccct acctggaaca gtatcgcatt gacttcgagc 2880
   agttcaaggg aatgtttgct cttctctttc cttgggcatg tggaactcac tctgacgttc 2940
   tggcctcccg cttgttccag ttattagatg aaaatggaga ctctttgatt aacttccggg 3000
   agtttgtctc tgggctaagt gctgcatgcc atggggacct cacagagaag ctcaaactcc 3060
   tgtacaaaat gcacgtcttg cctgagccat cctctgatca agatgaacca gattctgctt 3120
   ttgaagcaac tcagtacttc tttgaagata ttaccccaga atgtacacat gttgttggat 3180
   tggatagcag aagcaaacag ggtgcagatg atggctttgt tacggtgagc ctaaagccag 3240
   acaaagggaa gagagcaaat tcccaagaaa atcgtaatta tttgagactg tggactccag 3300
   aaaataaatc taagtcaaag aatgcaaagg atttacccaa attaaatcag gggcagttca 3360
   ttgaactgtg taagacaatg tataacatgt tcagcgaaga ccccaatgag caggagctgt 3420
   accatgccac ggcagcagtg accagcctcc tgctggagat tggggaggtc ggcaagttgt 3480
   tcgtggccca gcctgcaaag gaggacaagc tgcactgcga ggacatcgga gaggacacgg 3540
   tcctggtgcg gagcggccag ggcacggcgg cactgccccg gagcaccggc ctggaccggg 3600
   actgggccat caccttcgag cagttcctgg cctccctctt aactgagcct gccctggtca 3660
   agtactttga caagcccgtg tgcatgatgg ccaggattac cagtgcaaaa aacatccgga 3720
   tgatgggcaa gcccctcacc tcggccagtg actatgaaat ctcggccatg tccggctgac 3780
   acgggcgcct tcccggggga gtgggaggaa agggagggga gggatttttt atgttcttct 3840
   gtgttgagtt ttttctttct ttcttttaaa ttaaatattt attagtacct ggcttgaagc 3900
   ctagtgtttt cataatgtaa ttcaatgaaa actgttggag aaatatttaa acacctcaat 3960
   gtaggtacat tacactcttg ttgcggggag gggatttacc agaatacagt ttatttcgtg 4020
   aattctaaaa aacaaaaaga tgaatctgtc agtgatatgt gtgtattata acttattaat 4080
   cttgctgttg agctgtatac atggtttaaa aaatagtact gtttaatgct aagtaaggca 4140
   gcagtcattt gtgtattcag gctttttaaa taaaattaga gctgtaagga aaatgaaaag 4200
   ccacaaatgc aagactgttc ttaaatggaa ggcatagtca gcgagggtaa atcctatacc 4260
   actttaggaa gtattaaaaa tatttttaag atttgaaata tatttcatag aagtcctcta 4320
   ttcaaaatca tattccacag atgttcccct tcaaagggaa aacatttggg gttctaaaca 4380
   gttatgaaag taagtgattt ttacatgatt ccagaataac acttgtattg accaatttaa 4440
   acagatacca gaccaatttt gcatttaaga aattgttctg attatttacg tcaactcatt 4500
   agaattcagt gaaaagtaac agtcttttgt cacagagaat ctgaaagtag cagcaaagac 4560
   agagggctca tgacaggttt ttgcttttgc tttgcttttg tttttgaaag agtaaaagta 4620
   ctgatgcttc tgatactgga tgtttagctt cttactgcaa aaacataagt aaaacagtca 4680
   actttaccat ttccgtattc tccatagatt gaagaaattt ataccacata tcgcatatga 4740
   ccatctttcc atcaaatcaa tgtagagata atgtaaactg aaaaaaaatc tgcaagataa 4800
   tgtaactgaa tgttttaaaa acagaacttg tcactttata taaaagaata gtatgctcta 4860
   tttcctgaat ggatgtggaa atgaaagcta gcgcacctgc actttgaatt cttgcttctt 4920
   ttttattact gttatgattt tgctttttac agatgttgga cgattttttc ttctgattgt 4980
   tgaattcata atcatggtct catttccttt gcttctttgg aatatttctt tcaacacatt 5040
   cctttatttt attatacatt gtgtcctttt tttagctatt gctgctgttg ttttttattc 5100
   tatttacagg atgattttta aactgtcaaa tgaagtagtg ttaacctcaa ataggctaaa 5160
   tgtgaacaaa taaaatacag caaatactca g 5191
<210> 4
   <211> 5521
   <212> DNA
   <213> Homo sapiens
<400> 4
   cgccccgcat cgtgcccggc cccgtcgcgg agatcccgga cgaccgtcgc gggttgatgg     60
   tcgcattcca gatgtaaaca gcttcagaag cctgacggtc atatggtaga atcactgtgg    120
   actgagaccc acctttctag acctgaagcc caggaggagg aagaggaggc tggttggtac    180
   catgggcata atgctctgaa tcctagtctc tcacctagta tgtgagcagt ccctgcagat 240
   ggcccatttg gagatcttga caaagcctct tctgtttcca atggggtttt tggcgcattc 300
   tcacagactt agatgaaact gtgatggcca ccgcaggggg caggtgctga catcgtcccc 360
   agccctgtgg ctgttcatcc ggacatcatt tccaacctca atatctaaat gccacagtgc 420
   tcttggagca agttgggctg gggaccactg ttgcctttta agaccataaa accatgggaa 480
   acgcagaaag tcaacatgta gagcacgagt tttatggaga aaagcatgcc agcctggggc 540
   gcaacgacac ttcccgctcc ctgcgcctct cgcacaagac gcggaggacc aggcacgctt 600
   cctcggggaa ggtgatccac aggaactccg aagtgagcac ccgatccagc agcaccccca 660
   gcatccccca gtccctggct gaaaatggcc tggagccctt ctcccaagat ggtaccctag 720
   aagacttcgg gagccccatc tgggtggacc gagtggacat gggcttgaga cctgtgtctt 780
   acactgactc ttctgtcact cccagcgtag acagcagcat cgtcctcaca gcagcctctg 840
   tgcagagcat gccagacact gaggagagca ggctttacgg ggatgacgct acatatttgg 900
   ctgagggagg caggaggcag cattcctata catccaatgg gcccactttc atggagacgg 960
   cgagctttaa gaagaaacgc tccaaatctg cagacatctg gcgggaggac agcctggaat 1020
   tctcactctc tgatctgagc caagaacatt taacaagcaa cgaagaaatc ttgggttccg 1080
   ccgaagagaa ggactgcgag gaggctcggg ggatggaaac gcgggcgagt ccgcggcagc 1140
   tcagcacctg tcagagagcc aattccttgg gtgacttgta tgctcagaaa aactctggag 1200
   tgacagcaaa catggggccg gggagcaaat ttgcaggcta ctgtcggaat ttggtgtctg 1260
   atattcccaa tcttgcaaac cataagatgc caccagctgc tgctgaagag actcctccgt 1320
   acagtaatta taacacactt ccctgtagga aatctcactg tctctctgaa ggtgccacca 1380
   acccacaaat tagccatagc aacagcatgc aaggcagaag agctaaaaca actcaggatg 1440
   ttaatgcagg cgagggcagt gagtttgcag acagtgggat tgaaggggcc actaccgaca 1500
   cggacctcct gtccaggcga tctaatgcca ccaactccag ctactcaccc accacaggcc 1560
   gggcctttgt gggcagcgac agcggcagca gctccaccgg ggatgcggct cgtcaggggg 1620
   tgtacgagaa cttccggcgg gagctggaga tgagcaccac caacagcgag agcctggagg 1680
   aggccggctc tgcgcacagc gatgagcaga gcagcggcac cctgagctct ccgggccagt 1740
   cggacatcct gctgaccgcc gcacagggca cggtgcgcaa ggccggcgcc ctggccgtca 1800
   agaacttcct ggtgcacaag aagaacaaga aggtggagtc agccacccgg aggaagtgga 1860
   agcactactg ggtgtccctg aaaggatgca cgctattttt ctacgagagc gacggcaggt 1920
   ctgggataga ccacaacagc atccccaaac acgccgtctg ggtggagaac agcattgtgc 1980
   aggctgtgcc tgagcacccc aagaaggact ttgtcttctg cctcagcaat tccctgggtg 2040
   atgccttcct ttttcagacc actagccaga cggagcttga aaactggatc accgccatcc 2100
   actctgcctg cgccactgcg gtcgcgaggc accaccacaa ggaagacacg ctccgactcc 2160
   tgaaatcaga gatcaaaaaa ctggaacaga agattgacat ggatgaaaag atgaagaaaa 2220
   tgggtgaaat gcagctgtct tcagtcactg actcaaagaa aaagaaaaca atattagatc 2280
   agatctttgt ctgggagcaa aatctcgagc agttccaaat ggacctgttt cgtttccgct 2340
   gttatttagc cagccttcag ggtggggagc tgccaaaccc caaaaggctt ctcgcttttg 2400
   caagtcgacc aacgaaagtg gccatgggcc gccttggaat cttttcggta tcatcgtttc 2460
   atgccctggt ggcagcacgc actggtgaaa ctggagtgag aagacgtact caggccatgt 2520
   ccagatccgc gagcaagcga aggagcaggt tttcttctct gtggggtctg gatactacct 2580
   ccaaaaagaa gcagggacgg ccaagcatca atcaggtgtt tggagaggga accgaagctg 2640
   taaagaaatc tttagaggga atatttgatg acattgttcc agatggcaag agggagaaag 2700
   aagtggtctt acctaacgtt caccagcaca accctgactg cgacatttgg gtccacgagt 2760
   atttcactcc atcctggttc tgtctgccca ataatcagcc tgccctgacg gtcgtccggc 2820
   caggcgacac tgcacgggac accctggagc tgatttgcaa gacacatcaa ctggatcatt 2880
   ctgctcatta cctgcgcctg aaatttctaa tagaaaacaa aatgcagctc tatgttccac 2940
   agcccgagga agacatctat gagctgctgt acaaagaaat tgaaatctgt ccaaaagtca 3000
   ctcacagcat ccacattgag aagtcagata cagctgctga tacttacggg ttttcacttt 3060
   cttctgtgga agaagatggt attcgaaggc tgtacgtgaa tagtgtgaag gaaaccggtt 3120
   tagcttccaa gaaaggcctg aaagcaggag atgagattct tgagatcaat aatcgtgctg 3180
   ctgacgccct gaactcttct atgctcaaag atttcctctc acaaccctcg ctgggcctcc 3240
   tggtgaggac ctaccccgag ctggaggaag gagtggagct gctggaaagc ccgccccacc 3300
   gagtggacgg ccctgccgac cttgacgaga gccccctcgc ctttctcacc agcaacccag 3360
   ggcacagcct ttgcagcgag cagggcagca gtgctgagac cgctccagag gagaccgagg 3420
   ggccagactt ggaatcctca gatgagactg atcacagcag caagagtaca gaacaggtgg 3480
   ccgcattttg ccgcagtttg catgagatga acccctctga ccagaaccca tctcctcagg 3540
   actccacggg gcctcagctg gcgaccatga gacaactctc ggatgcagat aacgtgcgca 3600
   aggtgatctg cgagctcctg gagacggagc gcacctacgt gaaggattta aactgtctta 3660
   tggagagata cctaaagcct cttcaaaaag aaacttttct cacccaggat gagcttgacg 3720
   tgctttttgg aaatttaacg gaaatggtag agtttcaagt agaattcctt aaaactctag 3780
   aagatggagt gagactggta cctgatttgg aaaagcttga gaaggttgat caatttaaga 3840
   aagtgctgtt ctctctgggg ggatcattcc tgtattatgc tgaccgcttc aagctctaca 3900
   gtgccttctg cgccatccac acaaaagttc ccaaggtcct ggtgaaagcc aagacagaca 3960
   cggctttcaa ggcattcttg gatgcccaga acccgaagca gcagcactca tccacgctgg 4020
   agtcgtacct catcaagccc atccagagga tcctcaagta cccacttctg ctcagggagc 4080
   tgttcgccct gaccgatgcg gagagcgagg agcactacca cctggacgtg gccatcaaga 4140
   ccatgaacaa ggttgccagt cacatcaatg agatgcagaa aatccatgaa gagtttgggg 4200
   ctgtgtttga ccagctgatt gctgaacaga ctggtgagaa aaaagaggtt gcagatctga 4260
   gcatgggaga cctgcttttg cacactaccg tgatctggct gaacccgccg gcctcgctgg 4320
   gcaagtggaa aaaggaacca gagttggcag cattcgtctt caaaactgct gtggtccttg 4380
   tgtataaaga tggttccaaa cagaagaaga aacttgtagg atctcacagg ctttccattt 4440
   atgaggactg ggaccccttc agatttcgac acatgatccc cacggaagcg ctgcaggttc 4500
   gagctttggc gagtgcagat gcagaggcaa atgccgtgtg tgaaattgtc catgtaaaat 4560
   ccgagtctga agggaggccg gagagggtct ttcacttgtg ctgcagctcc ccagagagcc 4620
   gaaaggattt cctaaaggct gtgcattcaa tcctgcgtga taagcacaga agacagctcc 4680
   tcaaaaccga gagccttccc tcatcccagc aatatgtccc ttttggaggc aaaagattgt 4740
   gtgcactgaa gggggccagg ccggccatga gcagggcagt gtctgcccca agcaagtctc 4800
   ttgggaggag gaggcggcgg ctggctcgaa acaggtttac cattgattct gatgccgtct 4860
   ccgcaagcag cccggagaaa gagtcccagc agccccccgg tggtggggac actgaccgat 4920
   gggtagagga gcagtttgat cttgctcagt atgaggagca agatgacatc aaggagacag 4980
   acatcctcag tgacgatgat gagttctgtg agtccgtgaa gggtgcctca gtggacagag 5040
   acctgcagga gcggcttcag gccacctcca tcagtcagcg ggaaagaggc cggaaaaccc 5100
   tggatagtca cgcgtcccgc atggcacagc tcaagaagca agctgccctg tcggggatca 5160
   atggaggcct ggagagcgca agcgaggaag tcatttgggt taggcgtgaa gactttgccc 5220
   cctccaggaa actgaacact gagatctgac tgcgtcacct gccccgtaga gaatgtgtgt 5280
   agatacttcc tgccctaact ctgcccaccc tcctgtaccg tcgacaagaa tgtcccctta 5340
   ggtcgcgctc ttgcacacac ggttttggca gctgacttgg ttctgaagcc atgtagccac 5400
   ccaactttgt cattttcaac aacatcagaa agaattgatc agaatcccaa ataaaaccca 5460
   aaagtgtcta atgtattcat tcattagcta actaaaagcc caaaaaagac aagacaccca 5520
   g 5521
<210> 5
   <211> 1498
   <212> DNA
   <213> Homo sapiens
<400> 5
   accaaacctc ttcgaggcac aaggcacaac aggctgctct gggattctct tcagccaatc 60
   ttcattgctc aagtgtctga agcagccatg gcagaagtac ctgagctcgc cagtgaaatg 120
   atggcttatt acagtggcaa tgaggatgac ttgttctttg aagctgatgg ccctaaacag 180
   atgaagtgct ccttccagga cctggacctc tgccctctgg atggcggcat ccagctacga 240
   atctccgacc accactacag caagggcttc aggcaggccg cgtcagttgt tgtggccatg 300
   gacaagctga ggaagatgct ggttccctgc ccacagacct tccaggagaa tgacctgagc 360
   accttctttc ccttcatctt tgaagaagaa cctatcttct tcgacacatg ggataacgag 420
   gcttatgtgc acgatgcacc tgtacgatca ctgaactgca cgctccggga ctcacagcaa 480
   aaaagcttgg tgatgtctgg tccatatgaa ctgaaagctc tccacctcca gggacaggat 540
   atggagcaac aagtggtgtt ctccatgtcc tttgtacaag gagaagaaag taatgacaaa 600
   atacctgtgg ccttgggcct caaggaaaag aatctgtacc tgtcctgcgt gttgaaagat 660
   gataagccca ctctacagct ggagagtgta gatcccaaaa attacccaaa gaagaagatg 720
   gaaaagcgat ttgtcttcaa caagatagaa atcaataaca agctggaatt tgagtctgcc 780
   cagttcccca actggtacat cagcacctct caagcagaaa acatgcccgt cttcctggga 840
   gggaccaaag gcggccagga tataactgac ttcaccatgc aatttgtgtc ttcctaaaga 900
   gagctgtacc cagagagtcc tgtgctgaat gtggactcaa tccctagggc tggcagaaag 960
   ggaacagaaa ggtttttgag tacggctata gcctggactt tcctgttgtc tacaccaatg 1020
   cccaactgcc tgccttaggg tagtgctaag aggatctcct gtccatcagc caggacagtc 1080
   agctctctcc tttcagggcc aatccccagc ccttttgttg agccaggcct ctctcacctc 1140
   tcctactcac ttaaagcccg cctgacagaa accacggcca catttggttc taagaaaccc 1200
   tctgtcattc gctcccacat tctgatgagc aaccgcttcc ctatttattt atttatttgt 1260
   ttgtttgttt tattcattgg tctaatttat tcaaaggggg caagaagtag cagtgtctgt 1320
   aaaagagcct agtttttaat agctatggaa tcaattcaat ttggactggt gtgctctctt 1380
   taaatcaagt cctttaatta agactgaaaa tatataagct cagattattt aaatgggaat 1440
   atttataaat gagcaaatat catactgttc aatggttctg aaataaactt cactgaag 1498
<210> 6
   <211> 1245
   <212> DNA
   <213> Homo sapiens
<400> 6
   cgggcgtgta acccacgggt gcgcgcccac gaccgccaga ctcgagcagt ctctggaaca      60
   cgctgcgggg ctcccgggcc tgagccaggt ctgttctcca cgcaggtgtt ccgcgcgccc     120
   cgttcagcca tgtcgtccgg catccatgta gcgctggtga ctggaggcaa caagggcatc     180
   ggcttggcca tcgtgcgcga cctgtgccgg ctgttctcgg gggacgtggt gctcacggcg     240
   cgggacgtga cgcggggcca ggcggccgta cagcagctgc aggcggaggg cctgagcccg     300
   cgcttccacc agctggacat cgacgatctg cagagcatcc gcgccctgcg cgacttcctg     360
   cgcaaggagt acgggggcct ggacgtgctg gtcaacaacg cgggcatcgc cttcaaggtt     420
   gctgatccca caccctttca tattcaagct gaagtgacga tgaaaacaaa tttctttggt     480
   acccgagatg tgtgcacaga attactccct ctaataaaac cccaagggag agtggtgaac     540
   gtatctagca tcatgagcgt cagagccctt aaaagctgca gcccagagct gcagcagaag     600
   ttccgcagtg agaccatcac tgaggaggag ctggtggggc tcatgaacaa gtttgtggag     660
   gatacaaaga agggagtgca ccagaaggag ggctggccca gcagcgcata cggggtgacg     720
   aagattggcg tcaccgttct gtccaggatc cacgccagga aactgagtga gcagaggaaa     780
   ggggacaaga tcctcctgaa tgcctgctgc ccagggtggg tgagaactga catggcggga     840
   cccaaggcca ccaagagccc agaagaaggt gcagagaccc ctgtgtactt ggcccttttg      900
   cccccagatg ctgagggtcc ccatggacaa tttgtttcag agaagagagt tgaacagtgg     960
   tgagctgggc tcacagctcc atccatgggc cccattttgt accttgtcct gagttggtcc    1020
   aaagggcatt tacaatgtca taaatatcct tatataagaa aaaaaatgat ctcttatcaa    1080
   ttagcactca ctaatgtact actaattgag caacctacgc actcagttga ctacgtaaat    1140
   ctgtcaggtc ttttgtgatt tcctctgatg caggagagga aaaattgtaa ttgatgaaaa    1200
   taatgaatga aaatcaacag atgaataaat ggttctttat aagtg                    1245
<210> 7
   <211> 2460
   <212> DNA
   <213> Homo sapiens
<400> 7
   gtgtggaaca tgccttcacc acctccagct tctgctgccg gaggctgcac ccacctgtgc 60
   ccatggcctg cacaggccca tcacttccta gcgccttcga cattctaggt gcagcaggcc 120
   aggacaagct cttgtatctg aagcacaaac tgaagacccc acgcccaggc tgccaggggc 180
   aggacctcct gcatgccatg gttctcctga agctgggcca ggaaactgag gccaggatct 240
   ctctagaggc attgaaggcc gatgcggtgg cccggctggt ggcccgccag tgggctggcg 300
   tggacagcac cgaggaccca gaggagcccc cagatgtgtc ctgggctgtg gcccgcttgt 360
   accacctgct ggctgaggag aagctgtgcc ccgcctcgct gcgggacgtg gcctaccagg 420
   aagccgtccg caccctcagc tccagggacg accaccggct gggggaactt caggatgagg 480
   cccgaaaccg gtgtgggtgg gacattgctg gggatccagg gagcatccgg acgctccagt 540
   ccaatctggg ctgcctccca ccatcctcgg ctttgccctc tgggaccagg agcctcccac 600
   gccccattga cggtgtttcg gactggagcc aagggtgctc cctgcgatcc actggcagcc 660
   ctgcctccct ggccagcaac ttggaaatca gccagtcccc taccatgccc ttcctcagcc 720
   tgcaccgcag cccacatggg cccagcaagc tctgtgacga cccccaggcc agcttggtgc 780
   ccgagcctgt ccccggtggc tgccaggagc ctgaggagat gagctggccg ccatcggggg 840
   agattgccag cccaccagag ctgccaagca gcccacctcc tgggcttccc gaagtggccc 900
   cagatgcaac ctccactggc ctccctgata cccccgcagc tccagaaacc agcaccaact 960
   acccagtgga gtgcaccgag ggtctgcagg gcccccagtc tctccccttg cctattctgg 1020
   agccggtcaa aaacccctgc tctgtcaaag accagacgcc actccaactt tctgtagaag 1080
   ataccacctc tccaaatacc aagccgtgcc cacctactcc caccacccca gaaacatccc 1140
   ctcctcctcc tcctcctcct ccttcatcta ctccttgttc agctcacctg accccctcct 1200
   ccctgttccc ttcctccctg gaatcatcat cggaacagaa attctataac tttgtgatcc 1260
   tccacgccag ggcagacgaa cacatcgccc tgcgggttcg ggagaagctg gaggcccttg 1320
   gcgtgcccga cggggccacc ttctgcgagg atttccaggt gccggggcgc ggggagctga 1380
   gctgcctgca ggacgccata gaccactcag ctttcatcat cctacttctc acctccaact 1440
   tcgactgtcg cctgagcctg caccaggtga accaagccat gatgagcaac ctcacgcgac 1500
   aggggtcgcc agactgtgtc atccccttcc tgcccctgga gagctccccg gcccagctca 1560
   gctccgacac ggccagcctg ctctccgggc tggtgcggct ggacgaacac tcccagatct 1620
   tcgccaggaa ggtggccaac accttcaagc cccacaggct tcaggcccga aaggccatgt 1680
   ggaggaagga acaggacacc cgagccctgc gggaacagag ccaacacctg gacggtgagc   1740
   ggatgcaggc ggcggcactg aacgcagcct actcagccta cctccagagc tacttgtcct   1800
   accaggcaca gatggagcag ctccaggtgg cttttgggag ccacatgtca tttgggactg   1860
   gggcgcccta tggggctcga atgccctttg ggggccaggt gcccctggga gccccgccac   1920
   cctttcccac ttggccgggg tgcccgcagc cgccacccct gcacgcatgg caggctggca   1980
   cccccccacc gccctcccca cagccagcag cctttccaca gtcactgccc ttcccgcagt   2040
   ccccagcctt ccctacggcc tcacccgcac cccctcagag cccagggctg caacccctca   2100
   ttatccacca cgcacagatg gtacagctgg ggctgaacaa ccacatgtgg aaccagagag   2160
   ggtcccaggc gcccgaggac aagacgcagg aggcagaatg accgcgtgtc cttgcctgac   2220
   cacctgggga acacccctgg acccaggcat cggccaggac cccatagagc accccggtct   2280
   gccctgtgcc ctgtggacag tggaagatga ggtcatctgc cactttcagg acattgtccg   2340
   ggagcccttc atttaggaca aaacgggcgc gatgatgccc tggctttcag ggtggtcaga   2400
   actggatacg gtgtttacaa ttccaatctc tctatttctg ggtgaagggt cttggtggtg   2460
<210> 8
   <211> 2650
   <212> DNA
   <213> Homo sapiens
<400> 8
   gccgcgctgg tggcggcggc gcgtcgttgc agttgcgcca tctgtcagga gcggagccgg     60
   cgaggagggg gctgccgcgg gcgaggagga ggggtcgccg cgagccgaag gccttcgaga    120
   cccgcccgcc gcccggcggc gagagtagag gcgaggttgt tgtgcgagcg gcgcgtcctc    180
   tcccgcccgg gcgcgccgcg cttctcccag cgcaccgagg accgcccggg cgcacacaaa    240
   gccgccgccc gcgccgcacc gcccggcggc cgccgcccgc gccagggagg gattcggccg    300
   ccgggccggg gacaccccgg cgccgccccc tcggtgctct cggaaggccc accggctccc    360
   gggcccgccg gggacccccc ggagccgcct cggccgcgcc ggaggagggc ggggagagga    420
   ccatgtgagt gggctccgga gcctcagcgc cgcgcagttt ttttgaagaa gcaggatgct    480
   gatctaaacg tggaaaaaga ccagtcctgc ctctgttgta gaagacatgt ggtgtatata    540
   aagtttgtga tcgttggcgg acattttgga atttagataa tgggctgtgt gcaatgtaag    600
   gataaagaag caacaaaact gacggaggag agggacggca gcctgaacca gagctctggg    660
   taccgctatg gcacagaccc cacccctcag cactacccca gcttcggtgt gacctccatc    720
   cccaactaca acaacttcca cgcagccggg ggccaaggac tcaccgtctt tggaggtgtg    780
   aactcttcgt ctcatacggg gaccttgcgt acgagaggag gaacaggagt gacactcttt    840
   gtggcccttt atgactatga agcacggaca gaagatgacc tgagttttca caaaggagaa    900
   aaatttcaaa tattgaacag ctcggaagga gattggtggg aagcccgctc cttgacaact    960
   ggagagacag gttacattcc cagcaattat gtggctccag ttgactctat ccaggcagaa   1020
   gagtggtact ttggaaaact tggccgaaaa gatgctgagc gacagctatt gtcctttgga   1080
   aacccaagag gtacctttct tatccgcgag agtgaaacca ccaaaggtgc ctattcactt   1140
   tctatccgtg attgggatga tatgaaagga gaccatgtca aacattataa aattcgcaaa 1200
   cttgacaatg gtggatacta cattaccacc cgggcccagt ttgaaacact tcagcagctt 1260
   gtacaacatt actcagagag agctgcaggt ctctgctgcc gcctagtagt tccctgtcac 1320
   aaagggatgc caaggcttac cgatctgtct gtcaaaacca aagatgtctg ggaaatccct 1380
   cgagaatccc tgcagttgat caagagactg ggaaatgggc agtttgggga agtatggatg 1440
   ggtacctgga atggaaacac aaaagtagcc ataaagactc ttaaaccagg cacaatgtcc 1500
   cccgaatcat tccttgagga agcgcagatc atgaagaagc tgaagcacga caagctggtc 1560
   cagctctatg cagtggtgtc tgaggagccc atctacatcg tcaccgagta tatgaacaaa 1620
   ggaagtttac tggatttctt aaaagatgga gaaggaagag ctctgaaatt accaaatctt 1680
   gtggacatgg cagcacaggt ggctgcagga atggcttaca tcgagcgcat gaattatatc 1740
   catagagatc tgcgatcagc aaacattcta gtggggaatg gactcatatg caagattgct 1800
   gacttcggat tggcccgatt gatagaagac aatgagtaca cagcaagaca aggtgcaaag 1860
   ttccccatca agtggacggc ccccgaggca gccctgtacg ggaggttcac aatcaagtct 1920
   gacgtgtggt cttttggaat cttactcaca gagctggtca ccaaaggaag agtgccatac 1980
   ccaggcatga acaaccggga ggtgctggag caggtggagc gaggctacag gatgccctgc 2040
   ccgcaggact gccccatctc tctgcatgag ctcatgatcc actgctggaa aaaggaccct 2100
   gaagaacgcc ccacttttga gtacttgcag agcttcctgg aagactactt taccgcgaca 2160
   gagccccagt accaacctgg tgaaaacctg taaggcccgg gtctgcggag agaggccttg 2220
   tcccagaggc tgccccaccc ctccccatta gctttcaatt ccgtagccag ctgctcccca 2280
   gcagcggaac cgcccaggat cagattgcat gtgactctga agctgacgaa cttccatggc 2340
   cctcattaat gacacttgtc cccaaatccg aacctcctct gtgaagcatt cgagacagaa 2400
   ccttgttatt tctcagactt tggaaaatgc attgtatcga tgttatgtaa aaggccaaac 2460
   ctctgttcag tgtaaatagt tactccagtg ccaacaatcc tagtgctttc cttttttaaa 2520
   aatgcaaatc ctatgtgatt ttaactctgt cttcacctga ttcaactaaa aaaaaaaaag 2580
   tattattttc caaaagtggc ctctttgtct aaaacaataa aatttttttt catgttttaa 2640
   caaaaaccaa 2650
<210> 9
   <211> 3726
   <212> DNA
   <213> Homo sapiens
<400> 9
   cagcgctgct ccccgggcgc tcctccccgg gcgctcctcc ccaggcctcc cgggcgcttg      60
   gatcccggcc atctccgcac ccttcaagtg ggtgtgggtg atttcctggc ggggggagca    120
   gccaggtgag cccaagatgc tgctgcgctc gaagcctgcg ctgccgccgc cgctgatgct    180
   gctgctcctg gggccgctgg gtcccctctc ccctggcgcc ctgccccgac ctgcgcaagc     240
   acaggacgtc gtggacctgg acttcttcac ccaggagccg ctgcacctgg tgagcccctc    300
   gttcctgtcc gtcaccattg acgccaacct ggccacggac ccgcggttcc tcatcctcct     360
   gggttctcca aagcttcgta ccttggccag aggcttgtct cctgcgtacc tgaggtttgg     420
   tggcaccaag acagacttcc taattttcga tcccaagaag gaatcaacct ttgaagagag     480
   aagttactgg caatctcaag tcaaccagga tatttgcaaa tatggatcca tccctcctga     540
   tgtggaggag aagttacggt tggaatggcc ctaccaggag caattgctac tccgagaaca     600
   ctaccagaaa aagttcaaga acagcaccta ctcaagaagc tctgtagatg tgctatacac     660
   ttttgcaaac tgctcaggac tggacttgat ctttggccta aatgcgttat taagaacagc     720
   agatttgcag tggaacagtt ctaatgctca gttgctcctg gactactgct cttccaaggg     780
   gtataacatt tcttgggaac taggcaatga acctaacagt ttccttaaga aggctgatat     840
   tttcatcaat gggtcgcagt taggagaaga ttttattcaa ttgcataaac ttctaagaaa     900
   gtccaccttc aaaaatgcaa aactctatgg tcctgatgtt ggtcagcctc gaagaaagac     960
   ggctaagatg ctgaagagct tcctgaaggc tggtggagaa gtgattgatt cagttacatg    1020
   gcatcactac tatttgaatg gacggactgc taccagggaa gattttctaa accctgatgt    1080
   attggacatt tttatttcat ctgtgcaaaa agttttccag gtggttgaga gcaccaggcc    1140
   tggcaagaag gtctggttag gagaaacaag ctctgcatat ggaggcggag cgcccttgct    1200
   atccgacacc tttgcagctg gctttatgtg gctggataaa ttgggcctgt cagcccgaat    1260
   gggaatagaa gtggtgatga ggcaagtatt ctttggagca ggaaactacc atttagtgga    1320
   tgaaaacttc gatcctttac ctgattattg gctatctctt ctgttcaaga aattggtggg    1380
   caccaaggtg ttaatggcaa gcgtgcaagg ttcaaagaga aggaagcttc gagtatacct    1440
   tcattgcaca aacactgaca atccaaggta taaagaagga gatttaactc tgtatgccat    1500
   aaacctccat aatgtcacca agtacttgcg gttaccctat cctttttcta acaagcaagt    1560
   ggataaatac cttctaagac ctttgggacc tcatggatta ctttccaaat ctgtccaact    1620
   caatggtcta actctaaaga tggtggatga tcaaaccttg ccacctttaa tggaaaaacc    1680
   tctccggcca ggaagttcac tgggcttgcc agctttctca tatagttttt ttgtgataag    1740
   aaatgccaaa gttgctgctt gcatctgaaa ataaaatata ctagtcctga cactgaattt    1800
   ttcaagtata ctaagagtaa agcaactcaa gttataggaa aggaagcaga taccttgcaa    1860
   agcaactagt gggtgcttga gagacactgg gacactgtca gtgctagatt tagcacagta    1920
   ttttgatctc gctaggtaga acactgctaa taataatagc taataatacc ttgttccaaa    1980
   tactgcttag cattttgcat gttttacttt tatctaaagt tttgttttgt tttattattt    2040
   atttatttat ttattttgtg acggagagag attccatctc aaaaaaacaa gttattaaaa    2100
   atgtatatga atgctcctaa tatggtcagg aagcaaggaa gcgaaggata tattatgagt    2160
   tttaagaagg tgcttagctg tatatttatc tttcaaaatg tattagaaga ttttagaatt    2220
   ctttccttca tgtgccatct ctacaggcac ccatcagaaa aagcatactg ccgttaccgt    2280
   gaaactggtt gtaaaagaga aactatctat ttgcacctta aaagacagct agattttgct    2340
   gattttcttc tttcggtttt ctttgtcagc aataatatgt gagaggacag attgttagat    2400
   atgatagtat aaaaaatggt taatgacaat tcagaggcga ggagattctg taaacttaaa 2460
   attactataa atgaaattga tttgtcaaga ggataaattt tagaaaacac ccaatacctt 2520
   ataactgtct gttaatgctt gctttttctc tacctttctt ccttgtttca gttgggaagc 2580
   ttttggctgc aagtaacaga aactcctaat tcaaatggct taagcaataa ggaaatgtat 2640
   attcccacat aactagacgt tcaaacaggc caggctccag cacttcagta cgtcaccagg 2700
   ggatctgggt tcttcccagc tctctgctct gccatcttta gcgctggctt cattctcaga 2760
   ctctggtagc atgatggctg tagctgtttc atgggcccct tcaaacctca tagcaaccag 2820
   aggaagaaaa tgagccattt tttgagtctc cttcatagac ttgaataact ctttttcaga 2880
   gcttctcaca gcaaacctct cctcatgtct cctcatgtct tattgttcag aaatgggtaa 2940
   tgtggccatt tcaccagtca ctgccaacaa caacgaggtt cctataattg tctctgagta 3000
   accctttgga atggagaggg tgttggtcag tctacaaact gaacactgca gttctgcgct 3060
   ttttaccagt gaaaaaatgt aattattttc ccctcttaag gattaatatt cttcaaatgt 3120
   atgcctgtta tggatatagt atctttaaaa ttttttattt taatagcttt aggggtacac 3180
   actttttgct tacaggggtg aattgtgtag tggtgaagac tcggctttta atgtacttgt 3240
   cacctgagtg atgtacattg tacccaatag gtaatttttc atccattacc ctccttccgc 3300
   cctcttccct tctgagtctc caacatccct tataccactg tgtatgttct tgtgtaccta 3360
   cagctaagct tccacttata agtgagaaca tgcagtattt ggttttccat tcctgagtta 3420
   cttcccttag gataacagcc cccagttccg tccaagttgc tgcaaaatac attattcttc 3480
   tttatggctg agtaatagtc catggtacat atataccaca ttttctttat ccacttatca 3540
   gttgatggac acttaggtta attccattca atttcattca atttaagtat atttgtaagg 3600
   agctaaagct gaaaattaaa ttttagatct ttcaatactc ttaaatttta tatgtaagtg 3660
   gtttttatat tttcacattt gaaataaagt aatttttata accttgaaaa aaaaaaaaaa 3720
   aaaaaa 3726
<210> 10
   <211> 4912
   <212> DNA
   <213> Homo sapiens
<400> 10
   attggggtct gctctaagct gcagcaagag aaactgtgtg tgaggggaag aggcctgttt 60
   cgctgtcggg tctctagttc ttgcacgctc tttaagagtc tgcactggag gaactcctgc 120
   cattaccagc tcccttcttg cagaagggag ggggaaacat acatttattc atgccagtct 180
   gttgcatgca ggctttttgg cttcctacct tgcaacaaaa taattgcacc aactccttag 240
   tgccgattcc gcccacagag agtcctggag ccacagtctt ttttgctttg cattgtagga 300
   gagggactaa gtgctagaga ctatgtcgct ttcctgagct accgagagcg ctcgtgaact 360
   ggaatcaact gcttcaggga aaaagaaaaa aaaaaaaaaa agacttgcct gggaggccgc 420
   gagaaacttg cattggaagc ttcagcaacc agcattcgag aaactcctct ctactttagc 480
   acggtctcca gactcagccg agagacagca aactgcagcg cggtgagaga gcgagagaga 540
   gggagagaga gactctccag cctgggaact ataactcctc tgcgagaggc ggagaactcc 600
   ttccccaaat cttttgggga cttttctctc tttacccacc tccgcccctg cgaggagttg 660
   aggggccagt tcggccgccg cgcgcgtctt cccgttcggc gtgtgcttgg cccggggaac 720
   cgggagggcc cggcgatcgc gcggcggccg ccgcgagggt gtgagcgcgc gtgggcgccc 780
   gccgagccga ggccatggtg cagcaaacca acaatgccga gaacacggaa gcgctgctgg 840
   ccggcgagag ctcggactcg ggcgccggcc tcgagctggg aatcgcctcc tcccccacgc 900
   ccggctccac cgcctccacg ggcggcaagg ccgacgaccc gagctggtgc aagaccccga 960
   gtgggcacat caagcgaccc atgaacgcct tcatggtgtg gtcgcagatc gagcggcgca 1020
   agatcatgga gcagtcgccc gacatgcaca acgccgagat ctccaagcgg ctgggcaaac 1080
   gctggaagct gctcaaagac agcgacaaga tccctttcat tcgagaggcg gagcggctgc 1140
   gcctcaagca catggctgac taccccgact acaagtaccg gcccaggaag aaggtgaagt 1200
   ccggcaacgc caactccagc tcctcggccg ccgcctcctc caagccgggg gagaagggag 1260
   acaaggtcgg tggcagtggc gggggcggcc atgggggcgg cggcggcggc gggagcagca 1320
   acgcgggggg aggaggcggc ggtgcgagtg gcggcggcgc caactccaaa ccggcgcaga 1380
   aaaagagctg cggctccaaa gtggcgggcg gcgcgggcgg tggggttagc aaaccgcacg 1440
   ccaagctcat cctggcaggc ggcggcggcg gcgggaaagc agcggctgcc gccgccgcct 1500
   ccttcgccgc cgaacaggcg ggggccgccg ccctgctgcc cctgggcgcc gccgccgacc 1560
   accactcgct gtacaaggcg cggactccca gcgcctcggc ctccgcctcc tcggcagcct 1620
   cggcctccgc agcgctcgcg gccccgggca agcacctggc ggagaagaag gtgaagcgcg 1680
   tctacctgtt cggcggcctg ggcacgtcgt cgtcgcccgt gggcggcgtg ggcgcgggag 1740
   ccgaccccag cgaccccctg ggcctgtacg aggaggaggg cgcgggctgc tcgcccgacg 1800
   cgcccagcct gagcggccgc agcagcgccg cctcgtcccc cgccgccggc cgctcgcccg 1860
   ccgaccaccg cggctacgcc agcctgcgcg ccgcctcgcc cgccccgtcc agcgcgccct 1920
   cgcacgcgtc ctcctcggcc tcgtcccact cctcctcttc ctcctcctcg ggctcctcgt 1980
   cctccgacga cgagttcgaa gacgacctgc tcgacctgaa ccccagctca aactttgaga 2040
   gcatgtccct gggcagcttc agttcgtcgt cggcgctcga ccgggacctg gattttaact 2100
   tcgagcccgg ctccggctcg cacttcgagt tcccggacta ctgcacgccc gaggtgagcg 2160
   agatgatctc gggagactgg ctcgagtcca gcatctccaa cctggttttc acctactgaa 2220
   gggcgcgcag gcagggagaa gggccggggg gggtaggaga ggagaaaaaa aaagtgaaaa 2280
   aaagaaacga aaaggacaga cgaagagttt aaagagaaaa gggaaaaaag aaagaaaaag 2340
   taagcagggc tggcttcgcc cgcgttctcg tcgtcggatc aaggagcgcg gcggcgtttt 2400
   ggacccgcgc tcccatcccc caccttcccg ggccggggac ccactctgcc cagccggagg 2460
   gacgcggagg aggaagaggg tagacagggg cgacctgtga ttgttgttat tgatgttgtt 2520
   gttgatggca aaaaaaaaaa agcgacttcg agtttgctcc cctttgcttg aagagacccc 2580
   ctcccccttc caacgagctt ccggacttgt ctgcaccccc agcaagaagg cgagttagtt 2640
   ttctagagac ttgaaggagt ctcccccttc ctgcatcacc accttggttt tgttttattt 2700
   tgcttcttgg tcaagaaagg aggggagaac ccagcgcacc cctccccccc tttttttaaa 2760
   cgcgtgatga agacagaagg ctccggggtg acgaatttgg ccgatggcag atgttttggg 2820
   ggaacgccgg gactgagaga ctccacgcag gcgaattccc gtttggggct tttttttcct 2880
   ccctcttttc cccttgcccc ctctgcagcc ggaggaggag atgttgaggg gaggaggcca 2940
   gccagtgtga ccggcgctag gaaatgaccc gagaaccccg ttggaagcgc agcagcggga 3000
   gctaggggcg ggggcggagg aggacacgaa ctggaagggg gttcacggtc aaactgaaat 3060
   ggatttgcac gttggggagc tggcggcggc ggctgctggg cctccgcctt cttttctacg 3120
   tgaaatcagt gaggtgagac ttcccagacc ccggaggcgt ggaggagagg agactgtttg 3180
   atgtggtaca ggggcagtca gtggagggcg agtggtttcg gaaaaaaaaa aagaaaaaaa 3240
   gaaaaaaaaa gaaaaaaaaa agattttttt cttctcttaa tcggaatcgt gatggtgttg 3300
   gattatttca atggtggggt taatatagca tgttatcctg tctatctttt aaagatttct 3360
   gtataagact gttgagcagt ttttaaaata gtgtaggata atataaaaag cagatagatg 3420
   gcgctatgtt tgattcctac aacgaaatta tcaccagctt tttttcattc ttaactcttt 3480
   aaaggattca aacgcaactc aaatctgtgc tggactttaa aaaaacaatt caggaccaaa 3540
   ttttttctca gtgtgtgtgt ttattcctta taggtgtaaa tgagaagacg tgtttttttc 3600
   cttcaccgat gctccatcct cgtatttctt tttccttgta aatgtaatca gatgccattt 3660
   tatatgtgga cgtatttata ctggccaaac atattttttc ttttgtccct ttttttcttt 3720
   cctttctttt tacttccttt atttctttat tccttccttt tccttttttt cttttttttt 3780
   tctttttttt tttttttttt tggtagttgt tgttacccac gccattttac gtctccttca 3840
   ctgaagggct agagttttaa cttttaattt tttatattta aatgtagact tttgacactt 3900
   ttaaaaaaca aaaaaagaca agagagatga aaacgtttga ttattttctc agtgtatttt 3960
   tgtaaaaaat atataaaggg ggtgttaatc ggtgtaaatc gctgtttgga tttcctgatt 4020
   ttataacagg gcggctggtt aatatctcac acagtttaaa aaatcagccc ctaatttctc 4080
   catgtttaca cttcaatctg caggcttctt aaagtgacag tatcccttaa cctgccacca 4140
   gtgtccaccc tccggccccc gtcttgtaaa aaggggagga gaattagcca aacactgtaa 4200
   gcttttaaga aaaacaaagt tttaaacgaa atactgctct gtccagaggc tttaaaactg 4260
   gtgcaattac agcaaaaagg gattctgtag ctttaacttg taaaccacat cttttttgca 4320
   ctttttttat aagcaaaaac gtgccgttta aaccactgga tctatctaaa tgccgatttg 4380
   agttcgcgac actatgtact gcgtttttca ttcttgtatt tgactattta atcctttcta 4440
   cttgtcgcta aatataattg ttttagtctt atggcatgat gatagcatat gtgttcaggt 4500
   ttatagctgt tgtgtttaaa aattgaaaaa agtggaaaac atctttgtac atttaagtct 4560
   gtattataat aagcaaaaag attgtgtgta tgtatgttta atataacatg acaggcacta 4620
   ggacgtctgc ctttttaagg cagttccgtt aagggttttt gtttttaaac ttttttttgc 4680
   catccatcct gtgcaatatg ccgtgtagaa tatttgtctt aaaattcaag gccacaaaaa 4740
   caatgtttgg gggaaaaaaa agaaaaaatc atgccagcta atcatgtcaa gttcactgcc 4800
   tgtcagattg ttgatatata ccttctgtaa ataacttttt ttgagaagga aataaaatca 4860
   gctggaactg aaccctaaaa aaaaaaaaaa aaaaaaaaaa aaaaaaaaaa aa 4912
<210> 11
   <211> 4045
   <212> DNA
   <213> Homo sapiens
<400> 11
   gcagccagag ctcagcaggg ccctggagag atggccacgg tcccagcacc ggggaggact 60
   ggagagcgcg cgctgccacc gccccatgtc tcagccaggg cttccttcct cggctccacc 120
   ctgtggatgt aatggcggcc cctgctctgt cctggcgtct gcccctcctc atcctcctcc 180
   tgcccctggc tacctcttgg gcatctgcag cggtgaatgg cacttcccag ttcacatgct 240
   tctacaactc gagagccaac atctcctgtg tctggagcca agatggggct ctgcaggaca 300
   cttcctgcca agtccatgcc tggccggaca gacggcggtg gaaccaaacc tgtgagctgc 360
   tccccgtgag tcaagcatcc tgggcctgca acctgatcct cggagcccca gattctcaga 420
   aactgaccac agttgacatc gtcaccctga gggtgctgtg ccgtgagggg gtgcgatgga 480
   gggtgatggc catccaggac ttcaagccct ttgagaacct tcgcctgatg gcccccatct 540
   ccctccaagt tgtccacgtg gagacccaca gatgcaacat aagctgggaa atctcccaag 600
   cctcccacta ctttgaaaga cacctggagt tcgaggcccg gacgctgtcc ccaggccaca 660
   cctgggagga ggcccccctg ctgactctca agcagaagca ggaatggatc tgcctggaga 720
   cgctcacccc agacacccag tatgagtttc aggtgcgggt caagcctctg caaggcgagt 780
   tcacgacctg gagcccctgg agccagcccc tggccttcag gacaaagcct gcagcccttg 840
   ggaaggacac cattccgtgg ctcggccacc tcctcgtggg cctcagcggg gcttttggct 900
   tcatcatctt agtgtacttg ctgatcaact gcaggaacac cgggccatgg ctgaagaagg 960
   tcctgaagtg taacacccca gacccctcga agttcttttc ccagctgagc tcagagcatg 1020
   gaggagacgt ccagaagtgg ctctcttcgc ccttcccctc atcgtccttc agccctggcg 1080
   gcctggcacc tgagatctcg ccactagaag tgctggagag ggacaaggtg acgcagctgc 1140
   tcctgcagca ggacaaggtg cctgagcccg catccttaag cagcaaccac tcgctgacca 1200
   gctgcttcac caaccagggt tacttcttct tccacctccc ggatgccttg gagatagagg 1260
   cctgccaggt gtactttact tacgacccct actcagagga agaccctgat gagggtgtgg 1320
   ccggggcacc cacagggtct tccccccaac ccctgcagcc tctgtcaggg gaggacgacg 1380
   cctactgcac cttcccctcc agggatgacc tgctgctctt ctcccccagt ctcctcggtg 1440
   gccccagccc cccaagcact gcccctgggg gcagtggggc cggtgaagag aggatgcccc 1500
   cttctttgca agaaagagtc cccagagact gggaccccca gcccctgggg cctcccaccc 1560
   caggagtccc agacctggtg gattttcagc caccccctga gctggtgctg cgagaggctg 1620
   gggaggaggt ccctgacgct ggccccaggg agggagtcag tttcccctgg tccaggcctc 1680
   ctgggcaggg ggagttcagg gcccttaatg ctcgcctgcc cctgaacact gatgcctact 1740
   tgtccctcca agaactccag ggtcaggacc caactcactt ggtgtagaca gatggccagg 1800
   gtgggaggca ggcagctgcc tgctctgcgc cgagcctcag aaggaccctg ttgagggtcc 1860
   tcagtccact gctgaggaca ctcagtgtcc agttgcagct ggacttctcc acccggatgg 1920
   cccccaccca gtcctgcaca cttggtccat ccatttccaa acctccactg ctgctcccgg 1980
   gtcctgctgc ccgagccagg aactgtgtgt gttgcagggg ggcagtaact ccccaactcc 2040
   ctcgttaatc acaggatccc acgaatttag gctcagaagc atcgctcctc tccagccctg 2100
   cagctattca ccaatatcag tcctcgcggc tctccagggc tccctgccct gacctcttcc 2160
   ctgggttttc tgccccagcc tcctccttcc ctcccctccc cgtccacagg gcagcctgag 2220
   cgtgctttcc aaaacccaaa tatggccacg ctccccctcg gttcaaaacc ttgcacaggt 2280
   cccactgccc tcagccccac ttctcagcct ggtacttgta cctccggtgt cgtgtgggga 2340
   catccccttc tgcaatcctc cctaccgtcc tcctgagcca ctcagagctc cctcacaccc 2400
   cctctgttgc acatgctatt ccctggggct gctgtgcgct ccccctcatc taggtgacaa 2460
   acttccctga ctcttcaagt gccggttttg cttctcctgg agggaagcac tgcctccctt 2520
   aatctgccag aaacttctag cgtcagtgct ggagggagaa gctgtcaggg acccagggcg 2580
   cctggagaaa gaggccctgt tactattcct ttgggatctc tgaggcctca gagtgcttgg 2640
   ctgctgtatc tttaatgctg gggcccaagt aagggcacag atccccccac aaagtggatg 2700
   cctgctgcat cttcccacag tggcttcaca gacccacaag agaagctgat ggggagtaaa 2760
   ccctggagtc cgaggcccag gcagcagccc cgcctagtgg tgggccctga tgctgccagg 2820
   cctgggacct cccactgccc cctccactgg aggggtctcc tctgcagctc agggactggc 2880
   acactggcct ccagaagggc agctccacag ggcagggcct cattattttt cactgcccca 2940
   gacacagtgc ccaacacccc gtcgtatacc ctggatgaac gaattaatta cctggcacca 3000
   cctcgtctgg gctccctgcg cctgacattc acacagagag gcagagtccc gtgcccatta 3060
   ggtctggcat gccccctcct gcaaggggct caacccccta ccccgacccc tccacgtatc 3120
   tttcctaggc agatcacgtt gcaatggctc aaacaacatt ccaccccagc aggacagtga 3180
   ccccagtccc agctaactct gacctgggag ccctcaggca cctgcactta caggccttgc 3240
   tcacagctga ttgggcacct gaccacacgc ccccacaggc tctgaccagc agcctatgag 3300
   ggggtttggc accaagctct gtccaatcag gtaggctggg cctgaactag ccaatcagat 3360
   caactctgtc ttgggcgttt gaactcaggg agggaggccc ttgggagcag gtgcttgtgg 3420
   acaaggctcc acaagcgttg agccttggaa aggtagacaa gcgttgagcc actaagcaga 3480
   ggaccttggg ttcccaatac aaaaatacct actgctgaga gggctgctga ccatttggtc 3540
   aggattcctg ttgcctttat atccaaaata aactcccctt tcttgaggtt gtctgagtct 3600
   tgggtctatg ccttgaaaaa agctgaatta ttggacagtc tcacctcctg ccatagggtc 3660
   ctgaatgttt cagaccacaa ggggctccac acctttgctg tgtgttctgg ggcaacctac 3720
   taatcctctc tgcaagtcgg tctccttatc cccccaaatg gaaattgtat ttgccttctc 3780
   cactttggga ggctcccact tcttgggagg gttacatttt ttaagtctta atcatttgtg   3840
   acatatgtat ctatacatcc gtatctttta atgatccgtg tgtaccatct ttgtgattat   3900
   ttccttaata ttttttcttt aagtcagttc attttcgttg aaatacattt atttaaagaa   3960
   aaatctttgt tactctgtaa atgaaaaaac ccattttcgc tataaataaa aggtaactgt   4020
   acaaaataag tacaatgcaa caaaa                                         4045
<210> 12
   <211> 3033
   <212> DNA
   <213> Homo sapiens
<400> 12
   gcgggggcgc gtcggggctg gagccggagc gcgccgggcg ctgggcgcag cgagcgagag     60
   cgcggcggcc gcgggctccg gcgagggaca gacgcaccga tcgccggagg gacagacaca    120
   cgaccacgcg gcgccaccgc ccacgcctcc acccaccggc gcccaagtcc tccccgcgcc    180
   gcctcctctg tatggcacaa actttcctcc cgggacggaa cacgctgcct cagggagccc    240
   gcgaccgcgc cttctcctcc gccggtccca tacgctgctg aaatgggttg cggattgaac    300
   aagttagaga aacgtgatga aaaacggcct gggaatattt attcaacttt gaagaggcct    360
   caggtggaaa ccaagataga tgtgtcctat gaataccgct tcctggagtt cacgactctg    420
   agtgctgcgg agctccctgg gtcctcagca gtgaggctgg cctccctgcg tgacctgccc    480
   gcccagctcc tggagctgta ccagcagggc ttctcgctgg cggccctgca ccccttcgtg   540
   cagcccaccc atgagcggga gaagacgccc ctggagcaca tctttagagc catcctgatc    600
   aagaaaaccg acagatctca gaaaactgat cttcacaatg aaggctacat cttggaatta    660
   gattgctgtt cctccttaga ccacccgaca gaccagaaac tcatcccaga gttcattaag    720
   aagatccagg aggctgcaag ccagggcctg aaattcgttg gtgttatacc tcagtaccat    780
   tcctctgtga actcggcagg cagcagtgct ccggtgtcta ctgccaacag caccgaggat    840
   gccagagatg caaaaaacgc acgtggggat cacgcgtcac tggagaatga gaaaccgggg    900
   actggggatg tgtgcagtgc tccggctggg agaaaccaaa gcccagagcc cagctcaggc    960
   cccagagggg aggtgcccct cgccaagcag cccagctcac cctccggaga gggagatggt   1020
   ggagaacttt caccacaggg ggtgagcaag acactggatg gaccggagag caaccccttg   1080
   gaggtgcatg aagagccact ctcagggaaa atggagatct tcaccctttt caacaaaccg   1140
   aagagccatc agaagtgccg gcaatactac cctgtcacca ttcctctcca tgtctccaag   1200
   aatggccaga cagtgagcgg tttggacgcc aactggttag agcacatgag cgaccacttc   1260
   cggaaaggag gcatgctggt gaacgcagtc ttctaccttg gaatagtgaa tgattcctta   1320
   catggcttga cagatggagt attcatcttt gaagctgttt ccacagaaga tagcaaaacc   1380
   atacagggct atgatgctat tgtggttgaa caatggacag tcctggaagg tgtcgaagtg   1440
   cagacagact acgtgcccct gctgaactcg ctggcggcct atggctggca gctcacctgt   1500
   gtgctaccaa ctcccgtcgt caagactacc agcgagggga gtgtatccac caagcagatt   1560
   gtctttcttc agagaccttg tctacctcag aaaatcaaga agaaggaatc gaagtttcag   1620
   tggcgattct ccagagaaga aatgcacaac aggcagatga ggaaatcaaa aggtaaactc 1680
   agtgccagag acaaacaaca agcagaagaa aatgagaaga acttagaaga ccagtcttcc 1740
   aaagctggag acatgggaaa ctgtgtttca ggacagcagc aggagggtgg agtctccgag 1800
   gagatgaagg gccctgtcca agaggacaag ggagaacagc tgtcccctgg tggcctgctg 1860
   tgtggggtgg gtgtggaggg tgaggctgtg cagaatggtc ctgccagcca cagcagggcc 1920
   ctggtgggga tttgcactgg gcactccaat cctggagagg atgccaggga cggggatgct 1980
   gaggaagtca gagagcttgg tacggttgaa gaaaactgag tcttgggcaa tttgtgctaa 2040
   aactaggtga gttgccaaac ccaaggcatc ttaccaacag ctggtttggg ggctggtttc 2100
   cctggtgttg tgtgttacct accctttggc ttggcttgac ctctccttgt gagctcacct 2160
   gagccctccc agggccaggt tcctgacagt gttggttttt gcacatccac tggaaaggtg 2220
   tcattaatga cccagtgtta gaatgcaaga ggtcaggtta ttctagccct catggctgaa 2280
   ggcccagtcc tggctccacc actcctccag ccagagggtc tggaccatcc agtgcctgtc 2340
   ctcgccacag ggcctccagg gagcattcgg gtcaaatcca tggacaccct gggctacaaa 2400
   ccaaggctgc tgttcatccc acatcgtgtg gggcagtgtc catcccctgc agctacttgg 2460
   tgacttaaca actccaggag ccctgtcagc tgccctcctc cacctaaacc ccttcgactc 2520
   ttctgctttg acaaagaaaa tgacattggg gaggggaggt gctccgcctc ccagcttttc 2580
   tcaaaatagt cctatagata ctggtaatct ggaaatgaag aagtaattct gtctctgcac 2640
   ctacttttgc agaatgttca aggaagtatt ctgtgttagt attaatgcca aaaagttgtt 2700
   tttaaaggtt ttgtactcag cacatcatac aaaccacatt acttctgtca cttcagggca 2760
   tcgggactgg ctggcgccct tgttatgtgc tattttaatc agtgtaacat tggtcaagtt 2820
   gttacccatg tatgctgtgt ttatcatgtg tatatcgtcc agaaagtatt aaggctttag 2880
   gtagatgcaa ctggcgaacc ttggagaggg aatgctgatt gtcttgacca aacccacagc 2940
   ctgtctcttc tcttgtttag ttacttacgg caataaatca tctatgagtt agtgcaccgt 3000
   gaaaaaaaaa aaaaaaaaaa aaaaaaaaaa aaa 3033
<210> 13
   <211> 3146
   <212> DNA
   <213> Homo sapiens
<400> 13
   gccgcaaccc gtcccggagg tgtcctgtct cctgtcgccg ccgccgccgc caccaccgct     60
   gccactgccg ccctgccggg gccatgttcg ctctgggctt gcccttcttg gtgctcttgg    120
   tggcctcggt cgagagccat ctgggggttc tggggcccaa gaacgtctcg cagaaagacg    180
   ccgagtttga gcgcacctac gtggacgagg tcaacagcga gctggtcaac atctacacct    240
   tcaaccatac tgtgacccgc aacaggacag agggcgtgcg tgtgtctgtg aacgtcctga    300
   acaagcagaa gggggcgccg ttgctgtttg tggtccgcca gaaggaggct gtggtgtcct    360
   tccaggtgcc cctaatcctg cgagggatgt ttcagcgcaa gtacctctac caaaaagtgg    420
   aacgaaccct gtgtcagccc cccaccaaga atgagtcgga gattcagttc ttctacgtgg 480
   atgtgtccac cctgtcacca gtcaacacca cataccagct ccgggtcagc cgcatggacg 540
   attttgtgct caggactggg gagcagttca gcttcaatac cacagcagca cagccccagt 600
   acttcaagta tgagttccct gaaggcgtgg actcggtaat tgtcaaggtg acctccaaca 660
   aggccttccc ctgctcagtc atctccattc aggatgtgct gtgtcctgtc tatgacctgg 720
   acaacaacgt agccttcatc ggcatgtacc agacgatgac caagaaggcg gccatcaccg 780
   tacagcgcaa agacttcccc agcaacagct tttatgtggt ggtggtggtg aagaccgaag 840
   accaagcctg cgggggctcc ctgcctttct accccttcgc agaagatgaa ccggtcgatc 900
   aagggcaccg ccagaaaacc ctgtcagtgc tggtgtctca agcagtcacg tctgaggcat 960
   acgtcagtgg gatgctcttt tgcctgggta tatttctctc cttttacctg ctgaccgtcc 1020
   tcctggcctg ctgggagaac tggaggcaga agaagaagac cctgctggtg gccattgacc 1080
   gagcctgccc agaaagcggt caccctcgag tcctggctga ttcttttcct ggcagttccc 1140
   cttatgaggg ttacaactat ggctcctttg agaatgtttc tggatctacc gatggtctgg 1200
   ttgacagcgc tggcactggg gacctctctt acggttacca ggggcacgac cagttcaagc 1260
   ggcgcctccc ctctggccag atgcggcagc tgtgcattgc catgggccgc tcctttgaac 1320
   ctgtaggtac tcggccccga gtggactcca tgagctctgt ggaggaggat gactacgaca 1380
   cattgaccga catcgattcc gacaagaatg tcattcgcac caagcaatac ctctatgtgg 1440
   ctgacctggc acggaaggac aagcgtgttc tgcggaaaaa gtaccagatc tacttctgga 1500
   acattgccac cattgctgtc ttctatgccc ttcctgtggt gcagctggtg atcacctacc 1560
   agacggtggt gaatgtcaca gggaatcagg acatctgcta ctacaacttc ctctgcgccc 1620
   acccactggg caatctcagc gccttcaaca acatcctcag caacctgggg tacatcctgc 1680
   tggggctgct tttcctgctc atcatcctgc aacgggagat caaccacaac cgggccctgc 1740
   tgcgcaatga cctctgtgcc ctggaatgtg ggatccccaa acactttggg cttttctacg 1800
   ccatgggcac agccctgatg atggaggggc tgctcagtgc ttgctatcat gtgtgcccca 1860
   actataccaa tttccagttt gacacatcgt tcatgtacat gatcgccgga ctctgcatgc 1920
   tgaagctcta ccagaagcgg cacccggaca tcaacgccag cgcctacagt gcctacgcct 1980
   gcctggccat tgtcatcttc ttctctgtgc tgggcgtggt ctttggcaaa gggaacacgg 2040
   cgttctggat cgtcttctcc atcattcaca tcatcgccac cctgctcctc agcacgcagc 2100
   tctattacat gggccggtgg aaactggact cggggatctt ccgccgcatc ctccacgtgc 2160
   tctacacaga ctgcatccgg cagtgcagcg ggccgctcta cgtggaccgc atggtgctgc 2220
   tggtcatggg caacgtcatc aactggtcgc tggctgccta tgggcttatc atgcgcccca 2280
   atgatttcgc ttcctacttg ttggccattg gcatctgcaa cctgctcctt tacttcgcct 2340
   tctacatcat catgaagctc cggagtgggg agaggatcaa gctcatcccc ctgctctgca 2400
   tcgtttgcac ctccgtggtc tggggcttcg cgctcttctt cttcttccag ggactcagca 2460
   cctggcagaa aacccctgca gagtcgaggg agcacaaccg ggactgcatc ctcctcgact 2520
   tctttgacga ccacgacatc tggcacttcc tctcctccat cgccatgttc gggtccttcc    2580
   tggtaagcgg gcctcccggc gcagcgttga ggataacgtg aaaggtagca gctgcctcct    2640
   tctctgtgag ctgatctggc gtccacaccc caggtgttag ctgacactgg atgacgacct    2700
   ggatacttag aaaggggctt caggaaggga tgtgctgttt ccctctacgt gcccagtcct    2760
   agcctcgctc taggacccag ggctggcttc taagtttccg tccagtcttc aggcaagttc    2820
   tgtgttagtc atgcacacac atacctatga aaccttgaag tttacaaaga attgccccag    2880
   ctctgggcac cctggccacc ctggtccttg gatccccttc gtcccacctg gtccacccca    2940
   gatgctgagg atgggggagc tcaggcgggg cctctgcttt ggggatggga atgtgttttt    3000
   ctcccaaact tgtttttata gctctgcttg aagggctggg agatgaggtg ggtctggatc    3060
   ttttctcaga gcgtctccat gctatggttg catttccgtt ttctatgaat gaatttgcat    3120
   acaataacca accagactca gtaaaa                                         3146
<210> 14
   <211> 1558
   <212> DNA
   <213> Homo sapiens
<400> 14
   cggaggagag cgcaggagga aacagtaccg gctggaggcc ggtcttgcag gagcggggga     60
   ctgctggggg cggggcttgg tggtgaccgc tggcggggcg gggcctgggg ctcagagggg    120
   tgggctttgg agatcagagg gtcgacgctg cttcgttgcc tggactctgg tttccgccct    180
   ggagcaagcc ggggcctggt cggcagctgg gccgccatgg agtccacgct gggcgcgggc    240
   atcgtgatag ccgaggcgct acagaaccag ctagcctggc tggagaacgt gtggctctgg    300
   atcacctttc tgggcgatcc caagatcctc tttctgttct acttccccgc ggcctactac    360
   gcctcccgcc gtgtgggcat cgcggtgctc tggatcagcc tcatcaccga gtggctcaac    420
   ctcatcttca agtggtttct ttttggagac aggccctttt ggtgggtcca tgagtctggt    480
   tactacagcc aggctccagc ccaggttcac cagttcccct cttcttgtga gactggtcca    540
   ggcagccctt ctggacactg catgatcaca ggagcagccc tctggcccat aatgacggcc    600
   ctgtcttcgc aggtggccac tcgggcccgc agccgctggg taagggtgat gcctagcctg    660
   gcttattgca ccttcctttt ggcggttggc ttgtcgcgaa tcttcatctt agcacatttc    720
   cctcaccagg tgctggctgg cctaataact ggcgctgtcc tgggctggct gatgactccc    780
   cgagtgccta tggagcggga gctaagcttc tatgggttga ctgcactggc cctcatgcta    840
   ggcaccagcc tcatctattg gaccctcttt acactgggcc tggatctttc ttggtccatc    900
   agcctagcct tcaagtggtg tgagcggcct gagtggatac acgtggatag ccggcccttt    960
   gcctccctga gccgtgactc aggggctgcc ctgggcctgg gcattgcctt gcactctccc   1020
   tgctatgccc aggtgcgtcg ggcacagctg ggaaatggcc agaagatagc ctgccttgtg   1080
   ctggccatgg ggctgctggg ccccctggac tggctgggcc acccccctca gatcagcctc   1140
   ttctacattt tcaatttcct caagtacacc ctctggccat gcctagtcct ggccctcgtg   1200
   ccctgggcag tgcacatgtt cagtgcccag gaagcaccgc ccatccactc ttcctgactt   1260
   cttgtgtgcc tccctttcct ttccctccca caaagccaac actctgtgac caccacactc   1320
   caggaggcag ccccatcccc ttccagcccc taagtaggcc ctcccctccc taaatctgct   1380
   tccgcaccac ctggtcttag ccccaaagat gggccttctc tctcccagat aagttggtcc   1440
   tccctctgcc tttcctctca agcccccaaa gagcaaaggc aacagcaaga ccagcgggtt   1500
   cttgcaacac tgtgaggggc agccagggcg gccccaataa agcccttgaa tactttga      1558
<210> 15
   <211> 5388
   <212> DNA
   <213> Homo sapiens
<400> 15
   gcaacagcgg cggcggccgc ggcggctggc cggactcagg tgtttcggac gctattgccc     60
   ttcgcgccag ccgtcgagtg ggcagcagcg ggactcagcc gggcgccagg ttcctgccag    120
   gcagcgccgg gaagcgcggg cggccgagaa ctccttcctg ctacttcgcc cagcgccgct    180
   gcttcggctt cccagcgaag tgggagacct tcctccctgt ttgcagacgt ccgtgggaga    240
   cccttatttt ttccaccgct aaggttaaga gattctggaa tagaagcgtc gaaggagatc    300
   aagtgaacct tctacaactc ctcggatgtc gccagtctcc ctttcggggc ggaagactac    360
   gtttgagcat ctcactgagg tgcaggaatg gaagaaccca ccttgcagct tttctgcagt    420
   gtggcttgcc tgatctaccc ctaggaatga agaggaggct tgtaataatc cgatgaagta    480
   cagatgttga agaggatatc gcaggaccta aacttgtgat cgtttggggg aggtcacaca    540
   cgtttctgag tgggaatgga tgggcgtgaa tgacgtgccc tcttaaaaag cacaacagtc    600
   ctttaagagg agcaaaattg agttttccca ttttggccaa gattttgaag acagttcaat    660
   gtattctaca tttgacataa gatgagaact ttctaaagta ttctctccaa gagcgtaaac    720
   gatgactacc ccagccctgc tgcccctctc tggacgtagg ataccacctc tgaacctggg    780
   gccgccttcc ttcccacatc acagggctac cttgagactt tctgagaagt ttattcttct    840
   ccttattctt agtgccttca tcactctgtg ttttggggca ttctttttcc ttccagactc    900
   ttcaaaacac aaacgctttg atttgggttt agaagatgtg ttaattccac atgtagatgc    960
   cggtaaaggg gctaaaaacc ccggagtctt cctgatccat ggacccgatg aacatagaca   1020
   cagggaagag gaagaacgtc tgagaaataa aattcgagct gatcatgaga aggccttgga   1080
   agaagcaaaa gaaaaattaa gaaagtcaag agaggaaatt cgagcagaaa ttcagacaga   1140
   gaaaaataag gtagtccaag aaatgaagat aaaagagaac aagccactgc caccagtccc   1200
   tattcccaac cttgtaggaa tacgtggtgg agacccagaa gataatgaca taagagagaa   1260
   aagggaaaaa attaaagaga tgatgaaaca tgcttgggat aactatagga catatgggtg   1320
   gggacataat gaactcagac ctattgcaag gaaaggacac tcccctaaca tatttggaag   1380
   ttcacaaatg ggtgctacca tagtagatgc tttggatacc ctttatatca tgggacttca   1440
   tgatgaattc ctagatgggc aaagatggat tgaagacaac cttgatttca gtgtgaattc   1500
   agaggtgtct gtgtttgaag tcaacattcg atttattgga ggcctacttg cagcatatta   1560
   cctatcagga gaggagatat tcaagattaa agcagtgcaa ttggctgaga aactccttcc 1620
   tgcctttaac acacctactg ggattccttg ggcaatggtg aatttgaaaa gtggagtagg 1680
   gcgaaactgg ggctgggcat ctgcaggtag cagcattctg gctgaatttg gtacactaca 1740
   tatggagttc atccacctca gctacttgac aggggacctg acttactaca aaaaggttat 1800
   gcacattcgg aaactacttc agaaaatgga tcgtccaaat ggtctttatc caaattattt 1860
   gaaccccaga acagggcgct ggggtcagta tcatacatct gtcggtggcc tgggagacag 1920
   tttttatgaa tacttactga aagcatggtt gatgtcagat aaaacagacc atgaggcaag 1980
   aaagatgtat gatgatgcta ttgaggctat agaaaaacat cttattaaga agtctcgtgg 2040
   aggtcttacc tttattggag aatggaagaa tgggcacttg gaaaaaaaga tggggcattt 2100
   ggcctgcttt gctgggggaa tgtttgcact aggagcagat ggttccagag cagataaagc 2160
   tggtcattat ttagagctag gggcagaaat tgcacgtact tgtcatgagt catatgacag 2220
   aactgcatta aagctaggtc ctgaatcatt caagtttgat ggtgcagtgg aggctgtggc 2280
   tgtccggcag gctgaaaagt attatatcct ccgtccagaa gtaattgaaa cctattggta 2340
   cctatggcga ttcactcacg atccaagata caggcagtgg ggctgggaag cagcactggc 2400
   cattgaaaag tattgccgag ttaatggtgg gttttctgga gtcaaagatg tatattcctc 2460
   tactcctaca catgatgatg tacagcagag ctttttcttt gctgaaacat taaaatattt 2520
   gtatctgctg ttctccggtg atgacctttt acctttagac cactgggtgt ttaatacaga 2580
   ggctcaccct ctgcctgtgt tacatttagc caacaccaca ctttcaggta atcctgctgt 2640
   tcgatgaaag cagttccaga aggaccattc tcacctgtgt tttgtttaca tggaccacta 2700
   cagaaattag tttgaagggg cggcttttga aaacctggac ctctatgtca acatgacagg 2760
   gtgaaactat tccccctaag actgttcaac ttgtagatac atcaactttg aaattattcc 2820
   attttatacc tgaccaaaac atgttctgat atgtgtagga cagagacctg gatgtgcttt 2880
   gatcgttaat gaggtggtca catgagaaat gatacctgtt actactgtat tgtttttaga 2940
   gtcctgaagt ctggaggcta gacttcctga aagcaagtca agaatataga gcaccttgca 3000
   ggagttcaag atggcctttg gaaccaatta tgtatttgtt tcctcctaca gtggagcagc 3060
   attcaaatca aatatttaca tattgcttat cactttttct ccattttaat aatggaatga 3120
   actaaaataa acaagaacaa aagaatagta taattatatc agtaacaaga agactcaaaa 3180
   aagaaacagg agtacctatc cctatctgaa ttttcaagtt ccccattgga tgaccagact 3240
   ggcaaccatt tcaaatccca gtctatttca ttgaaatttc ttggttaagt ttaattttct 3300
   ctgggggcat gatctcacaa agaatactca agtctttttc ttcttatgga atcatcgaaa 3360
   ctgctattta tcataatcac cacttatgag cctgggtttg ggattttgtg catgtagttc 3420
   agtctagtgt tggtagcatg acagaaagtg gggaaaatgc cgcagtttgt tgccttgaaa 3480
   cctaagagca atccttggtt ttgttgctac attatttttc cagaccaaca catctaccaa 3540
   gtaaatttta ttcactttaa tttcataata aagttagtag agtcactcaa cttacaactt 3600
   tatttatgtg gcttggcaaa aatcactata aggcagctct aaatttgcct tgataagcta 3660
   aataaattac ttttataact tactaaagca gaacaaacag tgaaactttc taaaatattc 3720
   tatctggaat agggacaggg gatcttttat ttataatctc atcagatgag tgagttgttc 3780
   acagatattt tatgtttttt taattttctc caagaatatt tatagaattc caaagaatca 3840
   gaatagtttc aaaataattt tcagtgataa aagagtgttg taattaatca tattacacta 3900
   aaattgggat acatctaagg aactttatct tactatcagt aggttttgca ttgatatttc 3960
   tttttaaata aactactagt tctttatatt ttgacaaaaa gaacttaaat tttatcagga 4020
   actgtaagat aaatatctag tgcttataaa ttttctgtcc ttaaatttat gtgacagtgc 4080
   aagatacttt tgctcttttc atttaatata ggcatcttcc attgacatta ataaaactta 4140
   gaaacagtat aattagtata acatttactc tgaatttgaa gatttcctga aacaaagttt 4200
   gtacaagaag cccaccttgg aattctgaag gcttattttc ttgtttgata agcttttctt 4260
   ttaaacttag gttttaagtt ggggaaagac ttaattaact aatatagtat tttctaaggt 4320
   tgatcatctt ataccacgaa tcgttaattt tgacagttct actgatccgt aaatgataac 4380
   cactgcaaat tttttcagta taaaattttt cactgcaaaa aaatttcagt agaaaataag 4440
   gatgcagggc cagttacaat agtccttaag agagttaaat tatagcacat gttttgacat 4500
   tgtaatatct tttactactt gaacatttaa atttctaaat gagaaaggta tatatattac 4560
   tgtaactgta gaagggaaaa gggaaagtat ttggttctaa aaaatgttag ccttcctcgt 4620
   aaaagtagca caagcccact tatgaatcac tgagaaaaag tgaaaaactt gagttggcaa 4680
   agatgcagag cagcagtgca gatggcaatg aactctctga attctctttt accttattta 4740
   gaagaatgca gagtaaaggg accttcttgg ttctgcagga acttctcaag ggatgaggag 4800
   acagaacccc tacttccaag tgctctattt gtattaccca gatgactgaa gcttaagaga 4860
   aggcagggaa gtatacaagc agagccagtt ctggtacaaa caaagaattt gacagggaca 4920
   atggaagggt cttcttcacc actccttacc ttctatgtga tggaaagact agagcttata 4980
   aaagtacttc cattttttta ttctcctgaa taccaaaggc aattaaagtc agctacaaat 5040
   gacttgccag tgtcatgttt tatttttgtt atagattttt aaattatttc cttcaagatc 5100
   aattcttatc ccatataatg cttagcttcc aagaatattc tttactttct tctgtctttt 5160
   acagctcttt gcattttgta gaccttaata ctcaggttaa atattcattg catttataag 5220
   atcttctgca aaaagcccag aaatggtcct tttcaggtgc ctcttcaaag agctgacacc 5280
   ttaccttgtg cctttggcac aaatgtgcag aatagataca tcagttggtg cataatcgaa 5340
   aaaaatagga attttgaaca ctgttcttcc ttctacattt atttctct 5388
<210> 16
   <211> 2678
   <212> DNA
   <213> Homo sapiens
<400> 16
   atgcgacccg accgcgctga ggctccagga ccgcccgcca tggctgcagg aggtcccggc 60
   gcggggtctg cggccccggt ctcctccaca tcctcccttc ccctggctgc tctcaacatg 120
   cgagtgcggc gccgcctgtc tctgttcttg aacgtgcgga cacaggtggc ggccgactgg 180
   accgcgctgg cggaggagat ggactttgag tacttggaga tccggcaact ggagacacaa 240
   gcggacccca ctggcaggct gctggacgcc tggcagggac gccctggcgc ctctgtaggc 300
   cgactgctcg agctgcttac caagctgggc cgcgacgacg tgctgctgga gctgggaccc 360
   agcattgagg aggattgcca aaagtatatc ttgaagcagc agcaggagga ggctgagaag 420
   cctttacagg tggccgctgt agacagcagt gtcccacgga cagcagagct ggcgggcatc     480
   accacacttg atgaccccct ggggcatatg cctgagcgtt tcgatgcctt catctgctat     540
   tgccccagcg acatccagtt tgtgcaggag atgatccggc aactggaaca gacaaactat 600
   cgactgaagt tgtgtgtgtc tgaccgcgat gtcctgcctg gcacctgtgt ctggtctatt 660
   gctagtgagc tcatcgaaaa gaggtgccgc cggatggtgg tggttgtctc tgatgattac 720
   ctgcagagca aggaatgtga cttccagacc aaatttgcac tcagcctctc tccaggtgcc 780
   catcagaagc gactgatccc catcaagtac aaggcaatga agaaagagtt ccccagcatc 840
   ctgaggttca tcactgtctg cgactacacc aacccctgca ccaaatcttg gttctggact 900
   cgccttgcca aggccttgtc cctgccctga agactgttct gaggccctgg gtgtgtgtgt 960
   atctgtctgc ctgtccatgt acttctgccc tgcctcctcc tttcgttgta ggaggaatct 1020
   gtgctctact tacctctcaa ttcctggaga tgccaacttc acagacacgt ctgcagcagc 1080
   tggacatcac atttcatgtc ctgcatggaa ccagtggctg tgagtggcat gtccacttgc 1140
   tggattatca gccaggacac tatagaacag gaccagctga gactaagaag gaccagcaga 1200
   gccagctcag ctctgagcca ttcacacatc ttcaccctca gtttcctcac ttgaggagtg 1260
   ggatggggag aacagagagt agctgtgttt gaatccctgt aggaaatggt gaagcatagc 1320
   tctgggtctc ctgggggaga ccaggcttgg ctgcgggaga gctggctgtt gctggactac 1380
   atgctggcca ctgctgtgac cacgacactg ctggggcagc ttcttccaca gtgatgccta 1440
   ctgatgcttc agtgcctctg cacaccgccc attccacttc ctccttcccc acagggcagg 1500
   tggggaagca gtttggccca gcccaaggag accccatctt gagccttatt tcctaatggg 1560
   tccacctctc atctgcatct ttcacacctc ccagcttctg cccaaccttc agcagtgaca 1620
   agtccccaag agactcgcct gagcagcttg ggctgctttt catttccacc tgtcaggatg 1680
   cctgtggtca tgctctcagc tccacctggc atgagaaggg atcctggcct ctggcatatt 1740
   catcaagtat gagttctggg gatgagtcac tgtaatgatg tgagcaggga gccttcctcc 1800
   ctgggccacc tgcagagagc tttcccacca actttgtacc ttgattgcct tacaaagtta 1860
   tttgtttaca aacagcgacc atataaaagc ctcctgcccc aaagcttgtg ggcacatggg 1920
   cacatacaga ctcacataca gacacacaca tatatgtaca gacatgtact ctcacacaca 1980
   caggcaccag catacacacg tttttctagg tacagctccc aggaacagct aggtgggaaa 2040
   gtcccatcac tgagggagcc taaccatgtc cctgaacaaa aattgggcac tcatctattc 2100
   cttttctctt gtgtccctac tcattgaaac caaactctgg aaaggaccca atgtaccagt 2160
   atttatacct ctaatgaagc acagagagag gaagagagct gcttaaactc acacaacaat 2220
   gaactgcaga cacagctgtt ctctccctct ctccttccca gagcaattta tactttaccc 2280
   tcaggctgtc ctctggggag aaggtgccat ggtcttaggt gtctgtgccc caggacagac 2340
   cctaggaccc taaatccaat agaaaatgca tatctttgct ccactttcag ccaggctgga 2400
   gcaaggtacc ttttcttagg atcttgggag ggaatggatg cccctctctg catgatcttg 2460
   ttgaggcatt tagctgccat gcacctgtcc ccctttaata ctgggcattt taaagccatc 2520
   tcaagaggca tcttctacat gttttgtacg cattaaaata atttcaaaga tatctgagaa 2580
   aagccgatat ttgccattct tcctatatcc tggaatatat cttgcatcct gagtttataa 2640
   taataaataa tattctacct tggaaaaaaa aaaaaaaa 2678
<210> 17
   <211> 950
   <212> DNA
   <213> Homo sapiens
<400> 17
   gggaccttaa ttctctttcc catcttgcaa gatggcgggt gaaaaagttg agaagccaga 60
   tactaaagag aagaaacccg aagccaagaa ggttgatgct ggtggcaagg tgaaaaaggg 120
   taacctcaaa gctaaaaagc ccaagaaggg gaagccccat tgcagccgca accctgtcct 180
   tgtcagagga attggcaggt attcccgatc tgccatgtat tccagaaagg ccatgtacaa 240
   gaggaagtac tcagccgcta aatccaaggt tgaaaagaaa aagaaggaga aggttctcgc 300
   aactgttaca aaaccagttg gtggtgacaa gaacggcggt acccgggtgg ttaaacttcg 360
   caaaatgcct agatattatc ctactgaaga tgtgcctcga aagctgttga gccacggcaa 420
   aaaacccttc agtcagcacg tgagaaaact gcgagccagc attacccccg ggaccattct 480
   gatcatcctc actggacgcc acaggggcaa gagggtggtt ttcctgaagc agctggctag 540
   tggcttatta cttgtgactg gacctctggt cctcaatcga gttcctctac gaagaacaca 600
   ccagaaattt gtcattgcca cttcaaccaa aatcgatatc agcaatgtaa aaatcccaaa 660
   acatcttact gatgcttact tcaagaagaa gaagctgcgg aagcccagac accaggaagg 720
   tgagatcttc gacacagaaa aagagaaata tgagattacg gagcagcgca agattgatca 780
   gaaagctgtg gactcacaaa ttttaccaaa aatcaaagct attcctcagc tccagggcta 840
   cctgcgatct gtgtttgctc tgacgaatgg aatttatcct cacaaattgg tgttctaaat 900
   gtcttaagaa cctaattaaa tagctgacta caaaaaaaaa aaaaaaaaaa 950
<210> 18
   <211> 700
   <212> DNA
   <213> Homo sapiens
<400> 18
   gcggcgtgag aagccatgag cagcaaagtc tctcgcgaca ccctgtacga ggcggtgcgg 60
   gaagtcctgc acgggaacca gcgcaagcgc cgcaagttcc tggagacggt ggagttgcag 120
   atcagcttga agaactatga tccccagaag gacaagcgct tctcgggcac cgtcaggctt 180
   aagtccactc cccgccctaa gttctctgtg tgtgtcctgg gggaccagca gcactgtgac 240
   gaggctaagg ccgtggatat cccccacatg gacatcgagg cgctgaaaaa actcaacaag 300
   aataaaaaac tggtcaagaa gctggccaag aagtatgatg cgtttttggc ctcagagtct 360
   ctgatcaagc agattccacg aatcctcggc ccaggtttaa ataaggcagg aaagttccct 420
   tccctgctca cacacaacga aaacatggtg gccaaagtgg atgaggtgaa gtccacaatc 480
   aagttccaaa tgaagaaggt gttatgtctg gctgtagctg ttggtcacgt gaagatgaca 540
   gacgatgagc ttgtgtataa cattcacctg gctgtcaact tcttggtgtc attgctcaag 600
   aaaaactggc agaatgtccg ggccttatat atcaagagca ccatgggcaa gccccagcgc 660
   ctatattaag gcacatttga ataaattcta ttaccagttc 700
<210> 19
   <211> 1125
   <212> DNA
   <213> Homo sapiens
<400> 19
   cccccatgtg acagtgacgg ggtccccgct ccaggagacg ctcgagtctg cgtcccggcc      60
   ctcagcactg tccactgttt cggtgccagc agagaccagc aggcccggga cagttggtgt     120
   ttggccgtgc cgctgtctaa cttggtgtgc agagtgaatt gccgctgccg gagcggagag     180
   aggcggagcg gccaggagag aggggatttc tgtcagcgcc ggcctcggga gctcggagac     240
   atgaacggct tcacgcctga cgagatgagc cgcggcgggg atgcggccgc cgcagtggcc     300
   gcagtggtcg ctgccgcggc cgccgccgcc tcggcgggga acgggaccgg cgcgggcacc     360
   ggggctgagg tgccgggcgc gggggcggtc tcagcggctg ggcccccggg ggcggccggg     420
   ccgggccccg ggcaactgtg ctgcctgcgg gaggatggtg agcggtgcgg ccgggcggca     480
   ggcaacgcca gcttcagcaa gaggatccag aagagcatct cccagaagaa ggtgaagatc     540
   gagctggata agagcgcaag gcatctttac atatgtgatt atcataaaaa cttaattcag     600
   agtgttcgaa acagaagaaa gagaaaaggg agtgatgatg atggaggtga ttcacctgtt     660
   caagatattg ataccccaga ggttgattta taccaattac aagtaaatac acttaggaga     720
   tacaaaagac acttcaagct accaaccaga ccaggactta ataaagcaca acttgttgag     780
   atagttggtt gccactttag gtctattcca gtgaatgaaa aagacacctt aacatatttc     840
   atctactcag tgaagaatga caagaacaaa tcagatctca aggttgatag tggtgttcac     900
   taggagacgt ggaattgaga ctaataactt ggatgttaac actgtttact gttttttcac    960
   atgtagaaat gttctttgtg tattttttct acagaggatt ttctctgatt ttattttctt   1020
   tgtttctgac tctaataatt agttggaaac tcatataaaa tgagctttcc taaattaaat   1080
   ctattttaaa taaaggttat tactattaaa aaaaaaaaaa aaaaa                    1125
<210> 20
   <211> 3608
   <212> DNA
   <213> Homo sapiens
<400> 20
   gcggccgctt ccccccggcc gggcccccgc cgccccgcgg tccccagagc gccaggcccc      60
   cggggggagg gagggagggc gccgggccgg tgggagccag cggcgcgcgg tgggacccac     120
   ggagccccgc gacccgccga gcctggagcc gggccgggtc ggggaagccg gctccagccc 180
   ggagcgaact tcgcagcccg tcggggggcg gcggggaggg ggcccggagc cggaggaggg 240
   ggcggccgcg ggcacccccg cctgtgcccc ggcgtccccg ggcaccatgc tgtccaactc 300
   ccagggccag agcccgccgg tgccgttccc cgccccggcc ccgccgccgc agccccccac 360
   ccctgccctg ccgcaccccc cggcgcagcc gccgccgccg cccccgcagc agttcccgca 420
   gttccacgtc aagtccggcc tgcagatcaa gaagaacgcc atcatcgatg actacaaggt 480
   caccagccag gtcctggggc tgggcatcaa cggcaaagtt ttgcagatct tcaacaagag 540
   gacccaggag aaattcgccc tcaaaatgct tcaggactgc cccaaggccc gcagggaggt 600
   ggagctgcac tggcgggcct cccagtgccc gcacatcgta cggatcgtgg atgtgtacga 660
   gaatctgtac gcagggagga agtgcctgct gattgtcatg gaatgtttgg acggtggaga 720
   actctttagc cgaatccagg atcgaggaga ccaggcattc acagaaagag aagcatccga 780
   aatcatgaag agcatcggtg aggccatcca gtatctgcat tcaatcaaca ttgcccatcg 840
   ggatgtcaag cctgagaatc tcttatacac ctccaaaagg cccaacgcca tcctgaaact 900
   cactgacttt ggctttgcca aggaaaccac cagccacaac tctttgacca ctccttgtta 960
   tacaccgtac tatgtggctc cagaagtgct gggtccagag aagtatgaca agtcctgtga 1020
   catgtggtcc ctgggtgtca tcatgtacat cctgctgtgt gggtatcccc ccttctactc 1080
   caaccacggc cttgccatct ctccgggcat gaagactcgc atccgaatgg gccagtatga 1140
   atttcccaac ccagaatggt cagaagtatc agaggaagtg aagatgctca ttcggaatct 1200
   gctgaaaaca gagcccaccc agagaatgac catcaccgag tttatgaacc acccttggat 1260
   catgcaatca acaaaggtcc ctcaaacccc actgcacacc agccgggtcc tgaaggagga 1320
   caaggagcgg tgggaggatg tcaaggggtg tcttcatgac aagaacagcg accaggccac 1380
   ttggctgacc aggttgtgag cagaggattc tgtgttcctg tccaaactca gtgctgtttc 1440
   ttagaatcct tttattccct gggtctctaa tgggacctta aagaccatct ggtatcatct 1500
   tctcattttg cagaagagaa actgaggccc agaggcggag ggcagtctgc tcaaggtcac 1560
   gcagctggtg actggttggg gcagaccgga cccaggtttc ctgactcctg gcccaagtct 1620
   cttcctccta tcctgcggga tcactggggg gctctcaggg aacagcagca gtgccatagc 1680
   caggctctct gctgcccagc gctggggtga ggctgccgtt gtcagcgtgg accactaacc 1740
   agcccgtctt ctctctctgc tcccacccct gccgccctca ccctgccctt gttgtctctg 1800
   tctctcacgt ctctcttctg ctgtctctcc tacctgtctt ctggctctct ctgtaccctt 1860
   cctggtgctg ccgtgccccc aggaggagat gaccagtgcc ttggccacaa tgcgcgttga 1920
   ctacgagcag atcaagataa aaaagattga agatgcatcc aaccctctgc tgctgaagag 1980
   gcggaagaaa gctcgggccc tggaggctgc ggctctggcc cactgagcca ccgcgccctc 2040
   ctgcccacgg gaggacaagc aataactctc tacaggaata tattttttaa acgaagagac 2100
   agaactgtcc acatctgcct cctctcctcc tcagctgcat ggagcctgga actgcatcag 2160
   tgactgaatt ctgccttggt tctggccacc ccagagtggg agaggctggg aggttgggag 2220
   gctgtggaga gaagtgagca aggtgctctt gaacctgtgc tcattttgca attttatcag 2280
   taatttgact tagagttttt acgaaacctc ttttgttgtc cttgccccac tcctctccac 2340
   cagacgcctt cctctctgga tactgcaaag gcttgtggtt tgttagaggg tatttgtgga 2400
   aactgtcata gggattgtcc ctgtgttgtc ccatctgccc tccctgtttc tccacaacag 2460
   cctggggttg tccccgctgg ctcacgcgtt ctgggagctc aaggccacct tggaggagga 2520
   tgccacgcac ttcctctctc ggagccctca gacatctcca gtgtgccaga caaataggag 2580
   tgagtgtatg tgtgtgtgtg tgtgtgtgtg tgtgcacacg tgtgtatgag tgcgcagatc 2640
   tgtgcctggg atcgtgcatt tgaggggcca ggggcaggca gggctgcaga gggagacggc 2700
   cctgctgggg cttaggaacc ttctcccttc ttgggtctgc cctgcccata ctgagcctgc 2760
   caaagtgcct gggaagccca cccagattct gaaacaggcc ctctgtggcc tgtctctatt 2820
   agctgggttc cgggaggcag agaggagtga ccgggcactg gcactgcgat caggaagact 2880
   ggacccccag cccccagggc ccccctcccc ccacttagtg ctggtcctag gtcctctgag 2940
   gcactcatct actgaatgac ctctctactt ccccttcttg ccattattaa cccatttttg 3000
   tttattttcc ttaaattttt agccatttct ccatgggcca ccgcccagct catgtaggtg 3060
   agcctgggca gcttctgttg gcagagcttt tgcatttcct gtgtttgtcc tgggttctgg 3120
   ggcatcagcc agctacccct tgtgggcaaa ggcagggcca cttttgaagt cttccctcag 3180
   atttccattg tgtggcctgg tgggtcaggg ggagtctttg caccaaagat gtcctgactt 3240
   tgcccccttg cccatcagcc atttgccatc accccaaaca actcagcttc ggggccggtg 3300
   aggggagggg cctcccccag cacagatgag gagcagctgg ggtaggctgt ctgtgccatg 3360
   gccccccact cccccttccc ttggagggag aggtggcagg aatacttcac ctttcctctc 3420
   cctcaggggc aggtggtgga ggggcgccca gggtcgtctt tgtgtatggg ggaaggcgct 3480
   gggtgcctgc agcgcctccc ttgtctcaga tggtgtgtcc agcactcgat tgttgtaaac 3540
   tgttgttttg tatgagcgaa attgtcttta ctaaacagat ttaatagtta aaaaaaaaaa 3600
   aaaaaaaa 3608
<210> 21
   <211> 678
   <212> DNA
   <213> Homo sapiens
<400> 21
   ctcgcccaaa gaagactaca atctccaggg aaacctgggg cgtctcgcgc aaacgtccat 60
   aactgaaagt agctaaggca ccccagccgg aggaagtgag ctctcctggg gcgtggttgt 120
   tcgtgatcct tgcatctgtt acttagggtc aaggcttggg tcttgccccg cagacccttg 180
   ggacgacccg gccccagcgc agctatgaac ctggagcgag tgtccaatga ggagaaattg 240
   aacctgtgcc ggaagtacta cctggggggg tttgctttcc tgccttttct ctggttggtc 300
   aacatcttct ggttcttccg agaggccttc cttgtcccag cctacacaga acagagccaa 360
   atcaaaggct atgtctggcg ctcagctgtg ggcttcctct tctgggtgat agtgctcacc 420
   tcctggatca ccatcttcca gatctaccgg ccccgctggg gtgcccttgg ggactacctc 480
   tccttcacca tacccctggg caccccctga caacttctgc acatactggg gccctgctta 540
   ttctcccagg acaggctcct taaagcagag gagcctgtcc tgggagcccc ttctcaaact 600
   cctaagactt gttttcatgt cccacgttct ctgctgacat cccccaataa aggaccctaa 660
   ctttcaaaaa aaaaaaaa 678
<210> 22
   <211> 2272
   <212> DNA
   <213> Homo sapiens
<400> 22
   gaagcgactc tgagtcccgg gctcggagcg caggctcagc tccgcgctgc gagcgctacg 60
   ggcgcagggg cggggagccg gcccggagcg cagtttccag tggggccggg gtttcacccg 120
   ggccctctct gtttgaaccg aacccgacaa atgggcgcat gacgatggag agcagggaaa 180
   tggactgcta tctccgtcgc ctcaaacagg agctgatgtc catgaaggag gtgggtgatg 240
   gcttacagga tcagatgaac tgcatgatgg gtgcactgca agaactgaag ctcctccagg 300
   tgcagacagc actggaacag ctggagatct ctggaggggg tcctgtgcca ggcagccctg 360
   aaggtcccag gacccagtgc gagcaccctt gttgggaggg tggcagaggt cctgccaggc 420
   ccacagtctg ttccccctcc agtcaacctt ctcttggcag cagcaccaag tttccatccc 480
   ataggagtgt ctgtggaagg gatttagccc ccttgcccag gacacagcca catcaaagct 540
   gtgctcagca ggggccagag cgagtggaac cggatgactg gacctccacg ttgatgtccc 600
   ggggccggaa tcgacagcct ctggtgttag gggacaacgt ttttgcagac ctggtgggca 660
   attggctaga cttgccagaa ctggagaagg gtggggagaa gggtgagact gggggggcac 720
   gtgaacccaa aggagagaaa ggccagcccc aggagctggg ccgcaggttc gccctgacag 780
   caaacatctt taagaagttc ttgcgtagtg tgcggcctga ccgtgaccgg ctgctgaagg 840
   agaagccagg ctgggtgaca cccatggtcc ctgagtcccg aaccggccgc tcacagaagg 900
   tcaagaagcg gagcctttcc aagggctctg gacatttccc cttcccaggc accggggagc 960
   acaggcgagg ggagaatccc cccacaagct gccccaaggc cctggagcac tcaccctcag 1020
   gatttgatat taacacagct gtttgggtct gaatcctaga gacagaaagt tgactgagcc 1080
   tgaaagggcc aggtcccagt gctgggcccc tggggaggag ggagggtggg cggtatggct 1140
   ctcgaaagcc caactccaag ttcctttccc ccagaaagcg gggagaagcc agagttcttg 1200
   gctcaggact gaagggaagg tggttgggag aggctgtctt gggggctagc tggtggagga 1260
   ggtaagagta gctggagagt gagctgtgcg tgtgtgtgtg tgtgtgtgca tgtgtgtgtc 1320
   tgtctggcat gcatgcactc actttggggc tggaggtgac agtaggtgag ggcagaggag 1380
   gagatcagaa aatccctctg acatctccac tgcccccaaa gacctccgtt gaacattctg 1440
   tatggaaaag agccctggag catcaggttc cccagatagg cccccaaata aagacctgtc 1500
   tatggctctc ccaaccttct gtcagcttct ttggcaagac attgctccag gcacagggac 1560
   tgaaccccag gcctcctggg actggagcag cagtgaggca aaacccgacc tgctagccct 1620
   ttctgccttg gaggtttcag tccatacctg gactctgaga aaatgagctg aataaggagt   1680
   acagtgtgta aggagcagcc agggaagccc tagacactcc ccgcgtctcc cccatgcaca   1740
   ggggaaggat gttgacatag cactgggctg tttgaatgcc ttttcatctc catggtctca   1800
   tttgaaagtg agcgaggcag gcaggcatga tcccattttc cagataagga aacaagccta   1860
   gatatgctac atgtccagga acaactgcag ccaggaggca gaacagccta ggtctaactg   1920
   cagagtagaa gctggaccct ggagttacca acactcctcc ccaacagttc ttagcgcccc   1980
   gcaggctggg cgctgtggct cacgcctgta atcccagcac tttgggaggg caaggcaggc   2040
   ggattacctg gggtcaggag ttcatgacca gcctggccaa catggtgaaa ccccgtctct   2100
   actaaaaaaa tacgtaaaaa ttagccaggc gtggtggcac acgcctgtaa acccagctac   2160
   tcgggaggct gaggcaggag aattgcttga gcccgggaga gggaggttgc agtgagccga   2220
   gatcatgcca ctgcactcca gcctggctga cagagcaaga ctcccctgtc tc           2272
<210> 23
   <211> 2358
   <212> DNA
   <213> Homo sapiens
<400> 23
   ggagaggatc ccggagccgg tgagaattct ctgttttttc tctaccatcc tttccaggcc      60
   ttttcctcac ctaatgagtc gtagagacga gggcccagag agtctgtaaa gtggctggtg    120
   aaagattagt gtcccagggc cctacatccg ggaggtggtt cgggataaag agaactagtc    180
   ttgggaacaa tgtaggtggg aacttaaggg aatgggagag cggcccatag aggtggacgg    240
   agggcgcgat tggagtaaag cggaccctgt gtaggtatag agttgagtca agtggagtca    300
   ctgcctctgt ccctctggtc agcgtgatgg ccagaggcct ggggggcccc cactgggtgg     360
   ccgtgggact gctgacctgg gcgaccttgg ggcttctggt ggctggactc gggggtcatg    420
   acgacctgca cgacgatctg caagaggact tccatggcca cagccacagg cactcacatg    480
   aagatttcca ccatggtcac agccatgccc atggtcatgg ccacactcac gagagcatct    540
   ggcatggaca tacccacgat cacgaccatg gacattcaca tgaggattta caccatggcc    600
   atagccatgg ctactcccat gagagcctct accacagagg acatggacat gaccatgagc    660
   atagccatgg aggctatggg gagtctgggg ctccaggcat caagcaggac ctggatgctg    720
   tcactctctg ggcttatgca ctgggggcca cagtgctgat ctcagcagct ccattttttg     780
   tcctcttcct tatccccgtg gagtcgaact ctccccggca tcgctctcta cttcagatct    840
   tgctcagttt tgcttccggt gggctcctgg gagatgcttt cctgcacctc attcctcatg    900
   ctcttgaacc tcattctcac cacactctgg agcaacccgg acatggacac tcccacagtg    960
   gccagggccc cattctgtct gtgggcctgt gggttctcag tggaattgtt gcctttcttg   1020
   tcgtggagaa atttgtgaga catgtgaaag gaggacatgg tcacagtcat ggacatggac   1080
   acgctcacag tcatacacgt ggaagtcatg gacatggaag acaagagcgt tctaccaagg   1140
   agaagcagag ctcagaggaa gaaggaaagg aaacaagagg ggttcagaag aggcgaggag   1200
   ggagcacagt acccaaagat gggccagtga gacctcagaa cgctgaagaa gaaaaaagag 1260
   gcttagacct gcgtgtgtcg gggtacctga atctggctgc tgacttggca cacaacttca 1320
   ctgatggtct ggccattggg gcttcctttc gagggggccg gggactaggg atcctgacca 1380
   caatgactgt cctgctacat gaagtgcccc acgaggtcgg ggactttgcc atcttggtcc 1440
   agtctggctg caccaaaaag caggcgatgc gtctgcaact actgacagca gtaggggcac 1500
   tggcaggcac agctgtgccc ttctcactga aggaggagca gtggacagtg aaattgcagg 1560
   tggtgcaggt cctggctggg tcctgccatt tactgcaggt ggctttatct acgtagcaac 1620
   agtgtctgtg ttgcccgagc tgctgaggga ggcatcacca ttgcaatcac ttctggaggt 1680
   gctggggctg ctgggggGag ttatcatgat ggtgctgatt gcccaccttg agtgaggggt 1740
   ggataaacta ccctgcccca aacctctacc cctaactcca ggtcaggggt gcgtagaggt 1800
   tgggggccct ggccagggac atctgccaaa ggaaggaact gtagcctggg agcaatggtt 1860
   actttggcat tagggccttc aagggctggc agtcttacag aggctggagc ggtgagaatg 1920
   agaggccaga gggaccatag tgttgggcac tgtctgacca tgttgcattt ggaaggctaa 1980
   atggggccat gaagaaggct ggaagggaca gggggtgatg gcagcctacc tggtgtcccc 2040
   taccccacct gttctcggag aaccaagttg ctacacagga agttctccaa ggtccagttt 2100
   cctttctccc accagttggt ggaggcttca gggaagacca gagtcctgga cagagagggt 2160
   aacaggagga gtcggggata aacatcaaac atcaatcgtg tgtcctgatt tgggagtgat 2220
   tggggggatg ggtgggagaa gggttaattg gtattctcat ggcctgattt tttttgtttc 2280
   tattcctttt atatcactgt gtttgaatcg agggggaggg gtggtaaccg gaaataaaga 2340
   cctccgatct tccgcccc 2358
<210> 24
   <211> 1856
   <212> DNA
   <213> Homo sapiens
<400> 24
   agccaaaaga ggaagggacc ggcctcccac gtccacaggg acctgacttc cacctctctg      60
   cccagatttg cttatgtcac tgtcgccccg ggacggggag gtggggagct gagggcaagt     120
   cgcgcccgcc cctgaaatcc cagccgccta gcgattggct gcaagggtct cggcttggcc     180
   gcggattaat cacacccgag ggcttgaaag gtggctggga gcgccggaca cctcagacgg     240
   acggtggcca gggatcaggc agcggctcag gcgaccctga gtgtgccccc accccgccat     300
   ggcccggctg ctgcaggcgt cctgcctgct ttccctgctc ctggccggct tcgtctcgca     360
   gagccgggga caagagaagt cgaagatgga ctgccatggt ggcataagtg gcaccattta     420
   cgagtacgga gccctcacca ttgatgggga ggagtacatc cccttcaagc agtatgctgg     480
   caaatacgtc ctctttgtca acgtggccag ctactgaggc ctgacgggcc agtacattga     540
   actgaatgca ctacaggaag agcttgcacc attcggtctg gtcattctgg gctttccctg     600
   caaccaattt ggaaaacagg aaccaggaga gaactcagag atccttccta ccctcaagta     660
   tgtccgacca ggtggaggct ttgtccctaa tttccagctc tttgagaaag gggatgtcaa     720
   tggagagaaa gagcagaaat tctacacttt cctaaagaac tcctgtcctc ccacctcgga     780
   gctcctgggt acatctgacc gcctcttctg ggaacccatg aaggttcacg acatccgctg     840
   gaactttgag aagttcctgg tggggccaga tggtataccc atcatgcgct ggcaccaccg     900
   gaccacggtc agcaacgtca agatggacat cctgtcctac atgaggcggc aggcagccct     960
   gggggtcaag aggaagtaac tgaaggccgt ctcatcccat gtccaccatg taggggaggg    1020
   actttgttca ggaagaaatc cgtgtctcca accacactat ctacccatca cagacccctt    1080
   tcctatcact caaggcccca gcctggcaca aatggatgca tacagttctg tgtactgcca    1140
   ggcatgtggg tgtgggtgca atgtgggtgt ttacacacat gcctacaggt atgcgtgatt    1200
   gtgtgtgtgt gcatgggtgt acagccacgt gtctacctat gtgtctttct gggaatgtgt    1260
   accatctgtg tgcctgcagc tgtgtagtgc tggacagtga caaccctttc tctccagttc    1320
   tccactccaa tgataatagt tcacttatac ctaaacccaa aggaaaaacc agctctaggt    1380
   ccaattgttc tgctctaact gatacctcaa ccttggggcc agcatctccc actgcctcca    1440
   aatattagta actatgactg acgtccccag aagtttctgg gtctaccaca ctccccaacc    1500
   ccccactcct acttcctgaa gggccctccc aaggctacat ccccacccca cagttctccc    1560
   tgagagagat caacctccct gagatcaacc aaggcagatg tgacagcaag ggccacggac    1620
   cccatggcag gggtggcgtc ttcatgaggg aggggcccaa agcccttgtg ggcggacctc    1680
   ccctgagcct gtctgagggg ccagccctta gtgcattcag gctaaggccc ctgggcaggg    1740
   atgccacccc tgctccttcg gaggacgtgc cctcacccct cactggtcca ctggcttgag    1800
   actcaccccg tctgcccagt aaaagccttt ctgcagcaaa aaaaaaaaaa aaaaaa        1856
<210> 25
   <211> 2727
   <212> DNA
   <213> Homo sapiens
<400> 25
   ggggacactg gatcacctag tgtttcacaa gcaggtacct tctgctgtag gagagagaga    60
   actaaagttc tgaaagacct gttgcttttc accaggaagt tttactgggc atctcctgag   120
   cctaggcaat agctgtaggg tgacttctgg agccatcccc gtttccccgc cccccaaaag   180
   aagcggagat ttaacgggga cgtgcggcca gagctgggga aatgggcccg cgagccaggc   240
   cggcgcttct cctcctgatg cttttgcaga ccgcggtcct gcaggggcgc ttgctgcgtt   300
   cacactctct gcactacctc ttcatgggtg cctcagagca ggaccttggt ctttccttgt   360
   ttgaagcttt gggctacgtg gatgaccagc tgttcgtgtt ctatgatcat gagagtcgcc   420
   gtgtggagcc ccgaactcca tgggtttcca gtagaatttc aagccagatg tggctgcagc   480
   tgagtcagag tctgaaaggg tgggatcaca tgttcactgt tgacttctgg actattatgg   540
   aaaatcacaa ccacagcaag gagtcccaca ccctgcaggt catcctgggc tgtgaaatgc   600
   aagaagacaa cagtaccgag ggctactgga agtacgggta tgatgggcag gaccaccttg   660
   aattctgccc tgacacactg gattggagag cagcagaacc cagggcctgg cccaccaagc   720
   tggagtggga aaggcacaag attcgggcca ggcagaacag ggcctacctg gagagggact 780
   gccctgcaca gctgcagcag ttgctggagc tggggagagg tgttttggac caacaagtgc 840
   ctcctttggt gaaggtgaca catcatgtga cctcttcagt gaccactcta cggtgtcggg 900
   ccttgaacta ctacccccag aacatcacca tgaagtggct gaaggataag cagccaatgg 960
   atgccaagga gttcgaacct aaagacgtat tgcccaatgg ggatgggacc taccagggct 1020
   ggataacctt ggctgtaccc cctggggaag agcagagata tacgtgccag gtggagcacc 1080
   caggcctgga tcagcccctc attgtgatct gggagccctc accgtctggc accctagtca 1140
   ttggagtcat cagtggaatt gctgtttttg tcgtcatctt gttcattgga attttgttca 1200
   taatattaag gaagaggcag ggttcaagag gagccatggg gcactacgtc ttagctgaac 1260
   gtgagtgaca cgcagcctgc agactcactg tgggaaggag acaaaactag agactcaaag 1320
   agggagtgca tttatgagct cttcatgttt caggagagag ttgaacctaa acatagaaat 1380
   tgcctgacga actccttgat tttagccttc tctgttcatt tcctcaaaaa gatttcccca 1440
   tttaggtttc tgagttcctg catgccggtg atccctagct gtgacctctc ccctggaact 1500
   gtctctcatg aacctcaagc tgcatctaga ggcttccttc atttcctccg tcacctcaga 1560
   gacatacacc tatgtcattt catttcctat ttttggaaga ggactcctta aatttggggg 1620
   acttacatga ttcattttaa catctgagaa aagctttgaa ccctgggacg tggctagtca 1680
   taaccttacc agatttttac acatgtatct atgcattttc tggacccgtt caacttttcc 1740
   tttgaatcct ctctctgtgt tacccagtaa ctcatctgtc accaagcctt ggggattctt 1800
   ccatctgatt gtgatgtgag ttgcacagct atgaaggctg tacactgcac gaatggaaga 1860
   ggcacctgtc ccagaaaaag catcatggct atctgtgggt agtatgatgg gtgtttttag 1920
   caggtaggag gcaaatatct tgaaaggggt tgtgaagagg tgttttttct aattggcatg 1980
   aaggtgtcat acagatttgc aaagtttaat ggtgccttca tttgggatgc tactctagta 2040
   ttccagacct gaagaatcac aataattttc tacctggtct ctccttgttc tgataatgaa 2100
   aattatgata aggatgataa aagcacttac ttcgtgtccg actcttctga gcacctactt 2160
   acatgcatta ctgcatgcac ttcttacaat aattctatga gataggtact attatcccca 2220
   tttctttttt aaatgaagaa agtgaagtag gccgggcacg gtggctcacg cctgtaatcc 2280
   cagggtgctg agattacagg tgtgagccac cctgcccagc cgtcaaaaga gtcttaatat 2340
   atatatccag atggcatgtg tttactttat gttactacat gcacttggct gcataaatgt 2400
   ggtacaagca ttctgtcttg aagggcaggt gcttcaggat accatataca gctcagaagt 2460
   ttcttcttta ggcattaaat tttagcaaag atatctcatc tcttctttta aaccattttc 2520
   tttttttgtg gttagaaaag ttatgtagaa aaaagtaaat gtgatttacg ctcattgtag 2580
   aaaagctata aaatgaatac aattaaagct gttatttaat tagccagtga aaaactatta 2640
   acaacttgtc tattacctgt tagtattatt gttgcattaa aaatgcatat actttaataa 2700
   atgtatattg tattgtaaaa aaaaaaa 2727
<210> 26
   <211> 879
   <212> DNA
   <213> Homo sapiens
<400> 26
   ctcctcacag aagcctggag ctgggcatcc aagaagaagc agcctcattt gttttctggt     60
   gtcatcgtag gtggccacct atggcttttg ggaatgtaaa aagggcagct ctctggcatg    120
   ttcctgactg aggatctcat aacatttaac ttgaggaact tcctcctttt ccagctttgg    180
   gagtcaagct tctcacctgg ggcgggtggg ttctgcacca ccctcccacc ctccttcctc    240
   cgtgtggacg atagagccac atccagcacc acggacagct cccgggcgcc ttcatctcct    300
   cgtcctccag gcagcacaag ccattgtgga atctccacca ggtgtacaga acggtgcctc    360
   tgcgtcctgc cactcaggac ctctcaagtc cccgatgtga tggctcctca gcatgatcag    420
   gagaaattcc atgatcttgc ttattcctgt cttgggaagt ccttctccat gtctaaccaa    480
   gatctatatg gctatagcac cagctctttg gctcttggct tggcatggct aagttgggag    540
   accaaaaaga agaatgtact tcatctggtt gggctggatt ccctctgata agccttccca    600
   gttgactgaa agatgaggct aggctctagc aagttgaagt caaaccagct ccttcaagaa    660
   gctttgagca gaatgaagtg gggaggaccc agcttccagc ccaggaagcc cactgtacct    720
   ggagccatct gggataagac tttgacccat gactcccata tccacagcct gtccatccta    780
   gcccatccca gtttatcctg tatcatttga gctgggattc ccacatcctc tgagttggaa    840
   gtcccatctc aagtcttcaa taaagactct tgaatattg                          879
<210> 27
   <211> 3287
   <212> DNA
   <213> Homo sapiens
<400> 27
   atggcgctgg ctgtcttgcg ggtcctggag ccctttccga ccgagacacc cccgttggca     60
   gtgctgctgc cacccggggg cccgtggccg gcggcggagc tgggcctggt gctggccctg    120
   aggcctgcag gggagagccc ggcagggccg gcgctgctgg tggcagccct ggaggggccg    180
   gacgcgggca ccgaagagca gggtcccggg ccgccgcagc tactggttag ccgcgcgctg    240
   ctgcggctcc tggcactggg ctccggggcc tgggtgcggg cgcgggcggt gcggcggccc    300
   ccggcgctag gttgggcact gcttggcacc tcgctggggc ctgggctcgg accgcgagtc    360
   gggccgctgc tggtgaggcg cggagagacc ctcccagttc ccggaccgcg ggtgctggag    420
   acgcggccgg cgttgcaagg gctgctgggc ccagggactc ggctggctgt gactgagctc    480
   cgcgggcggg ccagactgtg tccagagtct ggggacagca gtcggccccc acccccgccc    540
   gtggtgtcct cctttgcggt ttctggcaca gtgcggcgac tccagggagt tctgggaggg    600
   actggagatt cactaggggt gagccggagc tgtctccgtg gccttggcct cttccagggc    660
   gaatgggtgt gggtggccca ggccagagag tcatcgaaca cttcacagcc gcacttggct    720
   agggtgcagg tcctagaacc tcgctgggac ctctctgata gactgggacc cggctctgga    780
   ccgctgggag agcccctcgc tgacggactg gcgcttgtcc ctgccacttt ggcttttaat    840
   cttggctgtg accccctgga aatgggagag ctcagaattc agaggtactt ggaaggctcc 900
   atcgcccctg aagacaaagg aagctgctca ttgctgcctg ggcctccatt tgccagagag 960
   ttacacatcg aaattgtgtc ttctccccac tacagcacta atggaaatta tgacggtgtt 1020
   ctttaccggc actttcagat acccagggta gtccaggaag gggatgttct atgtgtgcca 1080
   acaattgggc aagtagagat cctggaagga agtccagaga aactgcccag gtggcgggaa 1140
   atgtttttta aagtgaagaa aacagttggg gaagctccag atggaccagc cagtgcctac 1200
   ttggccgaca ccacccatac ctccttgtac atggtgggtt ctaccctgag ccctgttcca 1260
   tggctccctt cagaggaatc cactctctgg agcagtttgt ctcctccagg cctggaggcc 1320
   ttggtgtctg aactctgtgc tgtcctgaag cctcgcctcc agccaggggg tgccctgctg 1380
   acaggaacta gcagtgtcct tctacggggc cccccaggct gtgggaagac cacagtagtt 1440
   gctgctgcct gtagtcacct tgggctccac ttactgaagg tgccctgctc cagcctctgt 1500
   gcagaaagta gtggggctgt ggagacaaaa ctgcaggcca tcttctcccg ggcccgccgt 1560
   tgccggcctg cagtcctgtt gctcacagct gtggaccttc tgggccggga ccgtgatggg 1620
   ctgggtgagg atgcccgtgt gatggctgtg ctgcgtcacc tcctcctcaa tgaggacccc 1680
   ctcaacagct gccctcccct catggttgtg gccaccacaa gccgggccca ggacctgcct 1740
   gctgatgtgc agacagcatt tcctcatgag ctcgaggtgc ctgctctgtc agaggggcag 1800
   cggctcagca tcctgcgggc cctcactgcc caccttcccc tgggccagga ggtgaacttg 1860
   gcacagctag cacggcggtg tgcaggcttt gtggtagggg atctctatgc ccttctgacc 1920
   cacagcagcc gggcagcctg caccaggatc aagaactcag gtttggcagg tggcttgact 1980
   gaggaggatg agggggagct gtgtgctgcc ggctttcctc tcctggctga ggactttggg 2040
   caggcactgg agcaactgca gacagctcac tcccaggccg ttggagcccc caagatcccc 2100
   tcagtgtcct ggcatgatgt gggtgggctg caggaggtga agaaggagat cctggagacc 2160
   attcagctcc ccctggagca ccctgagcta ctgagcctgg gcctgagacg ctcaggcctt 2220
   ctgctccatg ggccccctgg caccggcaag acccttctgg ccaaggcagt agccactgag 2280
   tgcagcctta ccttcctcag cgtgaagggg ccagagctca ttaacatgta tgtgggccaa 2340
   agtgaggaga atgtgcggga agtgtttgcc agggccaggg ctgcagctcc atgcattatc 2400
   ttctttgatg aactggactc tttggcccca agccgggggc gaagtggaga ttctggagga 2460
   gtgatggaca gggtggtgtc tcagctcctt gccgagctag atgggctgca cagcactcag 2520
   gatgtgtttg tgattggagc caccaacaga ccagatctcc tggaccctgc ccttctgcgg 2580
   cctggcagat ttgacaagct ggtgtttgtg ggggcaaatg aggaccgggc ctcccagcta 2640
   cgcgttctaa gtgccatcac acgcaaattc aagctagagc catctgtgag cctggtaaac 2700
   gtgctagatt gctgccctcc ccagctgacg ggcgcggacc tctactctct ctgctctgat 2760
   gctatgacag ctgccctcaa acgcagggtt catgacctgg aggaagggct ggaacaaggt 2820
   agctcagcac tgatgctcac catggaggac ttgctgcagg ctgccgcccg gctgcaaccc 2880
   tcagtcagtg agcaggagct gctccggtac aagcgcatcc agcgcaagtt tgctgcctgc 2940
   taggagcccc ccagggtctg ggaccccgct cagcatggct gcaggtacct tgatagccca   3000
   cagagagatc tgggaaggaa gggctcctcc tcaggctgct gccaacccac ctggaggcca   3060
   cctccctcca ggagatccca gggtgcaaag tggcattgag acagcagcaa cagctcaaga   3120
   gatatctcct gcctacttgc ccctccttcc aggccggctc taagagaaag gcccatctac    3180
   tcaggaagag ggccagggcc ttgggttctg gggattgggc cctgagaggg ctagttctgt   3240
   ggctgaaaat aaagcatgtc ccgcccccta aaaaaaaaaa aaaaaaa                 3287
<210> 28
   <211> 7239
   <212> DNA
   <213> Homo sapiens
<400> 28
   cggggcagcc gagggcccct gactcggctc ctcgcggcga catggatcgg atggccagct     60
   ccatgaagca ggtgcccaac ccactgccca aggtgctgag ccggcgcggg gtcggcgctg    120
   ggctggaggc ggcggagcgc gagagcttcg agcggactca gactgtcagc atcaataagg    180
   ccattaatac gcaggaagtg gctgtaaagg aaaaacacgc cagaacgtgc atactgggca    240
   cccaccatga gaaaggggca cagaccttct ggtctgttgt caaccgcctg cctctgtcta    300
   gcaacgcagt gctctgctgg aagttctgcc atgtgttcca caaactcctc cgagatggac    360
   acccgaacgt cctgaaggac tctctgagat acagaaatga attgagtgac atgagcagga    420
   tgtggggcca cctgagcgag gggtatggcc agctgtgcag catctacctg aaactgctaa    480
   gaaccaagat ggagtaccac accaaaaatc ccaggttccc aggcaacctg cagatgagtg    540
   accgccagct ggacgaggct ggagaaagtg acgtgaacaa ctttttccag ttaacagtgg    600
   agatgtttga ctacctggag tgtgaactca acctcttcca aacagtattc aactccctgg    660
   acatgtcccg ctctgtgtcc gtgacggcag cagggcagtg ccgcctcgcc ccgctgatcc    720
   aggtcatctt ggactgcagc cacctttatg actacactgt caagcttctc ttcaaactcc    780
   actcctgcct cccagctgac accctgcaag gccaccggga ccgcttcatg gagcagttta    840
   caaagttgaa agatctgttc taccgctcca gcaacctgca gtacttcaag cggctcattc    900
   agatccccca gctgcctgag aacccaccca acttcctgcg agcctcagcc ctgtcagaac    960
   atatcagccc tgtggtggtg atccctgcag aggcctcatc ccccgacagc gagccagtcc   1020
   tagagaagga tgacctcatg gacatggatg cctctcagca gaatttattt gacaacaagt   1080
   ttgatgacat ctttggcagt tcattcagca gtgatccctt caatttcaac agtcaaaatg   1140
   gtgtgaacaa ggatgagaag gaccacttaa ttgagcgact atacagagag atcagtggat   1200
   tgaaggcaca gctagaaaac atgaagactg agagccagcg ggttgtgctg cagctgaagg   1260
   gccacgtcag cgagctggaa gcagatctgg ccgagcagca gcacctgcgg cagcaggcgg   1320
   ccgacgactg tgaattcctg cgggcagaac tggacgagct caggaggcag cgggaggaca   1380
   ccgagaaggc tcagcggagc ctgtctgaga tagaaaggaa agctcaagcc aatgaacagc   1440
   gatatagcaa gctaaaggag aagtacagcg agctggttca gaaccacgct gacctgctgc   1500
   ggaagaatgc agaggtgacc aaacaggtgt ccatggccag acaagcccag gtagatttgg   1560
   aacgagagaa aaaagagctg gaggattcgt tggagcgcat cagtgaccag ggccagcgga 1620
   agactcaaga acagctggaa gttctagaga gcttgaagca ggaacttgcc acaagccaac 1680
   gggagcttca ggttctgcaa ggcagcctgg aaacttctgc ccagtcagaa gcaaactggg 1740
   cagccgagtt cgccgagcta gagaaggagc gggacagcct ggtgagtggc gcagctcata 1800
   gggaggagga attatctgct cttcggaaag aactgcagga cactcagctc aaactggcca 1860
   gcacagagga atctatgtgc cagcttgcca aagaccaacg aaaaatgctt ctggtggggt 1920
   ccaggaaggc tgcggagcag gtgatacaag acgccctgaa ccagcttgaa gaacctcctc 1980
   tcatcagctg cgctgggtct gcagatcacc tcctctccac ggtcacatcc atttccagct 2040
   gcatcgagca actggagaaa agctggagcc agtatctggc ctgcccagaa gacatcagtg 2100
   gacttctcca ttccataacc ctgctggccc acttgaccag cgacgccatt gctcatggtg 2160
   ccaccacctg cctcagagcc ccacctgagc ctgccgactc actgaccgag gcctgtaagc 2220
   agtatggcag ggaaaccctc gcctacctgg cctccctgga ggaagaggga agccttgaga 2280
   atgccgacag cacagccatg aggaactgcc tgagcaagat caaggccatc ggcgaggagc 2340
   tcctgcccag gggactggac atcaagcagg aggagctggg ggacctggtg gacaaggaga 2400
   tggcggccac ttcagctgct attgaaactg ccacggccag aatagaggag atgctcagca 2460
   aatcccgagc aggagacaca ggagtcaaat tggaggtgaa tgaaaggatc cttggttgct 2520
   gtaccagcct catgcaagct attcaggtgc tcatcgtggc ctctaaggac ctccagagag 2580
   agattgtgga gagcggcagg ggtacagcat cccctaaaga gttttatgcc aagaactctc 2640
   gatggacaga aggacttatc tcagcctcca aggctgtggg ctggggagcc actgtcatgg 2700
   tggatgcagc tgatctggtg gtacaaggca gagggaaatt tgaggagcta atggtgtgtt 2760
   ctcatgaaat tgctgctagc acagcccagc ttgtggctgc atccaaggtg aaagctgata 2820
   aggacagccc caacctagcc cagctgcagc aggcctctcg gggagtgaac caggccactg 2880
   ccggcgttgt ggcctcaacc atttccggca aatcacagat cgaagagaca gacaacatgg 2940
   acttctcaag catgacgctg acacagatca aacgccaaga gatggattct caggttaggg 3000
   tgctagagct agaaaatgaa ttgcagaagg agcgtcaaaa actgggagag cttcggaaaa 3060
   agcactacga gcttgctggt gttgctgagg gctgggaaga aggaacagag gcatctccac 3120
   ctacactgca agaagtggta accgaaaaag aatagagcca aaccaacacc ccatatgtca 3180
   gtgtaaatcc ttgttaccta tctcgtgtgt gttatttccc cagccacagg ccaaatcctt 3240
   ggagtcccag gggcagccac accactgcca ttacccagtg ccgaggacat gcatgacact 3300
   tccaaagact ccctccatag cgacaccctt tctgtttgga cccatggtca tctctgttct 3360
   tttcccgcct ccctagttag catccaggct ggccagtgct gcccatgagc aagcctaggt 3420
   acgaagaggg gtggtggggg gcagggccac tcaacagaga ggaccaacat ccagtcctgc 3480
   tgactatttg acccccacaa caatgggtat ccttaataga ggagctgctt gttgtttgtt 3540
   gacagcttgg aaagggaaga tcttatgcct tttcttttct gttttcttct cagtcttttc 3600
   agtttcatca tttgcacaaa cttgtgagca tcagagggct gatggattcc aaaccaggac 3660
   actaccctga gatctgcaca gtcagaagga cggcaggagt gtcctggctg tgaatgccaa 3720
   agccattctc cccctctttg ggcagtgcca tggatttcca ctgcttctta tggtggttgg 3780
   ttgggttttt tggttttgtt ttttttttta agtttcactc acatagccaa ctctcccaaa 3840
   gggcacaccc ctggggctga gtctccaggg ccccccaact gtggtagctc cagcgatggt 3900
   gctgcccagg cctctcggtg ctccatctcc gcctccacac tgaccaagtg ctggcccacc 3960
   cagtccatgc tccagggtca ggcggagctg ctgagtgaca gctttcctca aaaagcagaa 4020
   ggagagtgag tgcctttccc tcctaaagct gaatcccggc ggaaagcctc tgtccgcctt 4080
   tacaagggag aagacaacag aaagagggac aagagggttc acacagccca gttcccgtga 4140
   cgaggctcaa aaacttgatc acatgcttga atggagctgg tgagatcaac aacactactt 4200
   ccctgccgga atgaactgtc cgtgaatggt ctctgtcaag cgggccgtct cccttggccc 4260
   agagacggag tgtgggagtg attcccaact cctttctgca gacgtctgcc ttggcatcct 4320
   cttgaatagg aagatcgttc caccttctac gcaattgaca aacccggaag atcagatgca 4380
   attgctccca tcagggaaga accctatact tggtttgcta cccttagtat ttattactaa 4440
   cctcccttaa gcagcaacag cctacaaaga gatgcttgga gcaatcagaa cttcaggtgt 4500
   gactctagca aggctcatct ttctgcccgg ctacatcagc cttcaagaat cagaagaaag 4560
   gccaaggtgc tggactgtta ctgacttgga tcccaaagca aggagatcat ttggagctct 4620
   tgggtcagag aaaatgagaa aggacagagc cagcggctcc aactcctttc agccacatgc 4680
   cccaggctct cgctgccctg tggacaggat gaggacagag ggcacatgaa cagcttgcca 4740
   gggatgggca gcccaacagc acttttcctc ttctagatgg accccagcat ttaagtgacc 4800
   ttctgatctt ggaaaaacag cgtcttcctt ctttatctat agcaactcat tggtggtagc 4860
   catcaagcac ttcccaggat ctgctccaac agaatattgc taggttttgc tacatgacgg 4920
   gttgtgagac ttctgtttga tcactgtgaa ccaaccccca tctccctagc ccacccccct 4980
   ccccaactcc ctctctgtgc attttctaag tgggacattc aaaaaactct ctcccaggac 5040
   ctcggatgac catactcaga cgtgtgacct ccatactggg ctaaggaagt atcagcacta 5100
   gaaattgggc agtcttaatg ttgaatgctg ctttctgctt agtatttttt tgattcaagg 5160
   ctcagaagga atggtgcgtg gcttccctgt cccagttgtg gcaactaaac caatcggtgt 5220
   gttcttgatg cgggtcaaca tttccaaaag tggctagtcc tcacttctag atctcagcca 5280
   ttctaactca tatgttccca attaccaagg ggtggccggg cacagtggct cacgcctgta 5340
   atcccagcac tttgagaggc tgaggtggta ggatcacctg aggtcaggag ttcaagacca 5400
   gcctgtccaa catggtgaaa cccccatctc tactaaaaat accaaaaatt agccgagcgt 5460
   agtgacgggt gcccgtaatc ccagctactc aggaggctga gacaggagaa tcacctgaac 5520
   cccagaggca gaggttgcag tgagctgaga tcacgccatt gtactccagc ctgggcaaca 5580
   agagcaaaac tccgtctcaa aaaaaaaaaa aaattacaaa tggggcaaac agtctagtgt 5640
   aatggatcaa attaagattc tctgcccagc cgggcacagt ggcgcatgcc tgtaatccca 5700
   gaactttggg aggccaagac gggatgattg cttgagctca ggagtttgag accaggctgg 5760
   gcatcatagc aagacctcat ctctactaaa attcaaaaac aaaattagcc gggcatgatg 5820
   gtgcatgcct gtagtctcag ctagttgggg agctaaggtg ggagaattgc ttgagcttgg 5880
   gaagtcgagg ctgcagtcag ccctgattgt gccagtgcac tccggcctgg gtgacagagt 5940
   gagaccctgt ctcaaaaaaa aaaagattct gtgtcagagc ccagcccagg agtttgaggc 6000
   tgcaatgagc catgatttcc cactgcactc cagcctgagt gacagagcga gactccatct 6060
   ctttaaaaac aaacaaaaaa ttatctgaat gatcctgtct ctaaaaagaa gccacagaaa 6120
   tgtttaaaaa cttcatcgac ttagcctgag tcataacggt taagaaagca cttaaacaga 6180
   agcagaggct aattcagtgt cacatgagga agtagctgtc agatgtcaca taattacttt 6240
   cgtaatagct cagattagaa tggctacccc attctctaga caaaatcaaa ttgtcctatt 6300
   gtgactcttc taaaaatgaa gatgaagagc tatttaatga cacaccttgg attaaaacgg 6360
   gaatcacatc ttaaagctaa aaatgaacct gcaagccttc taaatgagtc actgagcatc 6420
   actagtgaca agtctcgggt gagcgtaaat gggtcatgac aagatgggac agcaacaaaa 6480
   tcatggctta ggatcgacaa gaagttaaaa aacagctgca tctgttactt aagtttgtaa 6540
   gacagtgccc tgagacctct agagaaaaga tgtttgttta cataagagaa agaggccaga 6600
   catggtgtct cacacgttta atcccagcac tttgggaggc aggggcgggt ggatcacctg 6660
   aggtcaggag ttcaagacta gcctggccaa catggtgaaa ccccgtctct actaaaaata 6720
   caaaaattag ccgggcatgg tggcaggcgc ctataatccc agctactggg gaggctgagg 6780
   caggagaatc acttgaaccc gggggacaga ggttgtagtg agccaagatc gcaccactgc 6840
   actccagcct gggtcacaga gtgagactcc atctcaaaaa aaaaaaagag agagagagag 6900
   aaagaaatag aagagaagag ccatcttggc agggttattt tatatctgag caaggagttt 6960
   aaatgagact agtttagatt gtctgctgat gcagccgtcc atagcagtac ccctaaaatc 7020
   ccaccagaat acgggtccct ctaacccagt ggctggaaga accactgtct agagcaactt 7080
   ttcttggaac tgtcccagct accaagtcag acaccaaggt ttatgccacc aggtaacacg 7140
   ggaatcacag gtacatgtcg ctccggtcag attagttggc tttggcccct cgaccctgtg 7200
   caggagctag ttctcagctt gcagctggaa gttccctct 7239
<210> 29
   <211> 21
   <212> DNA
   <213> Homo sapiens
<400> 29
   tgactggcag atccagaggt t 21
<210> 30
   <211> 21
   <212> DNA
   <213> Homo sapiens
<400> 30
   gtagaatatg gacaggaaca c 21
<210> 31
   <211> 21
   <212> DNA
   <213> Homo sapiens
<400> 31 21
   cctgaagtgt aacaccccag a 21
<210> 32
   <211> 21
   <212> DNA
   <213> Homo sapiens
<400> 32 21
   tccctctcca gcacttctag t 21
<210> 33
   <211> 31
   <212> DNA
   <213> Homo sapiens
<400> 33
   tgctggagct gggacccagc attgaggagg a 31
<210> 34
   <211> 27
   <212> DNA
   <213> Homo sapiens
<400> 34
   tcagacacac acaacttcag tcgatag 27

## Description

La présente invention concerne un procédé de pronostic d'un syndrome septique.

Le syndrome septique, réponse systémique à l'infection, représente l'une des premières causes de mortalité dans les services de réanimation. Il peut résulter d'une infection bactérienne, virale, mycosique ou parasitaire. Parmi ce syndrome septique, on distingue par ordre croissant de gravité le sepsis, le sepsis sévère et le choc septique. Un groupe d'experts a ainsi proposé en 1992 des critères de définitions de ces trois syndromes cliniques (R. C. Bone et al, The ACCP/SCCM Consensus Conférence Committee. American College of Chest Physicians/Society of Critical Care Medicine. Chest 101 (6):1644-1655, 1992) :
- le sepsis est ainsi une réponse systémique inflammatoire liée à une infection,
- un sepsis sévère est un sepsis accompagné du disfonctionnement d'au moins un organe,
- le choc septique est un sepsis sévère associé à une hypotension persistante et peut être qualifié par :
   o la présence d'un site infectieux identifié,
   o une réponse inflammatoire généralisée se manifestant par au moins trois des signes suivants : a) température supérieure à 38°C ou inférieure à 36°C ; b) rythme cardiaque supérieur à 90 battements par minute ; c) rythme respiratoire supérieur à 20 respirations par minute ; d) nombre de leucocytes supérieur à 12000/mm³ ou inférieur à 4000/mm³,
   o une hypotension persistante malgré des remplissages adéquats et des traitements vasopresseurs.

D'une manière générale, les signes d'un sepsis, d'un sepsis sévère et d'un choc septique sont proches, et la différence entre ces 3 situations réside principalement dans l'importance de la perturbation de toutes les fonctions vitales. Lors d'un choc septique, on observe principalement une chute de pression artérielle, tachycardie, polypnée, marbrures cutanées, hypo- ou hyperthermie, frissons. Ces signes s'accompagnent également d'un dysfonctionnement d'organes "cibles" avec une altération de fonction d'organes à distance du foyer infectieux (reins, poumons, système nerveux central, appareil digestif et système hématologique le plus souvent atteints) se traduisant par une oligurie (<0,5 ml/kg/h), insuffisance rénale, hypoxémie, thrombopénie, agitation , confusion.

L'évolution d'un syndrome septique depuis le stade de sepsis vers un stade de sepsis sévère, puis de choc septique n'est pas systématique puisque environ 64% des patients septiques développent un sepsis sévère, et 23% des patients en sepsis sévère évoluent en choc septique. Avant cette étape ultime de choc septique, des traitements doivent être prescrits au patient afin d'interrompre et d'inverser le processus physiopathologique. Il faut ainsi restaurer un état hémodynamique satisfaisant et assurer une ventilation efficace. Il faut également mener de front le traitement symptomatique du choc et un traitement antibiotique adapté dès que possible aux données bactériologiques.

Il apparaît ainsi que si certains patients développant un syndrome septique, et notamment un choc septique, peuvent être réanimés par un traitement relativement simple, tel qu'un traitement antibiotique de large spectre mis en place avant les résultats des analyses bactériologiques indiquant la source infectieuse, d'autres patients, développant un syndrome septique beaucoup plus grave, nécessitent un traitement drastique et coûteux, tel qu'une injection de protéine C activée, dont le coût de l'injection est très élevé. De tels traitements sont non seulement coûteux, mais exposent également les patients à des risques d'effets indésirables très importants (troubles de la coagulation ...). Ce traitement ne doit donc être proposé qu'aux patients de mauvais pronostic nécessitant absolument ce traitement.

De ce fait, le diagnostic précoce d'un syndrome septique est essentiel et permet de proposer un traitement adapté au patient. De plus, le pronostic du syndrome septique, et notamment d'un choc septique est primordial pour proposer à chaque patient un traitement adapté, et discriminer au plus tôt les patients présentant un syndrome septique de mauvais pronostic, et qui nécessitent une thérapie lourde, des patients de bon pronostic. Enfin, il est également très intéressant de surveiller des patients à risque de développer un sepsis tels que des patients ayant subis une chirurgie, une greffe, ou des patients immunodéprimés, afin de pouvoir intervenir le plus tôt possible avant tous signes cliniques majeurs.

Actuellement, le diagnostic et le pronostic d'un syndrome septique, et notamment d'un choc septique sont essentiellement basés sur le nombre des défaillances viscérales, la réponse au traitement symptomatique, l'accessibilité à la thérapeutique médicale et/ou chirurgicale du foyer infectieux initial et des éventuels foyers secondaires.

Ceci présente toutefois l'inconvénient de n'être applicable qu'à un stade avancé du syndrome septique, et notamment du choc septique, réduisant les chances de survie du patient.

Le diagnostic et le pronostic d'un syndrome septique peuvent également être basés sur la détection de certaines protéines ou facteurs solubles impliqués dans ce syndrome. Ainsi, le dosage de certaines cytokines, impliquées lors du développement d'un syndrome septique peut être un moyen de diagnostic et de pronostic d'un syndrome septique.

Certains auteurs ont ainsi décrit une corrélation positive entre la teneur plasmatique d'IL-1 (Interleukine-1) et un syndrome septique de mauvais pronostic (Thijs & Hack, Intensive Care Med 31 : S258-263, 1995). Toutefois, d'autres auteurs n'ont trouvé aucune corrélation entre l'IL-1 et un mauvais pronostic du syndrome septique, suggérant une grande variabilité de ce facteur. De plus, des dosages élevés de TNF (Tumor Necrosis Factor) ont été associés aussi à un mauvais pronostic (Casey et al., Ann Intern Med. 1993. 119:771-778). Le TNF-α puis l'IL-1β sont les deux premières cytokines pro-intlammatoires libérées par monocytes après le déclenchement d'un état septique.

D'autres auteurs ont montré que la teneur en IL-10 (Interleukine-10) plasmatique est plus élevée chez les patients développant un sepsis de mauvais pronostic alors qu'elle décroît significativement chez les patients présentant un sepsis de bon pronostic pour ne pas être détectable chez les patients sains (Van der Poll, J. Infect. Dis. 175 :118-122, 1997). L'IL-10 est une cytokine anti inflammatoire très importante, et, qui, par sa capacité à inhiber la production du TNF-α et de l'IL-1β participe à la mise en place de l'état d'immuno-paralysie. Toutefois, cette augmentation de la teneur en IL-10 n'étant détectable que chez 80% des patients en choc septique, la détection unique de ce facteur reste insuffisante pour pronostiquer l'évolution du choc septique.

On peut citer également le brevet US-B-6,303,321 qui décrit une méthode de pronostic de la sévérité d'un syndrome septique comprenant la mesure de la concentration sérique en HMG1 (high mobility group 1 protein) par une technique immunoblot de type ELISA. L'HMG1 est, à l'inverse du TNF-α et de l'IL-1β, décrit comme un médiateur pro-inflammatoire tardif des syndromes septiques. Une forte concentration en HMG1 est corrélée à un mauvais pronostic, la concentration sérique en HMG1 n'étant pas détectée chez les patients sains. Il a par contre été décrit chez la souris une régulation post transcriptionnelle du gène HMG1, suggérant que l'expression de ce gène ne doit être analysé qu'au niveau protéique (Wang et al, Science, 1999, vol 285, p248-251).

La demande de brevet WO 04/108957 présente une méthode pour le pronostic d'un syndrome septique selon laquelle on détermine l'expression d'au moins deux gènes cibles choisis parmi : IL-10, TGFb, HMG1, T-bet, IL-1b, TNFa, GATA-3. L'utilisation d'un tel panel permet de classifier les patients de bons et de mauvais pronostic à plus de 80%. Il serait toutefois nécessaire d'augmenter encore ce pourcentage de classification, notamment en ce qui concerne la classification des patients de mauvais pronostic, afin de leur proposer un traitement drastique dès que possible.

La présente invention se propose de résoudre les inconvénients de l'état de la technique en présentant un nouvel outil fiable de pronostic d'un syndrome septique, tel que notamment un choc septique.

D'une manière surprenante, les inventeurs ont mis en évidence que l'analyse de l'expression de gènes cibles sélectionnés parmi 28 gènes tel que présenté dans le tableau 1 ci après, est très pertinente pour discriminer des bons pronostics de patients de mauvais pronostic. L'utilisation d'un tel panel permet notamment de classifier à 100% les patients de mauvais pronostic.

**Tableau 1- liste des 28 gènes cibles**

| **SEQ ID N°** | **Nom de gène** | **N° GENBANK** |
|---|---|---|
| **1** | chemokine (C-X3-C motif) receptor 1 | NM_001337 |
| **2** | T cell receptor delta diversity 3 | X72501 |
| **3** | KIAA0882 protein | NM_015130 |
| **4** | T-cell lymphoma invasion and metastasis 1 | NM_003253 |
| **5** | Interleukin 1, beta | NM_000576 |
| **6** | Carbonyl reductase 1 | NM_001757 |
| **7** | TIR domain containing molecule 1 | NM_182919 |
| **8** | FYN tyrosine kinase protooncogene | NM_002037 |
| **9** | Heparanase | NM_006665 |
| **10** | SRY (Sex determining région Y) box 4 | NM_003107 |
| **11** | Interleukin 2 receptor, beta | NM_000878 |
| **12** | Raft-linking protein | NM_015150 |
| **13** | CGI-40 protein Homo sapiens SID1 transmembrane family, member 2 | NM_015996 |
| **14** | glucose-6-phosphatase catalytic subunit 3 | NM_138387 |
| **15** | Mannosidase alpha, class 1A member 2 | NM_006699 |
| **16** | Myeloid differentiation primary response gene (88) | NM_002468 |
| **17** | Ribosomal protein L6 | NM_000970 |
| **18** | Ribosomal protein L10a | NM_007104 |
| **19** | sin3-associated polypeptide, 30kDa | NM_003864 |
| **20** | Mitogen activated protein kinase-activated protein kinase 2 | NM_004759 |
| **21** | Presenlin enhancer 2 | NM_172341 |
| **22** | Hypothetical protein LOC55924 | NM_019099 |
| **23** | Solute carrier family 39 (zinc transporter member 7) | NM_006979 |
| **24** | Glutathione peroxidase 3 (plasma) | NM_002084 |
| **25** | Hemochromatosis | NM_000410 |
| **26** | Transcriptional activator of the cfos promoter | NM_006365 |
| **27** | peroxisomal biogenesis factor 6 | NM_000287 |
| **28** | Huntingtin intercating protein | NM_005338 |

Il existe parfois plusieurs variants pour un même gène cible. Tous les variants sont pertinents. Il est bien entendu que si différentes isoformes de ces gènes existent, toutes les isoformes sont relevantes, et pas uniquement celles présentées dans le précèdent tableau. A ce titre, il convient notamment de noter qu'il existe trois variants pour le gène cible de SEQ ID N°8, seul le premier variant est présenté dans le tableau ci dessus, mais le deuxième variant, ayant pour numéro d'accession Genbank NM_153047, et le troisième variant ayant pour numéro Genbank NM_153048 sont tout aussi pertinents.

De même, il existe deux variants pour le gène cible de SEQ ID N°20, seul le premier variant est présenté dans le tableau ci-dessus, mais le deuxième variant, ayant pour numéro d'accession Genbank NM_032960, est tout aussi pertinent.

De même, il existe deux variants pour le gène cible de SEQ ID N°22, seul le premier variant est présenté dans le tableau ci-dessus, mais le deuxième variant, ayant pour numéro d'accession Genbank NM_198926, est tout aussi pertinent. Enfin, il existe onze variants pour le gène cible de SEQ ID N°25, seul le premier variant est présenté dans le tableau ci-dessus, mais les autres variants, ayant pour numéro d'accession Genbank NM_139002; NM_139003; NM_139004; NM_139005; NM_139006; NM_139007; NM_139008; NM_139009; NM_139010; NM_139011 sont tout aussi pertinents.

A cet effet, la présente invention concerne un procédé pour le pronostic d'un syndrome septique à partir d'un échantillon biologique d'un patient caractérisé en ce qu'il comprend les étapes suivantes :
a. on extrait du matériel biologique de l'échantillon biologique,
b. on met en contact le matériel biologique avec au moins une sonde d'hybridation spécifique d'au moins un gène cible, ledit au moins gène cible présentant la séquence nucléique SEQ ID N° 1; et
c. on détermine l'expression dudit au moins gène cible.

Au sens de la présente invention, on entend par échantillon biologique, tout échantillon prélevé chez un patient, et susceptible de contenir un matériel biologique tel que défini ci-après. Cet échantillon biologique peut être notamment un échantillon de sang, de sérum, de salive, tissu, de cellules circulantes du patient. On dispose de cet échantillon biologique par tout type de prélèvement connu de l'homme du métier. Selon un mode préféré de réalisation de l'invention, l'échantillon biologique prélevé chez le patient est un échantillon sanguin.

Lors de l'étape a) du procédé selon l'invention, on extrait le matériel biologique de l'échantillon biologique par tous les protocoles d'extraction et de purification d'acides nucléiques bien connus de l'homme du métier. Au sens de la présente invention, on entend par matériel biologique, tout matériel permettant de détecter l'expression d'un gène cible. Le matériel biologique peut comprendre notamment des protéines, ou des acides nucléiques tels que notamment les acides désoxyribonucléiques (ADN) ou les acides ribonucléiques (ARN). L'acide nucléique peut être notamment un ARN (acide ribonucléique). Selon un mode préféré de réalisation de l'invention, le matériel biologique extrait lors de l'étape a) comprend des acides nucléiques, préférentiellement, des ARN, et encore plus préférentiellement des ARN totaux. Les ARN totaux comprennent les ARN de transfert, les ARN messagers (ARNm), tel que les ARNm transcrits du gène cible, mais également transcrit de tout autre gène et les ARN ribosomaux. Ce matériel biologique comprend du matériel spécifique d'un gène cible, tel que notamment les ARNm transcrits du gène cible ou les protéines issues de ces ARNm mais peut comprendre également du matériel non spécifique d'un gène cible, tel que notamment les ARNm transcrits d'un gène autre que le gène cible, les ARNt, les ARNr issus d'autres gènes que le gène cible.

A titre indicatif, l'extraction d'acides nucléiques peut être réalisée par :
- une étape de lyse des cellules présentes dans l'échantillon biologique, afin de libérer les acides nucléiques contenus dans les cellules du patient. A titre d'exemple, on peut utiliser les méthodes de lyse telles que décrites dans les demandes de brevets:
   o WO 00/05338 sur la lyse mixte magnétique et mécanique,
   o WO 99/53304 sur la lyse électrique,
   o WO 99/15321 sur la lyse mécanique.

L'homme du métier pourra utiliser d'autres méthodes de lyse bien connues, telles que les chocs thermiques ou osmotiques ou les lyses chimiques par des agents chaotropiques tels que les sels de guanidium (US 5,234,809).
- une étape de purification, permettant la séparation entre les acides nucléiques et les autres constituants cellulaires relargués dans l'étape de lyse. Cette étape permet généralement de concentrer les acides nucléiques, et peut être adaptée à la purification d'ADN ou d'ARN. A titre d'exemple, on peut utiliser des particules magnétiques éventuellement revêtues d'oligonucléotides, par adsorption ou covalence (voir à ce sujet les brevets US 4,672,040 et US 5,750,338), et ainsi purifier les acides nucléiques qui se sont fixés sur ces particules magnétiques, par une étape de lavage. Cette étape de purification des acides nucléiques est particulièrement intéressante si l'on souhaite amplifier ultérieurement lesdits acides nucléiques. Un mode de réalisation particulièrement intéressant de ces particules magnétiques est décrit dans les demandes de brevets : WO-A-97/45202 et WO-A-99/35500. Un autre exemple intéressant de méthode de purification des acides nucléiques est l'utilisation de silice soit sous forme de colonne, soit sous forme de particules inertes (Boom R. et al., J. Clin. Microbiol., 1990, n°28(3), p. 495-503) ou magnétiques (Merck: MagPrep^{®} Silica, Promega: MagneSil^{™} Paramagnetic particles). D'autres méthodes très répandues reposent sur des résines échangeuses d'ions en colonne ou en format particulaire paramagnétique (Whatman: DEAE-Magarose) (Levison PR et al., J. Chromatography, 1998, p. 337-344). Une autre méthode très pertinente mais non exclusive pour l'invention est celle de l'adsorption sur support d'oxyde métallique (société Xtrana: matrice Xtra-Bind^{™}).

Lorsque l'on souhaite extraire spécifiquement l'ADN d'un échantillon biologique, on peut notamment réaliser une extraction par du phénol, du chloroforme et de l'alcool pour éliminer les protéines et précipiter l'ADN avec de l'éthanol 100%. L'ADN peut alors être culoté par centrifugation, lavé et remis en solution.

Lorsque l'on souhaite extraire spécifiquement les ARN d'un échantillon biologique, on peut notamment réaliser une extraction par du phénol, du chloroforme et de l'alcool pour éliminer les protéines et précipiter les ARN avec de l'éthanol 100%. Les ARN peuvent alors être culotés par centrifugation, lavés et remis en solution.

Lors de l'étape b), on entend par réactif spécifique, un réactif qui, lorsqu'il est mis en contact avec du matériel biologique tel que défini précédemment, se lie avec le matériel spécifique dudit gène cible. A titre indicatif, lorsque le réactif spécifique et le matériel biologique sont d'origine nucléique, la mise en contact du réactif spécifique et du matériel biologique permet l'hybridation du réactif spécifique avec le matériel spécifique du gène cible. Par hybridation, on entend le processus au cours duquel, dans des conditions appropriées, deux fragments nucléotidiques se lient avec des liaisons hydrogènes stables et spécifiques pour former un complexe double brin. Ces liaisons hydrogènes se forment entre les bases complémentaires Adénine (A) et thymine (T) (ou uracile (U)) (on parle de liaison A-T) ou entre les bases complémentaires Guanine (G) et cytosine (C) (on parle de liaison G-C). L'hybridation de deux fragments nucléotidiques peut être totale (on parle alors de fragments nucléotidiques ou de séquences complémentaires), c'est à dire que le complexe double brin obtenu lors de cette hybridation comprend uniquement des liaisons A-T et des liaisons C-G. Cette hybridation peut être partielle (on parle alors de fragments nucléotidiques ou de séquences suffisamment complémentaires), c'est à dire que le complexe double brin obtenu comprend des liaisons A-T et des liaisons C-G permettant de former le complexe double brin, mais également des bases non liées à une base complémentaire. L'hybridation entre deux fragments nucléotidiques dépend des conditions opératoires qui sont utilisées, et notamment de la stringence. La stringence est définie notamment en fonction de la composition en bases des deux fragments nucléotidiques, ainsi que par le degré de mésappariement entre deux fragments nucléotidiques. La stringence peut également être fonction des paramètres de la réaction, tels que la concentration et le type d'espèces ioniques présentes dans la solution d'hybridation, la nature et la concentration d'agents dénaturants et/ou la température d'hybridation. Toutes ces données sont bien connues et les conditions appropriées peuvent être déterminées par l'homme du métier. En général, selon la longueur des fragments nucléotidiques que l'on souhaite hybrider, la température d'hybridation est comprise entre environ 20 et 70°C, en particulier entre 35 et 65°C dans une solution saline à une concentration d'environ 0,5 à 1 M. Une séquence, ou fragment nucléotidique, ou oligonucléotide, ou polynucléotide, est un enchaînement de motifs nucléotidiques assemblés entre eux par des liaisons ester phosphoriques, caractérisé par la séquence informationnelle des acides nucléiques naturels, susceptibles de s'hybrider à un fragment nucléotidique, l'enchaînement pouvant contenir des monomères de structures différentes et être obtenu à partir d'une molécule d'acide nucléique naturelle et/ou par recombinaison génétique et/ou par synthèse chimique. Un motif est dérivé d'un monomère qui peut être un nucléotide naturel d'acide nucléique dont les éléments constitutifs sont un sucre, un groupement phosphate et une base azotée ; dans l'ADN le sucre est le désoxy-2-ribose, dans l'ARN le sucre est le ribose ; selon qu'il s'agisse de l'ADN ou l'ARN, la base azotée est choisie parmi l'adénine, la guanine, l'uracile, la cytosine, la thymine ; ou bien le monomère est un nucléotide modifié dans l'un au moins des trois éléments constitutifs ; à titre d'exemple, la modification peut intervenir soit au niveau des bases, avec des bases modifiées telles que l'inosine, la méthyl-5désoxycytidine, la désoxyuridine, la diméthylamino-5 désoxyuridine, la diamino-2,6-purine, la bromo-5désoxyuridine ou toute autre base modifiée capable d'hybridation, soit au niveau du sucre, par exemple le remplacement d'au moins un désoxyribose par un polyamide (P.E. Nielsen et al, Science, 254, 1497-1500 (1991), soit encore au niveau du groupement phosphate, par exemple son remplacement par des esters notamment choisis parmi les diphosphates, alkyl- et aryl-phosphonates et phosphorothioates.

Le réactif spécifique peut comprendre au moins une amorce d'amplification. On entend par amorce d'amplification, un fragment nucléotidique comprenant de 5 à 100 motifs nucléiques, préférentiellement de 15 à 30 motifs nucléiques permettant l'initiation d'une polymérisation enzymatique, telle que notamment une réaction d'amplification enzymatique. Par réaction d'amplification enzymatique, on entend un processus générant de multiples copies d'un fragment nucléotidique par l'action d'au moins une enzyme. De telles réactions d'amplification sont bien connues de l'homme du métier et on peut citer notamment les techniques suivantes :
- PCR (Polymerase Chain Reaction), telle que décrite dans les brevets US 4,683,195, US 4,683,202 et US 4,800,159,
- LCR (Ligase Chain Reaction), exposée par exemple dans la demande de brevet EP 0 201 184,
- RCR (Repair Chain Reaction), décrite dans la demande de brevet WO 90/01069,
- 3SR (Self Sustained Sequence Replication) avec la demande de brevet WO 90/06995,
- NASBA (Nucleic Acid Sequence-Based Amplification) avec la demande de brevet WO 91/02818, et
- TMA (Transcription Mediated Amplification) avec le brevet US 5,399,491.

Lorsque l'amplification enzymatique est une PCR, le réactif spécifique comprend au moins 2 amorces d'amplification, spécifiques d'un gène cible, permettant l'amplification du matériel spécifique du gène cible. Le matériel spécifique du gène cible comprend alors préférentiellement un ADN complémentaire obtenu par transcription inverse d'ARN messager issu du gène cible (on parle alors d'ADNc spécifique du gène cible) ou un ARN complémentaire obtenu par transcription des ADNc spécifiques d'un gène cible (on parle alors d'ARNc spécifique du gène cible). Lorsque l'amplification enzymatique est une PCR réalisée après une réaction de transcription reverse, on parle de RT-PCR.

Selon l'invention, le réactif spécifique de l'étape b) comprend au moins une sonde d'hybridation.

Par sonde d'hybridation, on entend un fragment nucléotidique comprenant au moins 5 motifs nucléotidiques, tel que de 5 à 100 motifs nucléiques, notamment de 10 à 35 motifs nucléiques, possédant une spécificité d'hybridation dans des conditions déterminées pour former un complexe d'hybridation avec le matériel spécifique d'un gène cible. Dans la présente invention, le matériel spécifique du gène cible peut être une séquence nucléotidique comprise dans un ARN messager issu du gène cible (on parle alors d'ARNm spécifique du gène cible), une séquence nucléotidique comprise dans un ADN complémentaire obtenu par transcription inverse dudit ARN messager (on parle alors d'ADNc spécifique du gène cible), ou encore une séquence nucléotidique comprise dans un ARN complémentaire obtenu par transcription dudit ADNc tel que décrit précédemment (on parlera alors d'ARNc spécifique du gène cible). La sonde d'hybridation peut comprendre un marqueur permettant sa détection. Par détection on entend soit une détection directe par une méthode physique, soit une détection indirecte par une méthode de détection à l'aide d'un marqueur. De nombreuses méthodes de détection existent pour la détection des acides nucléiques. [Voir par exemple Kricka et al., Clinical Chemistry, 1999, n° 45(4), p.453-458 ou Keller G.H. et al., DNA Probes, 2nd Ed., Stockton Press, 1993, sections 5 et 6, p.173-249]. Par marqueur, on entend un traceur capable d'engendrer un signal que l'on peut détecter. Une liste non limitative de ces traceurs comprend les enzymes qui produisent un signal détectable par exemple par colorimétrie, fluorescence ou luminescence, comme la peroxydase de raifort, la phosphatase alcaline, la beta galactosidase, la glucose-6-phosphate déshydrogénase; les chromophores comme les composés fluorescents, luminescents ou colorants ; les groupements à densité électronique détectables par microscopie électronique ou par leurs propriétés électriques comme la conductivité, par les méthodes d'ampérométrie ou de voltamétrie, ou par des mesures d'impédance ; les groupements détectables par des méthodes optiques comme la diffraction, la résonance plasmon de surface, la variation d'angle de contact ou par des méthodes physiques comme la spectroscopie de force atomique, l'effet tunnel, etc. ; les molécules radioactives comme ³²P, ³⁵S ou ¹²⁵I.

La sonde d'hybridation peut être une sonde dite de détection. Dans ce cas, la sonde dite de détection est marquée au moyen d'un marqueur tel que défini précédemment. La sonde de détection peut être notamment une sonde de détection « molecular beacons » telle que décrite par Tyagi & Kramer (Nature biotech, 1996, 14:303-308). Ces "molecular beacons" deviennent fluorescentes lors de l'hybridation. Elles possèdent une structure de type tige-boucle et contiennent un fluorophore et un groupe "quencher". La fixation de la séquence de boucle spécifique avec sa séquence complémentaire d'acide nucléique cible provoque un déroulement de la tige et l'émission d'un signal fluorescent lors de l'excitation à la longueur d'onde qui convient.

Pour la détection de la réaction d'hybridation, on peut utiliser des séquences cibles marquées, directement (notamment par l'incorporation d'un marqueur au sein de la séquence cible) ou indirectement (notamment par l'utilisation d'une sonde de détection telle que définie précédemment) la séquence cible. On peut notamment réaliser avant l'étape d'hybridation une étape de marquage et/ou de clivage de la séquence cible, par exemple en utilisant un désoxyribonucléotide triphosphate marqué lors de la réaction d'amplification enzymatique. Le clivage peut être réalisé notamment par l'action de l'imidazole et de chlorure de manganèse. La séquence cible peut aussi être marqué après l'étape d'amplification, par exemple en hybridant une sonde de détection selon la technique d'hybridation sandwich décrite dans le document WO 91/19812. Un autre mode particulier préférentiel de marquage d'acides nucléiques est décrit dans la demande FR 2 780 059.

La sonde de détection peut comprendre un flurophore et un quencher. Préférentiellement, la sonde d'hybridation comprend un flurophore FAM (6-carboxy-fluorescein) ou ROX (6-carboxy-X-rhodamine) en son extrémité 5' et un quencher (Dabsyl) en son extrémité 3'.

La sonde d'hybridation peut être également une sonde dite de capture. Dans ce cas, la sonde dite de capture est immobilisée ou immobilisable sur un support solide par tout moyen approprié, c'est-à-dire directement ou indirectement, par exemple par covalence ou adsorption. Comme support solide, on peut utiliser des matériaux de synthèse ou des matériaux naturels, éventuellement modifiés chimiquement, notamment les polysaccharides tels que les matériaux à base de cellulose, par exemple du papier, des dérivés de cellulose tels que l'acétate de cellulose et la nitrocellulose ou le dextrane, des polymères, des copolymères, notamment à base de monomères du type styrène, des fibres naturelles telles que le coton, et des fibres synthétiques telles que le nylon ; des matériaux minéraux tels que la silice, le quartz, des verres, des céramiques ; des latex ; des particules magnétiques ; des dérivés métalliques, des gels etc. Le support solide peut être sous la forme d'une plaque de microtitration, d'une membrane comme décrit dans la demande WO-A-94/12670, d'une particule. On peut également immobiliser sur le support plusieurs sondes de capture différentes, chacune étant spécifique d'un gène cible. En particulier, on peut utiliser comme support une biopuce sur laquelle peuvent être immobilisées un grand nombre de sondes. Par biopuce, on entend un support solide de dimension réduite où est fixée une multitude de sondes de capture à des positions prédéterminées. Le concept de biopuce, ou puce à ADN, date du début des années 90. Il repose sur une technologie pluridisciplinaire intégrant la micro-électronique, la chimie des acides nucléiques, l'analyse d'images et l'informatique. Le principe de fonctionnement repose sur un fondement de la biologie moléculaire: le phénomène d'hybridation, c'est-à-dire l'appariement par complémentarité des bases de deux séquences d'ADN et/ou d'ARN. La méthode des biopuces repose sur l'emploi de sondes de capture fixées sur un support solide sur lesquelles on fait agir un échantillon de fragments nucléotidiques cibles marqués directement ou indirectement avec des fluorochromes. Les sondes de capture sont positionnées de manière spécifique sur le support ou puce et chaque hybridation donne une information particulière, en relation avec le fragment nucléotidique cible. Les informations obtenues sont cumulatives, et permettent par exemple de quantifier le niveau d'expression d'un gène ou de plusieurs gènes cibles. Pour analyser l'expression d'un gène cible, on peut alors réaliser un support comprenant une multitude de sondes, qui correspondent à tout ou partie du gène cible, qui est transcrit en ARNm. On entend par support de basse densité, un support comprenant moins de 50 sondes. On entend par support de moyenne densité, un support comprenant de 50 sondes à 10 000 sondes. On entend par support de haute densité, un support comprenant plus de 10 000 sondes.

On hybride alors par exemple les ADNc ou les ARNc spécifiques d'un gène cible que l'on souhaite analyser sur des sondes de capture spécifique. Après hybridation, le support ou puce est lavé(e), et les complexes ADNc ou ARNc marquées / sondes de capture sont révélés par un ligand de forte affinité lié par exemple à un marqueur de type fluorochrome. La fluorescence est lue par exemple par un scanner et l'analyse de la fluorescence est traitée par informatique. On peut citer à titre indicatif, les puces à ADN mises au point par la société Affymetrix ("Accessing Genetic Information with High-Density DNA arrays", M. Chee et al., Science, 1996, 274, 610-614. "Light-generated oligonucleotide arrays for rapide DNA sequence analysis", A. Caviani Pease et al., Proc. Natl. Acad. Sci. USA, 1994, 91, 5022-5026), pour les diagnostics moléculaires. Dans cette technologie, les sondes de capture sont généralement de tailles réduites, autour de 25 nucléotides. D'autres exemples de biopuces sont donnés dans les publications de G. Ramsay, Nature Biotechnology, 1998, n° 16, p. 40-44 ; F. Ginot, Human Mutation, 1997, n°10, p.1-10; J. Cheng et al, Molecular diagnosis, 1996, n°1(3) p.183-200 ; T. Livache et al, Nucleic Acids Research, 1994, n° 22(15), p. 2915-2921 ; J. Cheng et al, Nature Biotechnology, 1998, n° 16, p. 541-546 ou dans les brevets US-A-4,981,783, US-A-5,700,637, US-A-5,445,934, US-A-5,744,305 et US-A-5,807,522. La caractéristique principale du support solide doit être de conserver les caractéristiques d'hybridation des sondes de capture sur les fragments nucléotidiques cibles tout en générant un bruit de fond minimum pour la méthode de détection.

Pour l'immobilisation des sondes sur le support, on distingue trois grands types de fabrication.

Il y a, tout d'abord, une première technique qui consiste en un dépôt de sondes pré-synthétisées. La fixation des sondes se fait par transfert direct, au moyen de micropipettes, de micro-pointes ou par un dispositif de type jet d'encre. Cette technique permet la fixation de sondes de taille allant de quelques bases (5 à 10) jusqu'à des tailles relativement importantes de 60 bases (impression) à quelques centaines de bases (micro-déposition) :

L'impression est une adaptation du procédé utilisé par les imprimantes à jet d'encre. Elle repose sur la propulsion de très petites sphères de fluide (volume <1 nl) et à un rythme pouvant atteindre 4000 gouttes/secondes. L'impression n'implique aucun contact entre le système libérant le fluide et la surface sur laquelle il est déposé.

La micro-déposition consiste à fixer des sondes longues de quelques dizaines à plusieurs centaines de bases à la surface d'une lame de verre. Ces sondes sont généralement extraites de bases de données et se présentent sous forme de produits amplifiés et purifiés. Cette technique permet de réaliser des puces dénommées microarrays portant environ dix mille spots, dit zones de reconnaissance, d'ADN sur une surface d'un peu moins de 4 cm2. Il ne faut toutefois pas oublier l'emploi de membranes de Nylon, dites « macroarrays », qui portent des produits amplifiés, généralement par PCR, avec un diamètre de 0,5 à 1 mm et dont la densité maximale est de 25 spots/cm2. Cette technique très flexible est utilisée par de nombreux laboratoires. Dans la présente divulgation, cette dernière technique est considérée comme faisant partie des biopuces. On peut toutefois déposer en fond de plaque de microtitration un certain volume d'échantillon dans chaque puits, comme c'est le cas dans les demandes de brevet WO-A-00/71750 et FR 00/14896, ou déposer au fond d'une même boîte de Pétri un certain nombre de gouttes séparées les unes des autres, selon une autre demande de brevet FR00/14691.

La deuxième technique de fixation des sondes sur le support ou puce est appelée la synthèse in situ. Cette technique aboutit à l'élaboration de sondes courtes directement à la surface de la puce. Elle repose sur la synthèse d'oligonucléotides in situ (voir notamment les demandes de brevet WO 89/10977 et WO 90/03382), et est fondée sur le procédé des synthétiseurs d'oligonucléotides. Elle consiste à déplacer une chambre de réactions, où se déroule la réaction d'élongation d'oligonucléotides, le long de la surface de verre.

Enfin, la troisième technique est appelée la photolithographie, qui est un procédé à l'origine des biopuces développées par Affymetrix. Il s'agit également d'une synthèse in situ. La photolithographie est dérivée des techniques des microprocesseurs. La surface de la puce est modifiée par la fixation de groupements chimiques photolabiles pouvant être activés par la lumière. Une fois illuminés, ces groupes sont susceptibles de réagir avec l'extrémité 3' d'un oligonucléotide. En protégeant cette surface par des masques de formes définies, on peut illuminer et donc activer sélectivement des zones de la puce où l'on souhaite fixer l'un ou l'autre des quatre nucléotides. L'utilisation successive de masques différents permet d'alterner des cycles de protection/réaction et donc de réaliser les sondes d'oligonucléotides sur des spots d'environ quelques dizaines de micromètre carré (µm2). Cette résolution permet de créer jusqu'à plusieurs centaines de milliers de spots sur une surface de quelques centimètres carré (cm2). La photolithographie présente des avantages : massivement parallèle, elle permet de créer une puce de N-mères en seulement 4 x N cycles. Toutes ces techniques sont utilisables avec la présente divulgation. Selon un mode préféré, le au moins un réactif spécifique de l'étape b) définie précédemment comprend au moins une sonde d'hybridation, qui est préférentiellement immobilisée sur un support. Ce support est préférentiellement un support de basse , haute ou moyenne densité tel que définie précédemment

Ces étapes d'hybridation sur support comprenant une multitude de sondes peuvent être précédées d'une étape de réaction d'amplification enzymatique, telle que définie précédemment, pour augmenter la quantité de matériel génétique cible.

Lors de l'étape c) la détermination de l'expression d'un gène cible peut être réalisée par tous les protocoles connus de l'homme du métier.

D'une manière générale, l'expression d'un gène cible peut être analysée par la détection des ARNm (ARN messagers) qui sont transcrits du gène cible à un instant donné ou par la détection des protéines issues de ces ARNm.

L'invention concerne la détermination de l'expression d'un gène cible par la détection des ARNm issus de ce gène cible selon tous les protocoles bien connus de l'homme du métier. Selon un mode particulier de réalisation de l'invention, on détermine simultanément l'expression de plusieurs gènes cibles, par la détection de plusieurs ARNm différents, chaque ARNm étant issus d'un gène cible.

Lorsque le réactif spécifique comprend au moins une amorce d'amplification, on peut, lors de l'étape c) du procédé selon l'invention, déterminer l'expression d'un gène cible de la manière suivante:
1) après avoir extrait comme matériel biologique, les ARN totaux (comprenant les ARN de transfert (ARNt), les ARN ribosomaux (ARNr) et les ARN messagers (ARNm)) d'un échantillon biologique tel que présenté précédemment, on réalise une étape de transcription reverse afin d'obtenir les ADN complémentaires (ou ADNc) desdits ARNm. A titre indicatif, cette réaction de transcription reverse peut être réalisée à l'aide d'une enzyme reverse transcriptase qui permet d'obtenir, à partir d'un fragment d'ARN, un fragment d'ADN complémentaire. On peut utiliser notamment l'enzyme reverse transcriptase provenant de l'AMV (Avian Myoblastosis Virus) ou de MMLV (Moloney Murine Leukaemia Virus). Lorsque l'on souhaite plus particulièrement obtenir uniquement les ADNc des ARNm, on réalise cette étape de transcription reverse en présence de fragments nucléotidiques comprenant uniquement des bases thymine (polyT), qui s'hybrident par complémentarité sur la séquence polyA des ARNm afin de former un complexe polyT-polyA qui sert alors de point de départ à la réaction de transcription reverse réalisée par l'enzyme reverse transcriptase. On obtient alors des ADNc complémentaires des ARNm issus d'un gène cible (ADNc spécifique du gène cible) et des ADNc complémentaires des ARNm issus d'autres gènes que le gène cible (ADNc non spécifique du gène cible).
2) on met en contact la ou les amorces d'amplification spécifiques d'un gène cible avec les ADNc spécifique du gène cible et les ADNc non spécifique du gène cible. La ou les amorces d'amplification spécifiques d'un gène cible s'hybrident avec les ADNc spécifique du gène cible et on amplifie spécifiquement une région prédéterminée, de longueur connue, des ADNc provenant des ARNm issus du gène cible. Les ADNc non spécifiques du gène cible ne sont pas amplifiés, alors qu'on obtient alors une grande quantité d'ADNc spécifiques du gène cible. Au sens de la présente invention, on parle indifféremment d' « ADNc spécifiques du gène cible » ou d' « ADNc provenant des ARNm issus du gène cible ». Cette étape peut être réalisée notamment par une réaction d'amplification de type PCR ou par toute autre technique d'amplification telle que définie précédemment. En PCR, on peut également amplifier simultanément plusieurs ADNc différents, chacun étant spécifique de différents gènes cibles par l'utilisation de plusieurs couples d'amorces d'amplification différents, chacune étant spécifique d'un gène cible: on parle alors d'amplification en multiplex.
3) on détermine l'expression du gène cible en détectant et quantifiant les ADNc spécifiques du gène cible obtenus lors de l'étape 2) ci dessus. Cette détection peut être réalisée après migration par électrophorèse des ADNc spécifiques du gène cible en fonction de leur taille. Le gel et le milieu de migration peuvent comprendre du bromure d'éthydium afin de permettre la détection directe des ADNc spécifiques du gène cible lorsque le gel est placé, après un temps de migration donné, sur une table lumineuse à rayons UV (ultra violet) par l'émission d'un signal lumineux. Ce signal est d'autant plus lumineux que la quantité des ADNc spécifiques du gène cible est importante. Ces techniques d'électrophorèse sont bien connues de l'homme du métier. Les ADNc spécifiques du gène cible peuvent également être détectés et quantifiés par l'utilisation d'une gamme de quantification obtenue par une réaction d'amplification conduite jusqu'à saturation. Afin de tenir compte de la variabilité d'efficacité enzymatique qui peut être observée lors des différentes étapes (transcription reverse, PCR...), on peut normaliser l'expression d'un gène cible de différents groupes de patients, par la détermination simultanée de l'expression d'un gène dit de ménage, dont l'expression est similaire chez les différents groupes de patients. En réalisant un rapport entre l'expression du gène cible et l'expression du gène de ménage, c'est à dire en réalisant un rapport entre la quantité d'ADNc spécifiques du gène cible, et la quantité d'ADNc spécifiques du gène de ménage, on corrige ainsi toute variabilité entre les différentes expérimentations. L'homme du métier pourra se référer notamment aux publications suivantes : Bustin SA, J Mol Endocrinol , 2002, 29 : 23-39 ; Giulietti A Methods, 2001, 25 : 386-401.

Lorsque le réactif spécifique comprend au moins une sonde d'hybridation, on peut déterminer l'expression d'un gène cible de la manière suivante:
1) après avoir extrait, comme matériel biologique, les ARN totaux d'un échantillon biologique tel que présenté précédemment, on réalise une étape de transcription reverse, telle que décrite précédemment afin d'obtenir des ADNc complémentaires des ARNm issus d'un gène cible (ADNc spécifique du gène cible) et des ADNc complémentaires des ARNm issus d'autres gènes que le gène cible (ADNc non spécifique du gène cible).
2) on met en contact tous les ADNc avec un support, sur lequel sont immobilisées des sondes de capture spécifiques du gène cible dont on souhaite analyser l'expression, afin de réaliser une réaction d'hybridation entre les ADNc spécifiques du gène cible et les sondes de capture, les ADNc non spécifiques du gène cible ne s'hybridant pas sur les sondes de capture. La réaction d'hybridation peut être réalisée sur un support solide qui inclut tous les matériaux tels qu'indiqués précédemment. Selon un mode préféré de réalisation, la sonde d'hybridation est immobilisée sur un support. Préférentiellement, le support est un support de basse , haute ou moyenne densité tel que définie précédemment. La réaction d'hybridation peut être précédée d'une étape d'amplification enzymatique des ADNc spécifique du gène cible telle que décrite précédemment pour obtenir une grande quantité d'ADNc spécifiques du gène cible et augmenter la probabilité qu'un ADNc spécifiques d'un gène cible s'hybride sur une sonde de capture spécifique du gène cible. La réaction d'hybridation peut également être précédée d'une étape de marquage et/ou de clivage des ADNc spécifiques du gène cible telle que décrite précédemment, par exemple en utilisant un désoxyribonucléotide triphosphate marqué pour la réaction d'amplification. Le clivage peut être réalisé notamment par l'action de l'imidazole et de chlorure de manganèse. L'ADNc spécifique du gène cible peut aussi être marqué après l'étape d'amplification, par exemple en hybridant une sonde marquée selon la technique d'hybridation sandwich décrite dans le document WO-A-91/19812. D'autres modes particuliers préférentiels de marquage et/ou clivage d'acides nucléiques sont décrit dans les demandes WO 99/65926, WO 01/44507, WO 01/44506, WO 02/090584, WO 02/090319.
3) on réalise ensuite une étape de détection de la réaction d'hybridation. La détection peut être réalisée par la mise en contact du support sur lequel sont hybridés les sondes de capture spécifiques du gène cible avec les ADNc spécifiques du gène cible avec une sonde dite de détection, marquée par un marqueur, et on détecte le signal émis par le marqueur. Lorsque l'ADNc spécifique du gène cible a été préalablement marqué par un marqueur, on détecte directement le signal émis par le marqueur.

Lorsque le au moins un réactif spécifique mis en contact l'étape b) du procédé selon l'invention comprend au moins une sonde d'hybridation, on peut également déterminer l'expression d'un gène cible de la manière suivante:
1) après avoir extrait, comme matériel biologique, les ARN totaux d'un échantillon biologique tel que présenté précédemment, on réalise une étape de transcription reverse, telle que décrite précédemment afin d'obtenir les ADNc des ARNm du matériel biologique. On réalise ensuite la polymérisation de l'ARN complémentaire du ADNc par l'utilisation d'une enzyme polymerase de type T7 polymérase qui fonctionne sous la dépendance d'un promoteur et qui permet d'obtenir, à partir d'une matrice d'ADN, l'ARN complémentaire. On obtient alors les ARNc des ADNc des ARNm spécifiques du gène cible (on parle alors d'ARNc spécifique du gène cible) et les ARNc des ADNc des ARNm non spécifiques du gène cible.
2) on met en contact tous les ARNc avec un support, sur lequel sont immobilisées des sondes de capture spécifiques du gène cible dont on souhaite analyser l'expression, afin de réaliser une réaction d'hybridation entre les ARNc spécifiques du gène cible et les sondes de capture, les ARNc non spécifiques du gène cible ne s'hybridant pas sur les sondes de capture. Lorsque l'on souhaite analyser simultanément l'expression de plusieurs gènes cibles, on peut immobiliser sur le support plusieurs sondes de capture différentes, chacune étant spécifique d'un gène cible. La réaction d'hybridation peut également être précédée d'une étape de marquage et/ou de clivage des ARNc spécifiques du gène cible telles que décrites précédemment.
3) on réalise ensuite une étape de détection de la réaction d'hybridation. La détection peut être réalisée par la mise en contact du support sur lequel sont hybridées les sondes de capture spécifiques du gène cible avec l'ARNc spécifique du gène cible avec une sonde dite de détection, marquée par un marqueur, et on détecte le signal émis par le marqueur. Lorsque l'ARNc spécifique du gène cible a été préalablement marqué par un marqueur, on détecte directement le signal émis par le marqueur. L'utilisation d'ARNc est particulièrement avantageux lorsqu'on utilise un support de type biopuce sur lequel est hybridé un grand nombre de sondes.

Les étapes B et C peuvent être effectuées en même temps. Ce mode préféré peut être notamment mise en oeuvre par « NASBA en temps réel » qui regroupe en une étape unique la technique d'amplification NASBA et la détection en temps réel qui fait appel à des "molecular beacons". La réaction NASBA intervient dans le tube, produisant de l'ARN simple brin avec lequel les "molecular beacons" spécifiques peuvent s'hybrider simultanément pour donner un signal fluorescent. La formation des nouvelles molécules d'ARN est mesurée en temps réel par contrôle continu du signal dans un lecteur fluorescent. Contrairement à une amplification par RT-PCR, l'amplification en NASBA peut se faire en présence d'ADN dans l'échantillon. Il n'est donc pas nécessaire de vérifier que l'ADN a bien été complètement éliminé lors de l'extraction des ARN.

L'analyse de l'expression d'un gène cible choisi parmi l'une quelconque des SEQ ID N°1 à 28 permet alors de disposer d'un outil pour le diagnostic/pronostic d'un syndrome septique. Préférentiellement, les gènes cibles de SEQ ID N° 1, 2, 4-8, 11 et 16 permettent de discriminer les deux groupes de patients.

On peut par exemple analyser l'expression d'un gène cible chez un patient dont on ne connaît pas le pronostic, et comparer avec des valeurs d'expression moyenne connues du gène cible de patients de bons pronostic (BP) et des valeurs d'expression moyenne connues du gène cible de patients de mauvais pronostic (MP), afin de proposer au patient un traitement adapté.

Selon un autre mode préféré de réalisation, lors de l'étape b) on met en contact le matériel biologique avec au moins 2, au moins 3, au moins 4, au moins 5, au moins 6, au moins 7, au moins 8, au moins 9, au moins 10, au moins 11, au moins 12, au moins 13, au moins 14, au moins 15, au moins 16, au moins 17, au moins 18, au moins 19, au moins 20, au moins 21, au moins 22, au moins 23, au moins 24, au moins 25, au moins 26, au moins 27 réactifs spécifiques choisis parmi les réactifs spécifiques des gènes cibles présentant une séquence nucléique ayant l'une quelconque des SEQ ID N° 1 à 28 et on détermine, lors de l'étape c, l'expression d'au moins 2, au moins 3, au moins 4, au moins 5, au moins 6, au moins 7, au moins 8, au moins 9, au moins 10, au moins 11, au moins 12, au moins 13, au moins 14, au moins 15, au moins 16, au moins 17, au moins 18, au moins 19, au moins 20, au moins 21, au moins 22, au moins 23, au moins 24, au moins 25, au moins 26, au moins 27 desdits gènes cibles.

Plus particulièrement, les inventeurs ont mis en évidence que l'analyse simultanée de l'expression d'un panel de 28 gènes tels que définis précédemment était très pertinente pour discriminer des patients BP et des patients MP. A ce titre, l'invention concerne également un procédé tel que défini précédemment caractérisé en ce qu'il comprend les étapes suivantes :
a. on extrait du matériel biologique de l'échantillon biologique,
b. on met en contact le matériel biologique avec au moins 28 sondes d'hybridation spécifiques de gènes cibles, lesdits gènes cibles présentant une séquence nucléique ayant l'une quelconque des SEQ ID N° 1 à 28 ; et
c. on détermine l'expression d'au moins lesdits 28 desdits gènes cibles.

L'expression d'un panel de 22 gènes particuliers, comprenant les gènes de SEQ ID N° 1,3, 7, 9-15, 17-28 permet à ce titre d'obtenir d'excellents résultats puisque qu'il permet de bien classifier 92% des patients de bons pronostic et 100% des patients de mauvais pronostic. A ce titre, l'invention concerne un procédé pour le diagnostic/pronostic d'un syndrome septique à partir d'un échantillon biologique d'un patient caractérisé en ce qu'il comprend les étapes suivantes :
a. on extrait du matériel biologique de l'échantillon biologique,
b. on met en contact le matériel biologique avec au moins 22 sondes d'hybridation spécifiques de gènes cibles, lesdits gènes cibles présentant une séquence nucléique ayant l'une quelconque des SEQ ID N° 1, 3, 7, 9-15, 7-28 ; et
c. on détermine l'expression d'au moins lesdits 22 desdits gènes cibles.

L'utilisation d'un panel de gènes restreint est particulièrement adapté pour obtenir un outil de pronostic. En effet, l'analyse de l'expression d'une vingtaine de gènes ne nécessite pas la fabrication à façon de puces à ADN, et peut être mise en oeuvre directement par des techniques de PCR ou de NASBA, ou encore de puce basse densité, ce qui présente un atout économique important et une mise en oeuvre simplifiée.

Un support est divulgué, tel que défini précédemment comprenant au moins 28 sondes d'hybridation choisis parmi les sondes spécifiques des gènes cibles présentant une séquence nucléique ayant l'une quelconque des SEQ ID N° 1 à 28 Un autre support divulgué ici comprend au moins 22 sondes d'hybridation choisis parmi les sondes spécifiques des gènes cibles présentant une séquence nucléique ayant l'une quelconque des SEQ ID N° N° 1, 3,7, 9-15, 17-28. Un autre support divulgué ici comprend au moins une sonde d'hybridation spécifique d'au moins un gène cible présentant une séquence nucléique ayant l'une quelconque des SEQ ID N° 1 à 28, préférentiellement au moins une sonde d'hybridation spécifique d'au moins un gène cible présentant une séquence nucléique ayant l'une quelconque des SEQ ID N° 1,2,4-8, 11 et 16.

Enfin l'utilisation d'un support tel que défini précédemment pour le diagnostic/pronostic d'un syndrome septique est également divulguée.

L'utilisation d'au moins 28 réactifs spécifiques des gènes cibles présentant une séquence nucléique ayant l'une quelconque des SEQ ID N°1 à 28 tel que défini précédemment pour le diagnostic/pronostic d'un syndrome septique est divulgué. Préférentiellement, au moins 22 réactifs spécifiques des gènes cibles présentant une séquence nucléique ayant l'une quelconque des SEQ ID N° 1,3, 7, 9-15, 17-28 tel que défini précédemment sont utilisés pour le diagnostic/pronostic d'un syndrome septique.

L'utilisation d'au moins un réactif spécifique des gènes cibles présentant une séquence nucléique ayant l'une quelconque des SEQ ID N° 1, 2, 4-8, 11 et 16 tel que défini précédemment pour le diagnostic/pronostic d'un syndrome septique est divulguée.

Un kit de diagnostic/pronostic d'un syndrome septique comprenant un support tel que défini précédemment est également divulgué. La présente divulgation concerne également un kit de diagnostic/pronostic d'un syndrome septique comprenant au moins 28 réactifs spécifiques des gènes cibles présentant une séquence nucléique ayant l'une quelconque des SEQ ID N°1 à 28 tel que défini précédemment pour le diagnostic/pronostic d'un syndrome septique. Préférentiellement, un kit de diagnostic/pronostic d'un syndrome septique comprend au moins 22 réactifs spécifiques des gènes cibles présentant une séquence nucléique ayant l'une quelconque des SEQ ID N° 1, 3, 7, 9-15, 17-28 tel que défini précédemment pour le diagnostic/pronostic d'un syndrome septique.

La présente divulgation concerne également un kit de diagnostic/pronostic d'un syndrome septique comprenant au moins un réactif spécifique des gènes cibles présentant une séquence nucléique ayant l'une quelconque des SEQ ID N° 1, 2, 4-8, 11 et 16 tel que défini précédemment pour le diagnostic/pronostic d'un syndrome septique.

Bien entendu, toutes les définitions indiquées précédemment dans la description s'appliquent pour tous les modes préférés de réalisation de l'invention.

Le figure ci-jointe est donnée à titre d'exemple explicatif et n'a aucun caractère limitatif. Elle permettra de mieux comprendre l'invention.

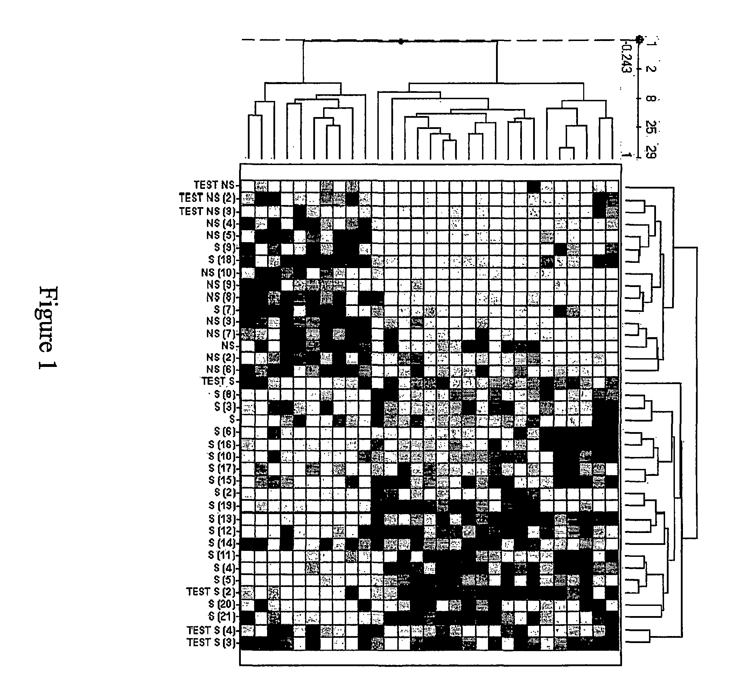

La figure 1 représente une analyse de clustering hiérarchique de 38 échantillons de sang obtenu à partir de 13 patients MP (appelé également NS) et 26 patients BP (appelé également S) en utilisant l'expression de 28 gènes selon l'invention, mesurée par 29 probe sets sur la biopuce Affymetrix. La fonction de clustering hiérarchique du logiciel Spofire organise les patients MP et BP en colonnes, et les gènes en lignes de manière à obtenir en position adjacente les patients ou les gènes présentant des profils d'expression comparables. Le coefficient de corrélation de Pearson a été utilisé comme indice de similarité pour les gènes et les patients. Par la suite, la méthode de clustering des moyennes non pondérées UPGMA d'une part, et, la valeur moyenne de tous les échantillons d'autre part, ont respectivement permis d'organiser les patients et les gènes. Les résultats correspondent au niveau de fluorescence Affymetrix normalisé par le logiciel « Affy ». Afin de tenir compte des différences constitutives d'expression entre les gènes, les niveaux d'expression de chaque gène ont été normalisés en appliquant une loi normale centrée réduite. Le blanc représente les faibles niveaux d'expression, le gris les niveaux intermédiaires et le noir les niveaux forts. La hauteur des branches du dendodogramme indique l'indice de similarité entres les profils d'expression.

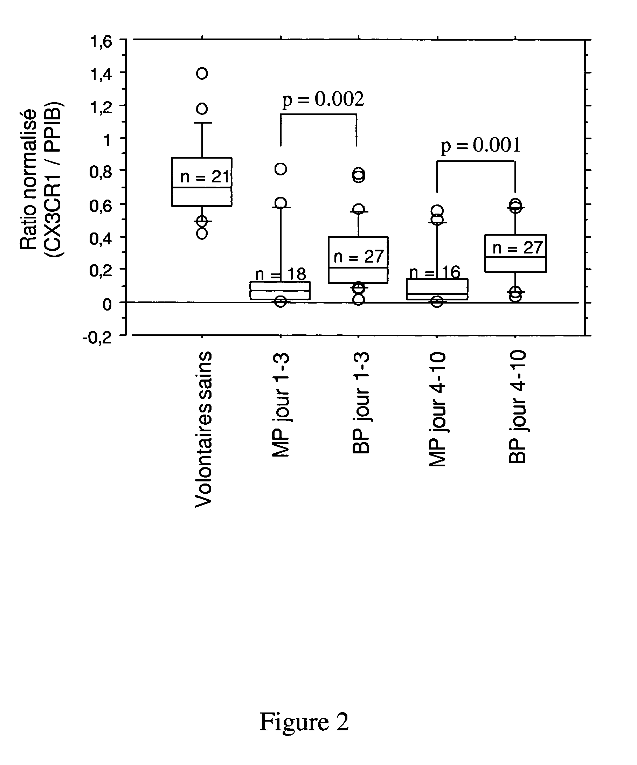

La figure 2 présente la quantification d'ARNm CX3CR1 dans le sang de patients en choc septique. Le niveau d'expression génique a été mesuré par RT-PCR quantitative chez 50 patients en choc septique (19 MP et 21 BP) et 21 volontaires sains. Les résultats ont été normalisés au niveau d'expression du gène de ménage PPIB. Les résultats sont présentés avec la médiane, le 25ème et le 75ème percentile. La comparaison statistique entre les PM et MP a été effectuée grâce au test non paramétrique de Mann Withney.

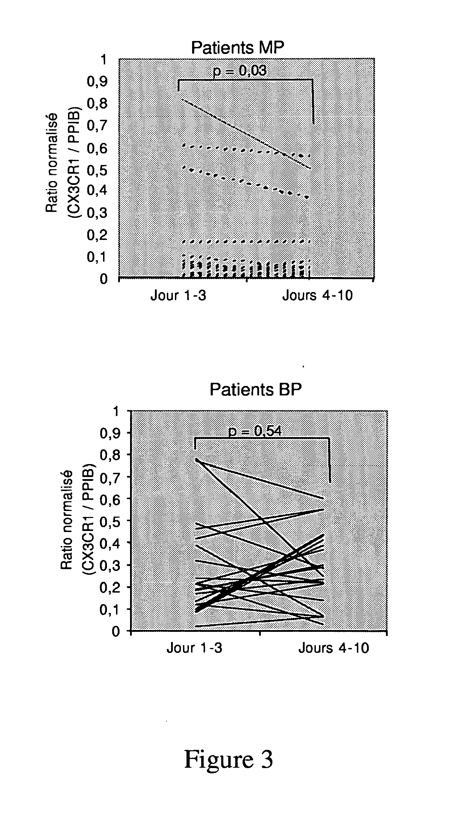

La figure 3 présente la quantification d'ARNm CX3CR1 quantifiés dans le sang de patients en choc septique. Le niveau d'expression génique a été mesuré par RT-PCR quantitative chez 37 patients en choc septique (12 MP et 21 BP). Pour chaque patient, un prélèvement PAXgene a été obtenu entre J1-J3 et un autre entre J4-J10. Les résultats ont été normalises au niveau d'expression du gène de ménage PPIB. L'évolution du niveau d'expression génique de CX3CR1 entre J1-J3 et J4-J10 chez les MP et BP a été effectuée grâce au test non paramétrique de Wilcoxon.

Les exemples suivants sont donnés à titre illustratif et n'ont aucun caractère limitatif. Ils permettront de mieux comprendre l'invention.

### Exemple 1 : Recherche d'un profil d'expression pour le diagnostic/pronostic d'un syndrome septique

*Caractéristiques des échantillons biologiques :* L'étude a été réalisée sur des patients ayant développés un syndrome septique, et admis dans le service de réanimation Chirurgicale ou Médicale du centre hospitalier Lyon-Sud. Pour être inclus dans l'étude, les patients devaient présenter les critères suivants : âge supérieur à 18 ans ; présence d'un choc septique selon la conférence de consensus décrite auparavant ; absence de co-morbidité (cancer métastatique, hémopathie maligne, diabète de type I, pathologie hépatique chronique, insuffisance rénales chronique, SIDA). L'objectif de l'étude étant d'étudier la mortalité tardive induite par un choc septique, les patients décédés au cours des 48 premières heures du syndrome ont été exclus de l'étude. La prise en charge de tous les patients inclus était similaire.

En considérant le jour de la première administration de catécholamine comme le J1 du choc septique, chaque patient a été suivi pendant une période de 28 jours maximum. Sur la base de la mortalité observée au cours de cette période, un groupe de 10 patients (MP), et un groupe de 21 patients (BP) ont été étudiés. Par la suite, le panel de gène selon l'invention a été validé en aveugle à l'aide de deux groupes de patients recrutés sur les même critères : un groupes de 3 patients MP et un groupe de 4 patients BP. Les analyses de génomique ont été effectuées à partir de prélèvements obtenus entre J2 et J4. Les caractéristiques démographiques de l'ensemble de la cohorte sont présentées dans le tableau suivant

| | | **BP** | | **MP** | | **Total** | ***P*^{a}** |
|---|---|---|---|---|---|---|---|
| | | **Train *n*=21 (%)** | **Test *n*=4 (%)** | **Train n=10 (%)** | **Test *n*=3 (%)** | ***n*=38 (%)** | |
| Homme | | 13 (62) | 2(50) | 7 (70) | 1 (33) | 23 (61) | 0.930 |
| Femme | | 8 (38) | 2(50) | 3 (30) | 2 (67) | 15 (39) | |
| Age (années)^{b} | | 67 (49-71) | 71 (66-75) | 68 (57-79) | 78 (63-80) | 67 (54-78) | 0.371 |
| SAPS II à l'admissionb | | 48 (40-55) | 45 (37-52) | 61 (59-73) | 61 (60-72) | 55 (42-61) | <0.001 |
| Durée d'hospitalisation en réa.^{b} | | 12 (10-26) | 32 (28-34) | 9(8-14) | 4 (4-10) | 12 (9-25) | 0.013 |
| BPCO | | 1 (5) | 2 (50) | 3 (30) | 1 (33) | 7 (18) | 0.203 |
| Critères de Mac Cabe et Jackson | 0 | 7 (33) | 1 (25) | 0 | 0 | 8 (21) | 0.045 |
| | 1 | 9 (43) | 2 (50) | 9 (90) | 1 (33) | 21 (55) | |
| | 2 | 5 (24) | 1 (25) | 0 | 2 (67) | 8 (21) | |
| | 3 | 0 | 0 | 1 (10) | 0 | 1 (3) | |
| Diagnostic microbiologiquement documenté | | 15 (71) | 4 (100) | 7 (70) | 3 (100) | 29 (76) | >0.999 |
| | Bacille Gram (-) | 8 (38) | 1 (25) | 3 (30) | 3 (100) | 15 (39) | 0.950 |
| | Cocci Gram (+) | 7(33) | 1 (25) | 5 (50) | 1 (33) | 14 (37) | |
| | Fungique | 6 (29) | 1 (25) | 3 (30) | 1 (33) | 11 (29) | |
| Type d'infection | Communautaire | 7 (33) | 4 (100) | 5 (50) | 1 (33) | 17 (45) | 0.900 |
| | Nosocomiale | 14 (67) | 0 (0) | 5 (50) | 2 (67) | 21 (55) | |
| Site de l'infection | Pulmonaire | 6 (29) | 2 (50) | 8 (80) | 1 (33) | 17 (45) | 0.061 |
| | Abdominale | 12 (57) | 1 (25) | 2 (20) | 2 (67) | 17 (45) | |
| | Autres | 3 (14) | 1 (25) | 0 (0) | 0 (0) | 4 (11) | |

| | | | | | | | |
|---|---|---|---|---|---|---|---|
| ^{a}: comparison between the overall population of survivors (n = 25) and non-survivors (n = 13) ^{b} : Median (Q1-Q3) BPCO : broncho-pneumopathie chronique obstructive | | | | | | | |

### Extraction du matériel biologique (ARN totaux) de l'échantillon biologique :

Les prélèvements ont été collectés directement dans des tubes PAXGene^{™} Blood RNA (PreAnalytix, Frankin Lakes, USA). Après l'étape de prélèvement de l'échantillon sanguin et afin d'obtenir une lyse totale des cellules, les tubes ont été laissés à température ambiante pendant 4 h puis conservés à -20°C jusqu'à l'extraction du matériel biologique. Plus précisément, dans ce protocole, les ARN totaux ont été extraits à l'aide des kits PAXGene Blood RNA® (PreAnalytix) en respectant les recommandations du fabriquant. Brièvement, les tubes ont été centrifugés (10 min, 3000 g) afin d'obtenir un culot d'acides nucléiques. Ce culot a été lavé et repris dans un tampon contenant de la protéinase K nécessaire à la digestion des protéines (10 min à 55°C). Une nouvelle centrifugation (5 min 19 000g) a été effectuée pour éliminer les débris cellulaires et de l'éthanol a été ajouté afin d'optimiser les conditions de fixation des acides nucléiques. Les ARN totaux ont été spécifiquement fixés sur les colonnes PAXgene RNA spin column et, avant l'élution de ceux-ci, une digestion de l'ADN contaminant a été effectuée à l'aide du RNAse free DNAse set (Qiagen Ltd, Crawley, UK). La qualité des ARN totaux a été analysée par le bio analyseur AGILENT 2100 (Agilent Technologies, Waldbronn, Germany). Les ARN totaux comprennent les ARN de transfert, les ARN messagers (ARNm) et les ARN ribosomaux.

*Synthèse d'ADNc, obtention des ARNc, marquage des ARNc et quantification :* Afin d'analyser l'expression des gènes cibles selon l'invention, les ADN complémentaires (ADNc) des ARNm contenus dans les ARN totaux tels que purifiés ci dessus, ont été obtenus à partir de 5 µg d'ARN totaux par l'utilisation de 400 unités de l'enzyme de transcription reverse SuperScriptII (Invitrogen) et 100 pmol d'amorce poly-T contenant le promoteur de la T7 promotor (T7-oligo(dT)24-primer, Proligo, Paris, France). Les ADNc ainsi obtenus ont ensuite été extraits avec du phénol/chloroforme, et précipités par de l'acétate d'ammonium et de l'éthanol, et remis en solution dans 24 µl d'eau DEPC. Un volume de 20 µl de cette solution purifiée d'ADNc a fait l'objet ensuite d'une transcription *in vitro* par l'utilisation d'une ARN polymérase T7 qui reconnaît spécifiquement le promoteur de la T7 polymérase tel que mentionné ci dessus. Cette transcription permet d'obtenir l'ARNc de l'ADNc. Cette transcription a été réalisée par l'utilisation d'un kit Bioarray High Yield RNA Transcript Labeling Kit (Enzo Diagnostics, Farmingdale, NY), qui permet non seulement d'obtenir l'ARNc mais également lincorporation de bases cytidine et uridine biotinylées lors de la synthèse de l'ARNc.

Les ARNc purifiés ont ensuite été quantifiés par spectrophotométrie, et la solution d'ARNc a été ajustée à une concentration de 1 µg/µl d'ARNc. L'étape de clivage de ces ARNc a ensuite été réalisée à 94°C pendant 35 min, par l'utilisation d'un tampon de fragmentation (40 mM de Tris acétate, pH 8,1, 100 mM d'acétate de potassium, 30 mM d'acétate de magnesium) afin de provoquer l'hydrolyse des ARNc et obtenir des fragments de 35 à 200 bp. Le succès d'une telle fragmentation a été vérifié par une électrophorèse sur gel d'agarose 1,5%.

*Mise en évidence d'un profils d'expression différentiel entre les patients MP et BP :* Pour cela, 20 µg d'ARNc fragmentés issus de chaque échantillon ont été ajoutés à un tampon d'hybridation (Affymetrix) et 200 µl de cette solution ont été mis en contact pendant 16 h à 45°C sur une puce d'expression (Human Genome U133A GeneChip® (Affymetrix), qui comporte 22 283 groupes de sondes représentant environs 14500 gènes selon le protocole d'Affymetrix tel que décrit sur le site internet d'Affymetrix. Afin d'enregistrer les meilleures performances d'hybridation et de lavage, des ARN qualifiés de « contrôle » biotinylés (bioB, bioC, bioD et cre) et des oligonucléotides (oligo B2) ont également été inclus dans le tampon d'hybridation. Après l'étape d'hybridation, la solution d'ARNc biotinylée et hybridée sur la puce, a été révélée par l'utilisation d'une solution de streptavidine-phycoerythrine et le signal a été amplifié par l'utilisation d'anticorps anti-streptavidine. L'hybridation a été réalisée dans une étuve d'hybridation «GeneChip Hybridisation oven » (Affymetrix), et le protocole Euk GE-WS2V4 du protocole d'Affymetrix a été suivi. Les étapes de lavage et de révélation ont été réalisées sur une station «Fluidics Station 450» (Affymetrix). Chaque puce U133A a ensuite été analysée sur un scanner Agilent G2500A GeneArray Scanner à une résolution de 3 microns afin de repérer les zones hybridées sur la puce. Ce scanner permet la détection du signal émis par les molécules fluorescentes après excitation par un laser argon en utilisant la technique du microscope à épifluorescence. On obtient ainsi pour chaque position, un signal proportionnel à la quantité de ARNc fixés. Le signal a ensuite été analysé par le logiciel Microarray Suite 5.0 software (MAS5.0, Affymetrix). Afin de prévenir les variations obtenues par l'utilisation de différentes puces, il a été réalisé une approche de normalisation globale utilisant le logiciel MAS5.0 (Affymetrix), qui, grâce à un algorithme statistique, permet de définir si un gène était exprimé ou non. Afin de pouvoir comparer les puces entres elles, les données brutes (fichier «.CELL ») ont été traitées par une étape de normalisation quantile à l'aide de l'implémentation «Affy» du logiciel «R » (Gautier, L. et al., Bioinformatics (2004), p.307-315).Chaque gène représenté sur la puce U133A était couvert par 11 couples de sondes de 25 oligonucléotides. Par couple de sondes, on entend une première sonde qui s'hybridait parfaitement (on parle alors de sondes PM ou perfect match) avec un des ARNc issus d'un gène cible, et une deuxième sonde, identique à la première sonde à l'exception d'un mésappariement (on parle alors de sonde MM ou mismatched) au centre de la sonde. Chaque sonde MM servait à estimer le bruit de fond correspondant à une hybridation entre deux fragments nucléotidiques de séquence non complémentaires. (Affymetrix technical note "Statistical Algorithms Référence Guide"; Lipshutz, et al (1999) Nat. Genet. 1 Suppl., 20-24). Les 38 échantillons de l'étude montraient une moyenne de 38.1 ± 4.2% de gènes exprimés.

L'analyse des données d'expression a été réalisée par le logiciel Microsoft Excel, le logiciel Spotfire Décision Site for Functionnal Genomics V7.1 (Spotfire AB, Gothenburg, Sweden), ainsi qu'un algorithme statistique : l'Algorithme Génétique (Gautier, L. et al., Bioinformatics (2004), p.307-315 ; Ooi, C.H. and Tan, P. Bioinformatics (2003), p.37-44).A partir des 22 283 groupes de sondes, représentant environ 14 500 gènes, de la puce, les inventeurs ont dûment sélectionné les gènes pertinents qui permettaient de différencier les patients MP des patients BP.

Pour cela, une première étape a consisté à exclure les gènes présentant un niveau d'expression comparable entre tous les groupes de patients. Quatre étapes ont été effectuées :
- les gènes non exprimés chez l'ensemble des patients ont été exclus (logiciel MAS5.0).
- les gènes dont la médiane de fluorescence était inférieure à 30 dans les deux groupes ont été exclus
- les gènes qui n'étaient pas exprimés chez au moins 30 % des patients dans l'un des deux groupes ont été exclus
- les gènes pour lesquels le rapport des médianes d'expression entre les patients BP et MP étaient compris entre 0,77 et 1,3 ont été exclus.

Suite à l'application de ces filtres, un groupe de 2216 groupe de sonde a été sélectionné et a servi de base de travail pour une analyse multi-paramétrique avec l'Algorithme Génétique.

*Résultats obtenus:* une liste de 28 gènes a été identifiée. L'augmentation ou la diminution d'expression de chacun de ces gènes, observée chez les patients AIA par rapport aux patients ATA est indiquée dans le tableau 2.

**Tableau 2 - Liste de 28 gènes exprimés différentiellement chez les patients MP et BP**

| **SEQ ID N°** | **Nom de gène** | **Nom abrégé** | **Expression chez AIA versus ATA** |
|---|---|---|---|
| **1** | chemokine (C-X3-C motif) receptor 1 | CX3CR1 | Augmentée* |
| **2** | T cell receptor delta diversity 3 | TRDD3 | Augmentée^{£} |
| **3** | KIAA0882 protein | KIAA0882 | Augmentée |
| **4** | T-cell lymphoma invasion and metastasis 1 | TIAM1 | Augmentée^{£} |
| **5** | Interleukin 1, beta | IL1B | Augmentée* |
| **6** | Carbonyl reductase 1 | CBR1 | Augmentée^{£} |
| **7** | TIR domain containing molecule 1 | TRIF | Augmentée* |
| **8** | FYN tyrosine kinase protooncogene | FYN | Augmentée^{£} |
| **9** | Heparanase | HPSE | Augmentée |
| **10** | SRY (Sex determining region Y) box 4 | SOX4 | Augmentée^{£} |
| **11** | Interleukin 2 receptor, beta | IL2RB | Augmentée* |
| **12** | Rafr-linking protein | RAFTLIN | Augmentée |
| **13** | CGI-40 protein Homo sapiens SID1 transmembrane family, member 2 | CGI-40 SIDT2 | Augmentée |
| **14** | glucose-6-phosphatase catalytic subunit 3 | G6PC3 | Augmentée |
| **15** | Mannosidase alpha, class 1A member 2 | MAN1A2 | Augmentée |
| **16** | Myeloid differentiation primary response gene (88) | MYD88 | Augmentée* |
| **17** | Ribosomal protein L6 | RPL6 | Augmentée |
| **18** | Ribosomal protein L10a | RPL10a | Augmentée |
| **19** | sin3-associated polypeptide, 30kDa | SAP30 | Diminuée |
| **20** | Mitogen activated protein kinase-activated protein kinase 2 | MAPKAPK 2 | Diminuée |
| **21** | Presenlin enhancer 2 | PEN2 | Diminuée |
| **22** | Hypothetical protein LOC55924 | LOC55924 | Diminuée |
| **23** | Solute carrier family 39 (zinc transporter member 7) | SLC39A7 | Diminuée^{£} |
| **24** | Glutathione peroxidase 3 (plasma) | GPX3 | Diminuée^{£} |
| **25** | Hemochromatosis | HFE | Diminuée |
| **26** | Transcriptional activator of the cfos promoter | CROC4 | Diminuée |
| **27** | peroxisomal biogenesis factor 6 | PEX6 | Diminuée |
| **28** | Huntingtin intercating protein | | Diminuée |

L'indication d'une * et ^{£} indiquent respectivement une différence statistiquement différente entre les deux groupes selon un test T avec correction de Bonferroni ou de Benjamini et Hochberg, respectivement. Ceci indique que ces gènes pris isolément sont très pertinents dans le diagnostic/pronostic d'un syndrome septique.

### Validation par RT-PCR quantitative

Afin de confirmer ces résultats à l'aide d'une autre technique de biologie moléculaire, certains gènes ont été dosés par RT-PCR quantitative. Brièvement, une réaction de transcription reverse (RT) a été réalisée dans un volume final de 20 µl L'ARN total (1 µg) a été mélangé avec 1 µl de polyT à 50 µM et 1 µl de dNTP mix (ThermoScript^{™} RT-PCR system, Invitrogen), puis incubé 5 min à 65°C. Après refroidissement dans la glace, la solution a été mélangée avec 4 µl de 5x cDNA synthesis buffer, 1 µl de RNAse out (40 U/µl), 1 µl d'eau traitée au DEPC et 1 µl de Thermoscript RT (15 U/µl), tous ces produits étant issus du ThermoScript^{™} RT-PCR system (Invitrogen). La transcription reverse a été effectuée pendant 1 h à 50°C puis stoppée par une incubation de 5 min à 85°C. Pour finir, chaque solution de cDNA a été diluée au 1/10 dans de l'eau DEPC.

Pour chacun des gènes d'intérêt, un standard a été préparé par une amplification PCR (polymerase chain reaction) conduite jusqu'à saturation. Les amplicons obtenus ont été purifiés (PCR purification kit, Qiagen Ltd) et la présence d'un amplicon unique a été vérifié par électrophorèse sur gel d'agarose et marquage au bromure d'éthidium.

Le standard du gène « de ménage » Peptidylpropyl isomerase B (PPIB) codant pour la cyclophilin B ont été obtenus chez Search-LC (Heidelberg, Allemagne).

### Analyse de l'expression des ARNm par PCR en temps réel

Les ARNm des gènes cibles de SEQ ID N°1, 5, 11, 16 ont été quantifiés par PCR quantitative en temps réel en utilisant le LightCycler^{™} (Roche). Les réactions PCR ont été effectuées à l'aide du Fast-Start^{™} DNA Master SYBR Green I real-time PCR kit (Roche Molecular Biochemicals). Chaque PCR a été effectuée dans un volume final de 20 µl contenant 1 µl de LC-Fast Start Reaction Mix SYBR Green I, 1 µl de LC-Fast Start DNA Master SYBR Green I/Enzyme (incluant la Taq DNA polymerase, le tampon de réaction et un mélange de deoxynucleotides triphosphate), du MgCl₂ (3 mM concentration finale), les amorces sens et anti-sens (0.5 µM concentration finale), et 10 µl de solution de cDNA. Après une étape de dénaturation de 10 min à 95°C, l'amplification a été effectuée à l'aide de 40 cycles d'un protocole de "touch-down" PCR (10 s à 95°C, 10 s d'hybridation à 68-58°C, suivi d'une extension de 16 s à 72°C). A la fin de chaque cycle, la fluorescence émise par le SYBR Green a été mesurée.

Pour confirmer la spécificité de l'amplification, les produits PCR ont systématiquement fait l'objet d'une analyse de courbe de fusion (LightCycler^{™} - Roche). Pour cela, les produits PCR ont été traités par une température croissante de 58 à 98°C avec une augmentation de 0,1°C/s. Pour chaque produit PCR, un seul pic a été obtenu lors de l'analyse de la courbe, caractérisé par une température de fusion spécifique.

Les combinaisons d'amorces nécessaires à la quantification du gène de ménage PPIB et IL-1β (SEQ ID n° 5) ont été obtenues chez Search LC (Heidelberg, Allemagne). Pour PPIB, le n° d'accesion Genbank était M60857 et on amplifiait la région 105-338. Pour l'IL-1β, le n° d'accesion Genbank était M15330 et on amplifiait la région 438-642. Les couples d'amorces utilisés pour doser les gènes cibles de SEQ ID N°1, 11, 16, la séquence Genbank utilisée comme référence et la position des amplicons sont décrits dans le tableau ci dessous.

| **GENE CIBLE DE SEQ ID N°** | | | **amplicon** |
|---|---|---|---|
| | Primer sens 5'-->3' | SEQ ID N°29 TGACTGGCAGATCCAGAGGTT | |
| 1 | Primer anti-sens 5'-->3 | SEQ ID N°30 GTAGAATATGGACAGGAACAC | 164 bases |
| | Primer sens 5'-->3' | SEQ ID N°31 CCTGAAGTGTAACACCCCAGA | |
| 11 | Primer anti-sens 5'-->3 | SEQ ID N°32 TCCCTCTCCAGCACTTCTAGT | 162 bases |
| | Primer sens 5'-->3' | SEQ ID N°33 TGCTGGAGCTGGGACCCAGCATTGAGGAGGA | |
| 16 | Primer anti-sens 5'-->3 | SEQ ID N°34 TCAGACACACACAACTTCAGTCGATAG | 280 bases |

La quantité d'ARNm cible relative à la quantité d'ARNm du gène de ménage PPIB a été analysée par la technique de quantification relative avec le LightCycler Relative Quantification Software (Roche Molecular Biochemicals). La "Second Derivative Maximum Method" du logiciel LightCycler^{™} (Roche) a été utilisée pour déterminer automatiquement le Crossing Point (Cp) pour chaque échantillon. La valeur du Cp a été définie comme le nombre de cycles pour lequel la fluorescence était significativement différente du bruit de fond.

Cinq dilutions en séries au 1/10 ont été réalisées en quadruplicate avec chaque standard afin de générer une courbe d'étalonnage exprimant le Cp en fonction du logarithme du nombre de copies. Les dilutions de standard ont été optimisées afin que la courbe d'étalonnage couvre le niveau d'expression attendu pour le gène cible et le gène de ménage. Les courbes standards relatives décrivant l'efficacité de PCR pour le gène cible et le gène de ménage ont été générées et utilisées pour réaliser une quantification avec le LightCycler Relative Quantification Software (Roche Molecular Biochemicals).

Les résultats obtenus pour le dosage des ARNm des gènes cibles de SEQ ID N° 1, 5, 11 et 16 en RT-PCR quantitative sont présentés dans le tableau 5 ci dessous. Les résultats correspondent à 25 échantillons (8 MP et 17 BP). La corrélation des résultats obtenus d'une part avec la biopuce et d'autre part avec la technique en RT-PCR quantitative a été établie grâce au test de corrélation de Spearman.

**Tableau 2 - Comparaison des niveaux d'expression de 4 gènes entre Affymetrix et RT-PCR quantitative.**

| **Nom du gène abrégé** | **médiane Affymetrix BP** | **médiane Affymetrix MP** | **médiane RT- PCR BP** | **médiane RT- PCR MP** | **Spearman coefficient corrélation: r** | **Spearman test significativité: p** |
|---|---|---|---|---|---|---|
| CX3CR1 | 582,965 | 92,995 | 0,04295 | 0,00663 | 0.94 | < 0.001 |
| IL-1β | 227,64 | 113,4 | 0,329 | 0,18 | 0.83 | < 0.001 |
| IL-2Rβ | 204,86 | 131,965 | 0,00075 | 0,00024 | 0.76 | < 0.001 |
| MvD88 | 2644,03 | 1986,315 | 0,0351 | 0,0294 | 0.56 | < 0.01 |

Pour les 4 gènes analysés, une corrélation significative a été observée entres les résultats Affymetrix et ceux de RT-PCR quantitative, confirmant la pertinence des gènes selon l'invention.

En suivant le même protocole que celui décrit dans les paragraphes précédents, les ARNm CX3CR1 ont été quantifiés à partir d'échantillons de sang prélevés chez 50 patients en choc septiques (19 MP et 21 BP). Un échantillon de sang a été obtenu au cours des 72 premières heures après le début du choc puis, un second échantillon a été prélevé plus tardivement au cours du syndrome. Le niveau d'expression de CX3CR1 a été normalisé à celui du gène de ménage PPIB. Les résultats sont présentés dans la figure 2. La comparaison entre BP et MP a été effectuée grâce au test non paramétrique de Mann Whitney. Il est donc particulièrement intéressant d'analyser l'expression de l'ARNm CX3CR1 en tant que facteur de mauvais pronostic.

Le niveau d'expression de l'ARNm CX3CR1 montrait une diminution significative au cours du temps chez les patients MP. Les résultats sont présentés sur la figure 3. L'évolution de l'expression au cours du temps a été testée grâce au test de Wilcoxon.

Il est donc particulièrement intéressant de suivre l'expression de l'ARNm CX3CR1 au cours du temps pour confirmer ce mauvais pronostic.

### Analyse de l'expression de panel de gènes

Les inventeurs ont également mis en évidence que l'analyse simultanée de l'expression de plusieurs gènes était très pertinente pour discriminer des patients BP et MP.

Les inventeurs ont ainsi mis en évidence que l'analyse simultanée de l'expression des 28 gènes décrit précédemment était très pertinente pour discriminer les deux groupes de BP et MP.

Les résultats sont présentés dans la figure 1. Cette liste permettait de clusteriser 88 % des échantillons de patients BP dans un groupe et 100 % des échantillons de patients MP dans un autre groupe.

De plus, les inventeurs ont mis en évidence que l'analyse simultanée de l'expression des gènes SEQ ID N° 1, 3, 7, 9-15, 17-28 parmi les 28 décris précédemment était aussi particulièrement pertinente pour discriminer les deux groupes de BP et MP. Les résultats sont présentés dans la figure 2. Cette liste permettait de clusteriser 92 % des échantillons de patients BP dans un groupe et 100 % des échantillons de patients MP dans un autre groupe. Parmi les 28 gènes décrits précédemment, chacun des 9 gènes de SEQ ID 1, 2, 4-8, 11 et 16 permet de discriminer les deux groupes de patients. Le tableau 3 représente la p value calculée à l'aide du T test avec la correction de Bonferroni ou de Benjamini et Hochberg. Tous ces gènes étaient sur-exprimés chez les BP par rapport au MP.

**Tableau 3. Gènes permettant de discriminer les deux groupes de patients.**

| **Gene name** | **Gene Symbol** | **Bonferroni correction** | **BHFDR correction** | **Fold change** |
|---|---|---|---|---|
| Chemokine (C-X3-C motif) receptor 1 | CX3CR1 | 6,3E-05 | 6,3E-05 | 8,33 |
| T cell receptor delta diversity 3 | TRDD3 | > 0.05 | 4,4E-02 | 4,00 |
| T-cell lymphoma invasion and metastasis 1 | TIAM1 | > 0.05 | 2,7E-02 | 2,08 |
| Interleukin 1, beta | IL1B | 4,9E-02 | 9,7E-03 | 2,08 |
| Carbonyl reductase 1 | CBR1 | > 0.05 | 2,8E-02 | 1,89 |
| TIR domain containing adaptor inducing interferon-beta | TRIF | 5,3E-04 | 2,6E-04 | 1,72 |
| FYN tyrosine kinase protooncogene | FYN | > 0.05 | 2,7E-02 | 1,67 |
| Interleukin 2 receptor, beta | IL2RB | 4,3E-02 | 9,7E-03 | 1,52 |
| Myeloid differentiation primary response gene (88) | MYD88 | 3,5E-02 | 9,7E-03 | 1,37 |

## Revendications

1. Procédé pour le pronostic d'un syndrome septique à partir d'un échantillon biologique d'un patient **caractérisé en ce qu'**il comprend les étapes suivantes :
a. on extrait du matériel biologique de l'échantillon biologique,
b. on met en contact le matériel biologique avec au moins une sonde d'hybridation spécifique d'au moins un gène cible, ledit au moins gène cible présentant la séquence nucléique SEQ ID N° 1; et
c. on détermine l'expression dudit au moins gène cible.

2. Procédé selon la revendication 1, dans lequel dans l'étape b) on met en contact le matériel biologique avec 22 sondes d'hybridation respectivement spécifiques de 22 gènes cibles présentant respectivement une séquence nucléique identifiée SEQ ID N° 1, 3, 7, 9-15, 17-28 ; et
on détermine l'expression d'au moins lesdits 22 desdits gènes cibles.

3. Procédé selon la revendication 1, dans lequel dans l'étape b) on met en contact le matériel biologique avec 28 sondes d'hybridation respectivement spécifiques de 28 gènes cibles présentant respectivement une séquence nucléique identifiée en SEQ ID N° 1 à 28 ; et
on détermine l'expression d'au moins lesdits 28 desdits gènes cibles.

4. Procédé selon l'une quelconque des revendications 1 à 3, **caractérisé en ce que** l'échantillon biologique prélevé chez le patient est un échantillon sanguin.

5. Procédé selon la revendication 1, **caractérisé en ce que** le matériel biologique extrait lors de l'étape a) comprend des acides nucléiques.

6. Procédé selon la revendication 1, **caractérisé en ce que** la sonde d'hybridation est immobilisée sur un support.

## Patentansprüche

1. Verfahren zur Prognose eines septischen Syndroms ausgehend von einer biologischen Probe eines Patienten **dadurch gekennzeichnet, dass** es die folgenden Schritte umfasst, in denen man:
a. biologisches Material aus der biologischen Probe extrahiert,
b. das biologische Material mit mindestens einer Hybridisierungssonde in Kontakt bringt, die für mindestens ein Zielgen spezifisch ist, wobei das mindestens eine Zielgen die Nukleinsäuresequenz SEQ ID NR: 1 aufweist, und
c. die Expression des mindestens einen Zielgens bestimmt.

2. Verfahren nach Anspruch 1, bei dem man in Schritt b) das biologische Material mit 22 Hybridisierungssonden in Kontakt bringt, die jeweils für 22 Zielgene spezifisch sind, die jeweils eine angegebene Nukleinsäuresequenz SEQ ID NR: 1, 3, 7, 9-15, 17-28 aufweisen, und
die Expression von mindestens diesen 22 der Zielgene bestimmt.

3. Verfahren nach Anspruch 1, bei dem man in Schritt b) das biologische Material mit 28 Hybridisierungssonden in Kontakt bringt, die jeweils für 28 Zielgene spezifisch sind, die jeweils eine in SEQ ID NR: 1-28 angegebene Nukleinsäuresequenz aufweisen, und
die Expression von mindestens diesen 28 der Zielgene bestimmt.

4. Verfahren nach einem der Ansprüche 1 bis 3, **dadurch gekennzeichnet, dass** es sich bei der von dem Patienten entnommenen biologischen Probe um eine Blutprobe handelt.

5. Verfahren nach Anspruch 1, **dadurch gekennzeichnet, dass** das im Schritt a) extrahierte biologische Material Nukleinsäuren umfasst.

6. Verfahren nach Anspruch 1, **dadurch gekennzeichnet, dass** die Hybridisierungssonde auf einem Träger immobilisiert ist.

## Claims

1. A method for the prognosis of a septic syndrome based on a biological sample from a patient, **characterized in that** it comprises the following steps:
a. biological material is extracted from the biological sample;
b. the biological material is brought into contact with at least one hybridization probe specific for at least one target gene, said at least one target gene having nucleic sequence SEQ ID No 1; and
c. the expression of said at least one target gene is determined.

2. The method according to claim 1, in which, in step b), the biological material is brought into contact with 22 hybridization probes, respectively specific for the 22 target genes respectively showing a nucleic sequence identified as SEQ ID Nos 1, 3, 7, 9-15,17-28; and
the expression of said at least 22 of said target genes is determined.

3. The method according to claim 1, in which in step b), the biological material is brought into contact with 28 hybridization probes, respectively specific for 28 target genes respectively showing a nucleic sequence identified as SEQ ID Nos 1 to 28; and the expression of said at least 28 target genes is determined.

4. The method according to any one of claims 1 to 3, **characterized in that** the biological sample taken from the patient is a blood sample.

5. The method according to claim 1, **characterized in that** the biological material extracted in step a) comprises nucleic acides.

6. The method according to claim 1, **characterized in that** the hybridization probe is immobilized on a substrate.
